(19) Europäisches Patentamt
European Patent Office
Office européen des brevets

(11) EP 4 382 603 A1

(12) **EUROPEAN PATENT APPLICATION**
published in accordance with Art. 153(4) EPC

(43) Date of publication:
**12.06.2024 Bulletin 2024/24**

(21) Application number: **22853032.5**

(22) Date of filing: **01.08.2022**

(51) International Patent Classification (IPC):
***C12N 15/113*** *(2010.01)* ***A61K 31/7088*** *(2006.01)*
***A61K 31/712*** *(2006.01)* ***A61K 31/713*** *(2006.01)*
***A61K 48/00*** *(2006.01)* ***A61P 43/00*** *(2006.01)*
***C12N 15/10*** *(2006.01)* ***C12Q 1/6813*** *(2018.01)*

(52) Cooperative Patent Classification (CPC):
**A61K 31/7088; A61K 31/712; A61K 31/713; A61K 48/00; A61P 43/00; C12N 15/10; C12N 15/113; C12Q 1/6813**

(86) International application number:
**PCT/JP2022/029566**

(87) International publication number:
**WO 2023/013613 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2021 JP 2021126578**

(71) Applicant: **Sophia School Corporation**
**Tokyo 102-8554 (JP)**

(72) Inventor: **KONDO Jiro**
**Tokyo 102-8554 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **ARTIFICIAL NUCLEIC ACID FOR INDUCING SPECIFIC THREE-DIMENSIONAL STRUCTURE**

(57) The object of the present invention is to provide a method for actively forming a three-dimensional structure; a modification mode for a nucleic acid maintaining a three-dimensional structure; and an artificial nucleic acid capable of stably binding to a target sequence regardless of mutation, by utilizing non-complementary base pairs in nucleic acid therapeutics. Provided are an artificial nucleic acid for inducing a specific three-dimensional structure by hybridizing with a nucleic acid of interest that does not form a functional three-dimensional structure; a gene expression inhibiting agent and a nucleic acid detecting agent comprising the artificial nucleic acid as an active ingredient; and a method for producing an artificial nucleic acid.

EP 4 382 603 A1

## Description

Technical Field

The present invention relates to an artificial nucleic acid for inducing a specific three-dimensional structure by hybridizing with a nucleic acid of interest that does not form a functional three-dimensional structure, a gene expression inhibiting agent, and a nucleic acid detecting agent comprising the same as an active ingredient, and a method for producing an artificial nucleic acid.

Background Art

Nucleic acid molecules, such as DNA and RNA, are composed of nucleotides having 4 types of bases: adenine (A), thymine (T) (uracil (U) for RNA), guanine (G), and cytosine (C), in which A and T or U, and C and G tend to be base-paired, and this nature is called complementarity of base pairs. In vivo, various phenomena, such as the formation of double helix structures of DNA, semiconservative replication of DNA, and transcription of RNA using DNA as templates, are conducted based on the complementarity of the base pairs. In addition, because the rules of complementarity are clear, it has been applied to almost all biotechnologies involving nucleic acids, such as PCR, DNA sequencing, gene knockdown, and gene knockout. In particular, the gene knockdown method has been widely applied as "nucleic acid therapeutics," third-generation pharmaceuticals, and the number of pharmaceutical approvals has been increasing in recent years.

Gene knockdown methods utilized as nucleic acid therapeutics are mainly based on antisense oligonucleotides (ASO) or small interfering RNA (siRNA). ASOs and siRNAs are easy to design, but unfortunately, the drug efficacy is not stable in cases where mutations occur in target sequences. This is because the formation of base pairs ignoring the complementarity results in attenuated binding between targets and these molecules. Thus, genetic regions containing mutations of individual diversity, represented by single nucleotide variants (SNVs), had to be excluded from candidate target sequences.

In recent years, it has been revealed that nucleic acids stably form non-complementary base pairs *in vivo.* For Example, Non-Patent Literature 1 discloses that in single-stranded nucleic acids that form functional three-dimensional structures, about 150 types of non-complementary base pairs form stable three-dimensional structures.

These non-complementary base pairs, and the functional three-dimensional structures induced thereby could be utilized to achieve the development, for example, of nucleic acid therapeutics that stably act on target sequences containing mutations as described above. However, it has not been investigated to solve the problem of conventional nucleic acid therapeutics by using non-complementary base pairs. Moreover, even the basic knowledge that is needed to utilize non-complementary base pairs for nucleic acid therapeutics, such as methods to actively form functional three-dimensional structures and methods for modification while maintaining the three-dimensional structures, has not been provided.

Citation List

Non-Patent Literature

Non-Patent Literature 1: Leontis, N.B. et al., Nucleic Acids Res. 2002 Aug 15; 30(16): 3497-3531.

Summary of Invention

Technical Problem

The Object of the present invention is to develop a method for inducing the formation of a three-dimensional structure comprising a non-complementary base pair, and provide an artificial nucleic acid that can bind to a target sequence stably without being affected by any mutations in the target sequence.

Solution to Problem

In order to solve the above problems, the present inventors have intensively advanced the research and development and developed an artificial nucleic acid that hybridizes with a target nucleic acid not forming a three-dimensional structure to induce a specific three-dimensional structure. It has also been demonstrated that the introduction of modification into the artificial nucleic acid further improves the stability of the double-stranded nucleic acid. The present invention is based on novel findings such as those described above, and provides the following.

(1) An artificial nucleic acid for inducing a specific functional three-dimensional structure by hybridizing with a nucleic acid of interest that does not form a functional three-dimensional structure, wherein

said artificial nucleic acid comprises a three-dimensional structure formation-inducing domain that forms a three-dimensional structure together with a target domain in said nucleic acid of interest,
said target domain and said three-dimensional structure formation-inducing domain form a sequence motif composed of double strands forming the specific three-dimensional structure,
in said sequence motif, said three-dimensional structure formation-inducing domain and said target domain comprises complementary regions consisting of mutually complementary sequences,
in said sequence motif, said three-dimensional structure formation-inducing domain and/or said target domain further comprises a non-complementary-containing region with 1 or more bases comprising a mutually non-complementary sequence, and
said non-complementary-containing region comprises non-complementary sequences at its both ends.

(2) The artificial nucleic acid of (1), wherein said three-dimensional structure formation-inducing domain and/or said target domain comprises a plurality of said complementary regions, and wherein said non-complementary-containing region is located between said plurality of the complementary regions.
(3) The artificial nucleic acid of (1) or (2), wherein said non-complementary-containing region is composed of 2-7 bases.
(4) The artificial nucleic acid of any of (1) to (3), wherein said specific three-dimensional structure comprises 1 or more selected from the group consisting of Kink-turn structure, bulged-G structure, Reverse Kink-turn structure, 5S loop E structure, C-loop structure, and tandem GA structure.
(5) The artificial nucleic acid of (4), wherein

the Kink-turn structure is composed of SEQ ID NO: 3 (5'-NNNNGAN-3') and SEQ ID NO: 4 (5'-NGAN-3'),
the bulged-G structure is composed of SEQ ID NO: 1 (5'-NNNGUAN-3') and SEQ ID NO: 2 (5'-NGANNN-3'),
the Reverse Kink-turn structure is composed of SEQ ID NO: 5 (5'-NNNNAAN-3') and SEQ ID NO: 6 (5'-NGAN-3'),
the 5S loop E structure is composed of SEQ ID NO: 7 (5'-NGUAN-3') and SEQ ID NO: 8 (5'-NGAUN-3'),
the C-loop structure is composed of SEQ ID NO: 9 (5'-NCACU-3') and SEQ ID NO: 10 (5'-ANN-3'), or
the tandem GA structure is composed of SEQ ID NO: 11 (5'-NGAN-3') and SEQ ID NO: 12 (5'-NGAN-3'), and wherein N is A, C, G, or U in the base sequences.

(6) The artificial nucleic acid of any of (1) to (5), wherein the nucleic acid of interest is mRNA or miRNA.
(7) The artificial nucleic acid of any of (1) to (6), wherein said hybridization is performed under high stringent conditions.
(8) The artificial nucleic acid of any of (1) to (7), wherein said three-dimensional structure formation-inducing domain comprises 1 or more modified nucleotides.
(9) The artificial nucleic acid of (8), wherein said modified nucleotide is selected from the group consisting of 2'-OMe RNA, 2'-MOE RNA, LNA, 2'-O, 5'-N BNA, 2'-deoxy-trans-3',4'-BNA, and DNA.
(10) The artificial nucleic acid of (8) or (9), wherein said modified nucleotide comprises fluoro group-modification at the 2' position of the ribose.
(11) The artificial nucleic acid of any of (1) to (10), wherein said target domain comprises said non-complementary-containing region containing a mutation.
(12) The artificial nucleic acid of (11), wherein said mutation is a single nucleotide variant, insertion-deletion mutation, structural variant, or a combination thereof.
(13) The artificial nucleic acid of any of (1) to (12), further comprising a hybridizable domain(s) composed of 6-120 bases, adjacent to one or both side(s) of said three-dimensional structure formation-inducing domain.
(14) A gene expression inhibiting agent, comprising the artificial nucleic acid of any of (1) to (13) as an active ingredient.
(15) A nucleic acid detecting agent, comprising the artificial nucleic acid of any of (1) to (13) as an active ingredient.
(16) A method for producing an artificial nucleic acid for inducing a specific functional three-dimensional structure by hybridizing with a nucleic acid of interest that does not form a functional three-dimensional structure, comprising:

a target domain selecting step of searching said nucleic acid of interest for the sequence information for one side of a sequence motif composed of double strands forming said specific three-dimensional structure, and selecting 1 or more of said sequence information as a target domain;
a three-dimensional structure formation-inducing domain determining step of determining the sequence of said three-dimensional structure formation-inducing domain such that said three-dimensional structure formation-inducing domain forms a sequence motif together with said target domain; and
a nucleic acid synthesizing step of synthesizing said artificial nucleic acid based on the sequence information

determined in said three-dimensional structure formation-inducing domain determining step, wherein

in said sequence motif, said target domain and said three-dimensional structure formation-inducing domain comprise complementary regions consisting of mutually complementary sequences,

in said sequence motif, said target domain and/or said three-dimensional structure formation-inducing domain further comprises a non-complementary-containing region with 1 or more bases comprising a mutually non-complementary sequence, and

said non-complementary-containing region comprises non-complementary sequences at its both ends.

(17) The method of (16), further comprising a hybridizable domain determining step of determining the sequence of a hybridizable domain composed of 6 bases to 120 bases, adjacent to one or both side(s) of said three-dimensional structure formation-inducing domain.

The present specification encompasses the disclosure of JP 2021-126578A that serves as a basis for the priority of the present application.

Advantageous Effects of Invention

The artificial nucleic acid of the present invention enables induction of a specific functional three-dimensional structure into a nucleic acid that does not form a functional three-dimensional structure.

The gene expression inhibiting agent of the present invention enables downregulation of the expression of a target gene.

The nucleic acid detecting agent of the present invention enables detection of a nucleic acid of interest.

The method for producing an artificial nucleic acid of the present invention enables production of the artificial nucleic acid of the present invention.

Brief Description of Drawings

[Figure 1] Figure 1 illustrates the three-dimensional structure and the secondary structures of the bulged-G (BG) structure. Figure 1A shows an exemplary three-dimensional structure. Figure 1B shows exemplary secondary structures. In Figure 1B, the upper chain is an alfa chain; the lower chain is a beta chain; the inside of the box represents consensus sequences; and N represents any base. A bar between bases represents complementary pairing, and a white circle represents an important non-complementary pairing, $\alpha$1 to $\alpha$6 and $\beta$1 to $\beta$5 represent the positions of the bases in the alfa and beta chains, respectively, in the consensus sequences; * represents the position of a nucleotide that has been demonstrated to take C2'-endo conformation; and # represents the position of a nucleotide in which the hydroxy group at the 2' position of the ribose is involved in the hydrogen bond.

[Figure 2] Figure 2 illustrates the three-dimensional structure and the secondary structures of the Kink-turn (KT) structure. Figure 2A shows an exemplary three-dimensional structure. Figure 2B shows an exemplary secondary structure of the standard motif. Figure 2C shows exemplary secondary structures of non-standard motifs. In Figures 2B and 2C, the upper chain is an alfa chain; the lower chain is a beta chain; the inside of the box represents consensus sequences; and N represents any base. A thick bar between bases represents complementary pairing; a white circle represents an important non-complementary pairing; and a thin bar represents an internucleotide linkage. $\alpha$1 to $\alpha$7 and $\beta$1 to $\beta$4 represent the positions of the bases in the alfa and beta chains, respectively, in the consensus sequences; * represents the position of a nucleotide that has been demonstrated to take C2'-endo conformation; and # represents the position of a nucleotide in which the hydroxy group at the 2' position of the ribose is involved in the hydrogen bond.

[Figure 3] Figure 3 illustrates the three-dimensional structure and the secondary structures of the Reverse Kink-turn (RKT) structure. Figure 3A shows an exemplary three-dimensional structure. Figure 3B shows an exemplary secondary structure. In Figure 3B, the upper chain is an alfa chain; the lower chain is a beta chain; the inside of the box represents consensus sequences; and N represents any base. A bar between bases represents complementary pairing, and a white circle represents an important non-complementary pairing, $\alpha$1 to $\alpha$5 and $\beta$1 to $\beta$2 represent the positions of the bases in the alfa and beta chains, respectively, in the consensus sequences; * represents the position of a nucleotide that has been demonstrated to take C2'-endo conformation; and # represents the position of a nucleotide in which the hydroxy group at the 2' position of the ribose is involved in the hydrogen bond.

[Figure 4] Figure 4 illustrates the three-dimensional structure and the secondary structures of the 5S loop E (5S) structure. Figure 4A shows an exemplary three-dimensional structure. Figure 4B shows exemplary secondary structures of simple motifs. Figure 4C shows exemplary secondary structures of complex motifs. In Figures 4B and 4C,

the upper chain is an alfa chain; the lower chain is a beta chain; the inside of the box represents consensus sequences; and N represents any base. A bar between bases represents complementary pairing, and a white circle represents an important non-complementary pairing, $\alpha$1 to $\alpha$7 and $\beta$1 to $\beta$7 represent the positions of the bases in the alfa and beta chains, respectively, in the consensus sequences, and # represents the position of a nucleotide in which the hydroxy group at the 2' position of the ribose is involved in the hydrogen bond.

[Figure 5] Figure 5 illustrates the three-dimensional structure and the secondary structures of the C-loop (CL) structure. Figure 5A shows an exemplary three-dimensional structure. Figure 5B shows exemplary secondary structures. In Figure 5B, the upper chain is an alfa chain; the lower chain is a beta chain; the inside of the box represents consensus sequences; and N represents any base. A bar between bases represents complementary pairing, and a white circle represents an important non-complementary pairing, $\alpha$1 to $\alpha$5 and $\beta$1 to $\beta$3 represent the positions of the bases in the alfa and beta chains, respectively, in the consensus sequences; * represents the position of a nucleotide that has been demonstrated to take C2'-endo conformation; and # represents the position of a nucleotide in which the hydroxy group at the 2' position of the ribose is involved in the hydrogen bond.

[Figure 6] Figure 6 illustrates the three-dimensional structure and the secondary structures of tandem GA (GA) structure. Figure 6A shows an exemplary three-dimensional structure. Figure 6B shows an exemplary secondary structure. In Figure 6B, the upper chain is an alfa chain; the lower chain is a beta chain; the inside of the box represents consensus sequences; and N represents any base. A bar between bases represents complementary pairing, and a white circle represents an important non-complementary pairing, $\alpha$1 to $\alpha$2 and $\beta$1 to $\beta$2 represent the positions of the bases in the alfa and beta chains, respectively, in the consensus sequences, and # represents the position of a nucleotide in which the hydroxy group at the 2' position of the ribose is involved in the hydrogen bond.

[Figure 7] Figure 7 illustrates the base sequences and secondary structures of double-stranded nucleic acid molecules used in Example 1. A (ROI/RNA-ASO) represents an RNA of interest (ROI) and a nucleic acid that is completely complementary thereto (RNA-ASO); B (ROI/RNA-BG) represents an ROI and a nucleic acid that can form a bulged-G structure (RNA-BG); C (ROI/RNA-KT) represents an ROI and a nucleic acid that can form a Kink-turn structure (RNA-KT); D (ROI/RNA-RKT) represents an ROI and a nucleic acid that can form a Reverse Kink-turn structure (RNA-RKT); E (ROI/RNA-5 S) represents an ROI and a nucleic acid that can form a 5S loop E structure (RNA-5S); F (ROI/RNA-CL) represents an ROI and a nucleic acid that can form a C-loop structure (RNA-CL); and G (ROI/RNA-GA) represents an ROI and a nucleic acid that can form a tandem GA structure (RNA-GA). In the figure, the inside of a dashed-line box represents a region comprising sequences forming a three-dimensional structure as described above. A bar between bases represents complementary pairing, and a white circle represents an important non-complementary pairing.

[Figure 8] Figure 8 is a diagram showing the melting curves of the double-stranded nucleic acid molecules, as shown in Figure 7. In the figure, A to G correspond to the double-stranded nucleic acid molecules A to G, as shown in Figure 7, respectively.

[Figure 9] Figure 9 is a diagram showing melting curves of double-stranded nucleic acid molecules formed by a nucleic acid of interest and a nucleic acid having 2'-O-methyl (2'-OMe) modification. A (ROI/OMe-ASO) represents the result for a double-stranded nucleic acid between an ROI and a modified nucleic acid that is completely complementary thereto (OMe-ASO); B (ROI/OMe-BG) represents the result for a double-stranded nucleic acid between an ROI and a modified nucleic acid that can form a bulged-G structure (OMe-BG); C (ROI/OMe-5S) represents the result for a double-stranded nucleic acid between an ROI and a modified nucleic acid that can form a 5S loop E structure (OMe-5S); D (ROI/OMe-GA) represents the result for a double-stranded nucleic acid between an ROI and a modified nucleic acid that can form a tandem GA structure (OMe-GA).

[Figure 10] Figure 10 illustrates nucleic acids forming a bulged-G (BG) structure. Figure 10A represents the secondary structure of the base sequence of an unmodified nucleic acid (unmodified BG) used in a crystal structure analysis. Figure 10B represents the result of the crystal structure analysis on crystal I of the nucleic acid in Figure 10A. Figure 10C represents the result of the crystal structure analysis on crystal II of the nucleic acid in Figure 10A. Figure 10D represents the secondary structure of the base sequence of a modified nucleic acid (modified BG) used in a crystal structure analysis. Figure 10E represents the result of the crystal structure analysis on crystal I of the nucleic acid in Figure 10D. Figure 10F represents the result of the crystal structure analysis on crystal II of the nucleic acid in Figure 10D. In the figures, the inside of a dashed-line box represents a region forming a bulged-G structure. In Figures 10A and 10D, an uppercase letter represents an unmodified RNA nucleotide; a lowercase letter represents a 2'-OMe-modified RNA nucleotide; and an italicized lowercase letter represents a DNA nucleotide. A bar between bases represents complementary pairing, and a white circle represents an important non-complementary pairing.

[Figure 11] Figure 11 illustrates nucleic acids forming a bulged-G (BG) structure. Figure 11A represents the secondary structure of the base sequence of an unmodified BG. Figure 11B represents the secondary structure of the base sequence of a modified BG. In Figures 11A and 11B, the G-A base pairs boxed with solid lines represent the positions of the base pairs shown in Figures 11C and 11D below, respectively. Figure 11C represents the result of the crystal structure analysis on G-A base pairs in the unmodified BG. Figure 11D represents the result of the crystal structure

analysis on G-A base pairs in the modified BG. Figure 11E represents the secondary structure of the base sequence of an unmodified BG. Figure 11F represents the secondary structure of the base sequence of a modified BG. In Figures 11E and 11F, the GU-A triples boxed with solid lines represent the positions of the base triples shown in Figures 11G and 11H below, respectively. Figure 11G represents the result of the crystal structure analysis on the GU-A triple in the unmodified BG. Figure 11H represents the result of the crystal structure analysis on the GU-A triple in the modified BG shown in Figure 11D. In Figures 11A, 11B, 11E, and 11F, a bar between bases represents complementary pairing, and a white circle represents a non-complementary pairing. The 2'-OMeA and 2'-OMeU in Figures 11D and 11H represent A and U that are 2'-OMe-modified RNA nucleotides, respectively. The dG in Figure 11H represents G that is a DNA nucleotide. The dashed lines represent hydrogen bonds.

[Figure 12] Figure 12 illustrates nucleic acids forming a bulged-G (BG) structure. Figure 12A represents the secondary structure of the base sequence of an unmodified BG. Figure 12B represents the secondary structure of the base sequence of a modified BG. In Figures 12A and 12B, the CA base pairs boxed with solid lines represent the positions of the base pairs shown in Figures 12C and 12D below, respectively. Figure 12C represents the result of the crystal structure analysis on the CA base pair in the unmodified BG. Figure 12D represents the result of the crystal structure analysis on the CA base pair in the modified BG. Figure 12E represents the secondary structure of the base sequence of an unmodified BG. Figure 12F represents the secondary structure of the base sequence of a modified BG. In Figures 12E and 12F, the CU base pairs boxed with solid lines represent the positions of the base pairs shown in Figures 12G and 12H below, respectively. Figure 12G represents the result of the crystal structure analysis on the CU base pair in the unmodified BG. Figure 12H represents the result of the crystal structure analysis on the CU base pair in the modified BG. In Figures 12A, 12B, 12E, and 12F, a bar between bases represents complementary pairing, and a white circle represents a non-complementary pairing. The dA in Figure 12D represents that A is a DNA nucleotide. The 2'-OMeU in Figure 12H represents that U is a 2'-OMe-modified RNA nucleotide.

[Figure 13] Figure 13 illustrates nucleic acids forming 2 Kink-turn (KT) structures (dashed-line boxes). Figure 13A represents the secondary structure of the base sequence of an unmodified nucleic acid (unmodified KT) used in a crystal structure analysis. Figure 13B represents the result of the crystal structure analysis on the nucleic acid in Figure 13A. Figure 13C represents the secondary structure of the base sequence of a modified nucleic acid (modified KT) used in the crystal structure analysis. Figure 13D represents the result of the crystal structure analysis on the nucleic acid in Figure 13C. In Figures 13A and 13C, an uppercase letter represents an unmodified RNA nucleotide; a lowercase letter represents a 2'-OMe-modified RNA nucleotide; and an italicized lowercase letter represents a DNA nucleotide. A bar between bases represents complementary pairing, and a white circle represents an important non-complementary pairing.

[Figure 14] Figure 14 illustrates one of the Kink-turn (KT) structures in each nucleic acid in Figure 13. Figure 14A represents the secondary structure of the base sequence comprising 1 Kink-turn structure of an unmodified KT. Figure 14B represents the result of the crystal structure analysis on the nucleic acid in Figure 14A. Figure 14C represents the secondary structure of the base sequence comprising 1 Kink-turn structure of a modified KT. Figure 14D represents the result of the crystal structure analysis on the nucleic acid in Figure 14C. In Figures 14A and 14C, an uppercase letter represents an unmodified RNA nucleotide; a lowercase letter represents a 2'-OMe-modified RNA nucleotide; and an italicized lowercase letter represents a DNA nucleotide. A bar between bases represents complementary pairing, and a white circle represents an important non-complementary pairing.

[Figure 15] Figure 15 illustrates nucleic acids forming a Kink-turn (KT) structure. Figure 15A represents the secondary structure of the base sequence of an unmodified KT. Figure 15B represents the secondary structure of the base sequence of a modified KT. In Figures 15A and 15B, the G-AG triples boxed with solid lines represent the positions of the triples shown in Figures 15C and 15D below, respectively. Figure 15C represents the result of the crystal structure analysis on the G-AG triple in the unmodified KT. Figure 15D represents the result of the crystal structure analysis on the G-AG triple in the modified KT. Figure 15E represents the secondary structure of the base sequence of an unmodified KT. Figure 15F represents the secondary structure of the base sequence of a modified KT. In Figures 15E and 15F, the GAG triples boxed with solid lines represent the positions of the triples shown in Figures 15G and 15H below, respectively. Figure 15G represents the result of the crystal structure analysis on the G-AG triple in the unmodified KT. Figure 15H represents the result of the crystal structure analysis on the G-AG triple in the modified KT. In the figures, the nucleotide bases shown in the figures are shown with solid-line boxes. In Figures 15A, 15B, 15E, and 15F, a bar between bases represents complementary pairing, and a white circle represents a representative non-complementary pairing. The dA and dG in Figures 15D and 15H represent A and G that are DNA nucleotides, respectively. A dashed line represents a hydrogen bond, and a solid line circle represents a functional group on position 2' of ribose of G.

[Figure 16] Figure 16 illustrates nucleic acids forming a Kink-turn (KT) structure. Figure 16A represents the secondary structure of the base sequence of an unmodified KT. Figure 16B represents the secondary structure of the base sequence of a modified KT. In Figures 16A and 16B, the G-A base pairs boxed with solid lines represent the positions of the base pairs shown in Figures 16C and 16D below, respectively. Figure 16C represents the result of the crystal

structure analysis on the bases at the positions shown in Figure 16A in the unmodified KT. Figure 16D represents the result of the crystal structure analysis on the bases at the positions shown in Figure 16B in the modified KT. In Figures 16A and 16B, a bar between bases represents complementary pairing, and a white circle represents a non-complementary pairing. In Figure 16D, the 2'-OMeG represents G that is a 2'-OMe-modified RNA nucleotide. A dashed line represents a hydrogen bond, and a solid line circle represents a functional group on position 2' of ribose of G.

[Figure 17] Figure 17 illustrates nucleic acids forming a tetraloop receptor (TLR) structure together with a tetraloop (TL) structure. Figure 17A represents the secondary structure of the base sequence of an unmodified nucleic acid (unmodified TLR) used in a crystal structure analysis. Figure 17B represents the result of the crystal structure analysis on the nucleic acid in Figure 17A. Figure 17C represents the secondary structure of the base sequence of a nucleic acid in which some RNA nucleotides are replaced by DNA nucleotides (modified TLR) used in the crystal structure analysis. Figure 17D represents the result of the crystal structure analysis on the nucleic acid in Figure 17C. In Figures 17A and 17C, an uppercase letter represents an unmodified RNA nucleotide, and a lowercase letter represents a DNA nucleotide. In Figures 17A and 17C, a bar between bases represents complementary pairing, and a white circle represents an important non-complementary pairing. In addition, the inside of a solid-line box represents a region forming a tetraloop receptor structure, and the inside of a dashed-line box represents a region forming a tetraloop structure.

[Figure 18] Figure 18 illustrates the interaction between a tetraloop (TL) structure and a tetraloop receptor (TLR) structure. Figure 18A represents the state of the binding between a tetraloop structure and a tetraloop receptor structure of unmodified TLR molecules. Figure 18B represents the state of the binding between a tetraloop structure and a tetraloop receptor structure of modified TLR molecules. The inside of a dashed-line box represents a tetraloop structure, while the gray image on the right side of the figure represents a tetraloop receptor structure in the space-filling model.

[Figure 19] Figure 19 illustrates the interaction between a tetraloop (TL) structure and a tetraloop receptor (TLR). Figure 19A represents the result of the crystal structure analysis on the interaction. In the figure, the inside of the solid-line box represents the position of the U-A-A triple shown in Figures 19B and 19C below. Figure 19B represents the result of the crystal structure analysis on the U-A-A triple in an unmodified TLR. Figure 19C represents the result of the crystal structure analysis on the U-A-A triple in a modified TLR. Figure 19D represents the result of the crystal structure analysis on the interaction. In the figure, the inside of the solid-line box represents the position of the U-G-A triple shown in Figures 19E and 19F below. Figure 19E represents the result of the crystal structure analysis on the U-G-A triple in an unmodified TLR. Figure 19F represents the result of the crystal structure analysis on the U-G-A triple in a modified TLR. In Figures 19C and 19F, the dA and dG represent A and G that are DNA nucleotides, respectively. A dashed line represents a hydrogen bond, and a solid line circle represents a functional group on position 2' of ribose with a G base.

[Figure 20] Figure 20 illustrates the interaction between a tetraloop (TL) structure and a tetraloop receptor (TLR) structure. Figure 20A represents the result of the crystal structure analysis on the interaction. In the figure, the inside of the solid-line box represents the position of the C-G-A triple shown in Figures 20B and 20C below. Figure 20B represents the result of the crystal structure analysis on the C-G-A triple in an unmodified TLR. Figure 20C represents the result of the crystal structure analysis on the C-G-A triple in a modified TLR. In Figure 20C, the dG represents G that is a DNA nucleotide. A dashed line represents a hydrogen bond, and a solid line circle represents a functional group on position 2' of ribose with a G base.

[Figure 21] Figure 21 illustrates the base sequences and secondary structures of nucleic acid molecules used in Example 6. A (ROI-U/ASO-A) represents an RNA of interest with a base sequence near the center that is UUU (ROI-U) and a nucleic acid that is completely complementary thereto (ASO-A); B (ROI-A/ASO-A) represents an RNA of interest with a base sequence near the center mutated into AAA (ROI-A), and an ASO-A; C (ROI-U/KT-SKIP) represents an ROI-U and an RNA molecule that induces a Kink-turn structure such that the above-described portion to be mutated is included in a bulge structure (KT-SKIP); and D (ROI-A/KT-SKIP) represents an ROI-A and a KT-SKIP. In the figure, the inside of a dashed-line box represents a region comprising sequences forming a three-dimensional structure as described above. A bar between bases represents complementary pairing, and a white circle represents an important non-complementary pairing.

[Figure 22] Figure 22 illustrates diagrams showing the melting curves of the double-stranded nucleic acids, as shown in Figure 21. Figure 22A represents melting curves of double-stranded nucleic acids between nucleic acids of interest and ASO-A. Figure 22B represents melting curves of double-stranded nucleic acids between nucleic acids of interest and KT-SKIP.

[Figure 23] Figure 23 illustrates the base sequences and secondary structures of nucleic acid molecules used in Example 7. Figure 23A represents the sequence of a target region for hybridization (SO-GFP) and a nucleic acid that is completely complementary thereto (ASO-GFP); Figure 23B represents a SO-GFP and an RNA molecule that induces a tandem GA structure (GA-GFP); and Figure 23C represents an SO-GFP and an RNA molecule that

induces a Kink-turn structure (KT-GFP). In the figures, the upper sequence represents that of a specific target region in a pUC-frGFP mRNA, which is used as a target region for hybridization. The inside of a dashed-line box represents a region comprising sequences forming a three-dimensional structure as described above. A bar between bases represents complementary pairing, and a white circle represents an important non-complementary pairing.

[Figure 24] Figure 24 is a diagram showing the measurement results of frGFP-derived fluorescence with the addition of various nucleic acids. A represents the result without any nucleic acid; B for the addition of an RNA molecule having the same sequence as the target region (SO-GFP); C for the addition of an ASO-GFP; D for the addition of a GA-GFP; and E for the addition of a KT-GFP.

[Figure 25] Figure 25 illustrates a fluorescence-labeled RNA probe (RNA-2AP: the lower sequence) that is hybridized with its target RNA (upper sequence) in Example 8. In the figure, X represents 2-aminopurine (2AP). A white circle represents a non-complementary base pair between guanine (G) and adenine (A) (Sheared G-A base pair), and a vertical line represents a complementary base pair.

[Figure 26] Figure 26A is a diagram showing the measurement results of the fluorescence spectrum of a solution containing RNA-2AP (control), and a solution of an equimolar amount of a target RNA or non-target RNA added relative to RNA-2AP, using an excitation wavelength of 305 nm. Figure 26B is a diagram showing the rate of change in the fluorescence intensity (ΔF) at a wavelength of 370 nm after addition of a target RNA or non-target RNAs to RNA-2AP.

[Figure 27] Figure 27 shows schematic diagrams illustrating the configuration of an artificial nucleic acid of the present invention. Figure 27A represents a schematic arrangement of the domains in the artificial nucleic acid. Figure 27B represents a schematic arrangement of the target domain in the nucleic acid of interest. Figure 27C is a diagram showing the state of hybridization between the artificial nucleic acid in Figure 27A and the nucleic acid of interest in Figure 27B. In the figures, italic letters represent complementary regions in the domains; bold letters represent non-complementary-containing regions in the domains; and the inside of the dashed-line box represents a sequence motif.

[Figure 28] Figure 28 illustrates the base sequences and secondary structures of nucleic acid molecules used in Example 7. Figure 28A represents the sequence of a target region for hybridization (SO-GFP) and a nucleic acid that is completely complementary thereto (ASO-GFP); Figure 28B represents a SO-GFP and an RNA molecule that induces a tandem GA structure (GA-GFP); and Figure 28C represents an SO-GFP and an RNA molecule that induces a Kink-turn structure (KT-GFP). In the figures, the upper sequence represents that in a pUC-frGFP mRNA, which is used as a target region for hybridization. The inside of a dashed-line box represents a region comprising sequences forming a three-dimensional structure as described above. A bar between bases represents complementary pairing, and a white circle represents an important non-complementary pairing.

## Description of Embodiments

## 1. Artificial Nucleic Acid

### 1-1. Summary

The first aspect of the present invention is an artificial nucleic acid. The artificial nucleic acid of the present invention comprises a three-dimensional structure formation-inducing domain as an essential component, and hybridizes with a nucleic acid of interest to form a specific three-dimensional structure. The artificial nucleic acid of the present invention can be an active ingredient in a gene expression inhibiting agent and a nucleic acid detecting agent of the present invention, as described below.

### 1-2. Definition

The terms used herein are defined below.

#### (1) Nucleic Acid

As used herein, the terms "nucleic acids" and "nucleic acid molecules" refer to biopolymers comprising nucleotides as constituent units, which are linked together by phosphodiester bonds. Nucleic acids can be broadly classified into natural nucleic acids and artificial nucleic acids, both of which are encompassed herein.

As used herein, the term "natural nucleic acids" refers to nucleic acids present in nature. For example, DNA and RNA fall under them. Examples of RNA include mRNA and miRNA.

As used herein, the term "miRNAs" means single-stranded noncoding RNAs that are present in organisms, control the expression of specific genes (target genes), and have a base length of 18 to 25.

As used herein, the term "artificial nucleic acids" refers to nucleic acid molecules that are artificially synthesized by biological or chemical synthesis methods. Unless otherwise described, an artificial nucleic acid described herein, for example, may be all composed only of unmodified natural nucleotides, or may contain unnatural nucleotides or modified nucleotides.

The term "nucleotide" refers to a molecule in which a phosphate group is covalently linked to a sugar moiety of a nucleoside. In the case of nucleotide comprising a pentofuranosyl sugar, a phosphate group is typically linked to the hydroxyl group at the 3' position or 5' position of the sugar.

The term "nucleoside" generally refers to a molecule composed of a combination of a base and a sugar. The sugar is usually, but not limited to, composed of a pentofuranosyl sugar. Examples of the pentofuranosyl sugar include ribose and deoxyribose. Examples of the base (nucleobase) include adenine (A), guanine (G), cytosine (C), thymine (T), and uracil (U). As used herein, unless otherwise described, the base may be a modified base or unmodified base.

As used herein, the term "conformation of the sugar moiety" refers to the three-dimensional structure taken by the ribose or deoxyribose in a nucleotide. Main examples of the conformation include C2'-endo and C3'-endo types, which are illustrated below for deoxyribose.

[Chem 1]

C2'-endo

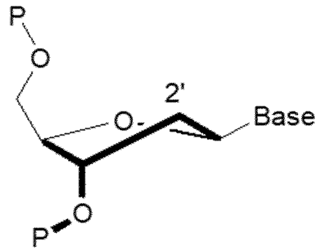

C3'-endo

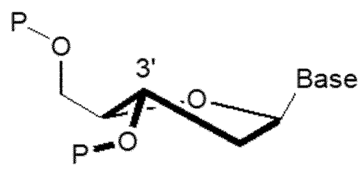

wherein "Base" represents a base moiety, and the numbers represent the position of the carbons in the ribose rings, respectively.

The C2'-endo type is a conformation wherein the 2' carbon projects toward the base side of the ribose ring plane, and in a broader sense, also includes C2'-endo-C3'-exo and C3'-exo types, and is also referred to as South type, S type, and the like. On the contrary, the C3'-endo type is a conformation wherein the 3' carbon projects toward the base side of the ribose ring plane, and in a broader sense, also includes C3'-endo-C2'-exo and C2'-exo types, and is also referred to as North type, N type, and the like. Ribose and deoxyribose can take any of the conformations, while usually ribose tends to take the C3'-endo type and deoxyribose tends to take the C2'-endo type.

As used herein, the term "hydroxy group at the 2' position of ribose" refers to a hydroxy group bound to the carbon at the 2' position of ribose.

As used herein, the term "modification" refers to the replacement of a part of or the entire nucleotide, a constituent unit of a nucleic acid, or of a nucleoside, a constituent unit of a nucleotide, by other atomic groups, or the addition of a functional group or the like. Specific examples include sugar modification, base modification, and modification of phosphodiester bonds.

As used herein, the term "modified nucleotide" refers to a nucleotide, a part of which or the entirety is replaced by other atomic groups, or to which a functional group or the like is added. The term "unmodified nucleotides" refers to nucleotides other than modified nucleotides. In principle, many of natural nucleotides fall into unmodified nucleotides.

Modified nucleotides include both artificially constructed modified nucleotides and natural modified nucleotides. Artificial nucleotides (nucleotide analogs) having similar properties and/or structures to unmodified nucleotides, and artificial nucleotides comprising modified nucleosides or modified bases having similar properties and/or structures to unmodified nucleosides or unmodified bases that are components of unmodified nucleotides are included. Specific examples of the modified nucleosides include abasic nucleoside, arabinonucleoside, 2'-deoxyuridine, α-deoxyribonucleoside, and β-L-deoxyribonucleoside. Specific examples of the modified bases include a 2-oxo(1H)-pyridine-3-yl group, a 5-substituted-2-oxo(1H)-pyridine-3-yl group, a 2-amino-6-(2-thiazolyl)purine-9-yl group, a 2-amino-6-(2-thiazolyl)purine-9-yl group, and a 2-amino-6-(2-oxazolyl)purine-9-yl group.

As used herein, the term "sugar modification" refers to replacement at and/or any change in a sugar moiety of a nucleic acid molecule. Specific examples include 2'-O-methylribose (2'-OMe) obtained by replacing the 2'-hydroxy group by a methoxy group, 2'-O-ethylribose obtained by replacing the 2'-hydroxy group by an ethoxy group, 2'-O-propylribose obtained by replacing the 2'-hydroxy group by a propoxy group, and 2'-O-butylribose obtained by replacing

the 2'-hydroxy group by a butoxy group; 2'-deoxy-2'-fluororibose obtained by replacing the hydroxy group by a fluoro group; and 2'-O-methoxyethylribose (2'-MOE) obtained by replacing the hydroxy group by a 2'-O-methoxy-ethyl group. The hydroxy group may be replaced by a functional group other than hydrocarbon. Specific examples include replacement by H, and halogen elements. The (deoxy)ribose moieties of nucleosides may be replaced by other molecules, such as sugars, morpholino rings, PNAs, and XNAs. Specific examples include replacement of the ribose moiety by arabinose, 2'-fluoro-β-D-arabinose, ribose derivatives obtained by crosslinking the hydroxy group at the 2' position with the carbon atom at the 4' position of ribose, and ribose derivatives obtained by replacing the oxygen at the 4' position of the ribose ring by sulfur. In addition, the examples also include replacement of the oxygen atom on the ribofuranose ring (the oxygen atom at the 4' position of ribose) by sulfur. In particular, nucleotides having crosslinked ribose derivatives are called crosslinked nucleic acids, including 2'-OMe RNAs, 2'-MOE RNAs, LNAs, 2'-O,5'-N BNAs, and 2'-deoxy-trans-3',4'-BNAs.

The term "modified bases" refers to nucleobases other than natural adenine, cytosine, guanine, thymine, or uracil, and the term "base modification" refers to changing into those nucleobases. Examples of modified nucleobases include, but not limited to, 5-methylcytosine, 5-fluorocytosine, 5-bromocytosine, 5-iodocytosine or N4-methylcytosine; N6-methyladenine or 8-bromoadenine; 2-thio-thymine; N2-methylguanine or 8-bromoguanine; and 5-fluorouracil, 5-bromouracil, 5-iodouracil, or 5-hydroxyuracil.

As used herein, the term "non-complementary" means a relationship between nucleobases that do not form a so-called Watson-Crick base pair (natural base pair). As used herein, the term "non-complementary pairing" refers to 2 non-complementary bases facing and interacting with each other by a hydrogen bond, and the term "non-complementary base pair" refers to the 2 non-complementary bases. 2 bases facing each other and forming a base pair refers to "forming a base pair." Specific examples of the non-complementary base pair include Sheared-type base pairs.

As used herein, the term "Sheared type base pairs" is one of the non-complementary base pairs formed between 2 nucleic acid chains and refers to those in which a functional group on the shallow groove edge of one of the bases is involved in a hydrogen bond.

[Chem 2]

Purine base

Pyrimidine base

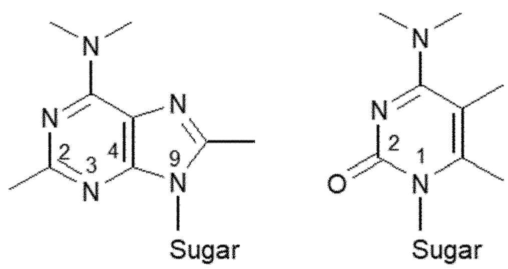

Wherein, the numbers in the ring structures represent the positions where each functional group is present. The term "functional group on the shallow groove edge" refers to functional groups at the 2- to 4-positions for purine base, and a functional group at the 2-position for pyrimidine base. The functional groups on the shallow groove edge are also referred to as functional groups on the sugar edge. Specific examples of the Sheared type base pairs include G-A base pair, A-A base pair, U-C base pair, and C-C base pair.

(2) Mutation

As used herein, the term "mutation" refers to diversity in base sequence present in genome of a population of biological species. In general, discrimination is made such that diversity found in a population at a frequency of 1% or

more as polymorphism, while diversity found at a frequency of less than 1% as mutation. However, in this specification, polymorphism is included in mutation, regardless of the frequency in populations.

As used herein, the term "single nucleotide variant (SNV)" refers to a type of variation mutation in base sequence present in the genome of a population of biological species, with a mutation size of 1 base. In general, the case where the mutation is found at a frequency of 1% or more in a population is particularly referred to as single nucleotide polymorphism (SNP). The single nucleotide variant herein is regardless of the frequency of the variant in a population.

As used herein, the term "insertion-deletion mutation (in-del mutation)" refers to a type of mutation in base sequence present in the genome of a population of biological species, with a mutation size of 2 bases or more and less than 50 bases. The type of mutation is not particularly limited. Examples include mutation due to change in the number of repeats in a short repetitive sequence (typically, 2-7 bases) (STR: short tandem repeat; also referred to as microsatellite polymorphism), and variable number of tandem repeat (VNTR) with the size of the repetitive sequence of less than 50 bases. Regarding the insertion-deletion mutation herein, the frequency of the mutation in a population is not limited.

As used herein, the term "structural variant" refers to a type of mutation in base sequence present in the genome of a population of biological species, with a mutation size of 50 bases or more. Specific examples of the mutation include inversion, translocation, deletion, change in the copy number, and insertion. A structural variant includes, for example, variable number of tandem repeat (VNTR) with the size of the repetitive sequence of 50 bases or more. Regarding the structural variant herein, the frequency of the variant in a population is not limited.

(3) Secondary Structure

As used herein, the term "secondary structure" refers to base pairs formed between two nucleic acid chains and overhang of bases. In particular, secondary structures, when used in a narrower sense, refer to structures comprising characteristic shapes. Specifically, secondary structures include, for example, bulge structure, loop structure, and stem structure. The bulge structure will be described below as a representative secondary structure.

As used herein, the term "bulge structure" refers to a three-dimensional structure of a portion that forms non-complementary base pairs between the two nucleic acid chains, and a portion(s) in one or both chain(s) in which bases overhang partially or entirely toward the outside of the double-stranded nucleic acid molecule, in secondary structures of double-stranded nucleic acids hybridizing as a whole. As used herein, bulge structures also include, for example, any of bulge loop, internal loop, and multi-branch loop structures. The actual three-dimensional structure of a region with a bulge structure is not necessarily bulged compared to other regions.

(4) Three-dimensional Structure

As used herein, the term "three-dimensional structure" means any other conformation (spacial structure) than the double helix structure formed by a completely complementary double-stranded nucleic acid molecule.

As used herein, the term "specific three-dimensional structure" refers to a three-dimensional structure of interest induced by the artificial nucleic acid of the present invention.

As used herein, the term "functional three-dimensional structure" refers to a three-dimensional structure having at least one function in a living body. For example, three-dimensional structures in ribozymes, aptamers, transfer RNAs, ribosomal RNAs (rRNAs), and the like, and hairpin structures are functional three-dimensional structures. On the contrary, for example, a three-dimensional structure formed incidentally by a single-stranded RNA and unrelated to the function of the RNA is not a functional three-dimensional structure.

As used herein, the term "inducing a three-dimensional structure" refers to the hybridization of an artificial nucleic acid with a nucleic acid of interest to form a three-dimensional structure. For example, a three-dimensional structure that is found in rRNA is induced.

As used herein, the term "sequence motif' refers to a motif that may induce a specific three-dimensional structure and contains sequence information composed of double strands. In the schematic view shown in Figure 27C, a sequence motif corresponds to the inside of the dashed-line box. Each of the double strands forming the sequence motif comprises a complementary region (italics in Figure 27C), one or both of the nucleic acid chains further comprise(s) a non-complementary-containing region (bold letters in Figure 27C).

As used herein, the term "consensus sequence" refers to the sequence information preserved among a plurality of sequences that form the same three-dimensional structure.

As used herein, the term "complementary regions" refers to regions consisting of mutually complementary sequences. For example, in the schematic view shown in Figure 27C, a base shown in italics corresponds to a complementary region in each nucleic acid chain.

As used herein, the term "non-complementary-containing regions" refers to regions of 2 bases or more comprising mutually non-complementary sequences. For example, in the schematic view shown in Figure 27C, a base shown in a bold letter corresponds to a complementary region in each nucleic acid chain. The above-described region hereby

comprises non-complementary sequences at its both ends.

Specific examples of three-dimensional structures include bulged-G structure, Kink-turn structure, Reverse Kink-turn structure, 5S loop E structure, C-loop structure, and tandem GA structure. Specific examples of three-dimensional structures and sequence motifs will be described below.

(i) Bulged-G Structure

As used herein, the term "Bulged-G structure" refers to a three-dimensional structure where both nucleic acid chains form a bulge structure, which has a base triple (a set of 2 bases from one chain and 1 base from the other chain) flanked by a Sheared type G-A base pair and a bulge structure as characteristics in the sequence. The appearance of an exemplary bulged-G structure is shown in Figure 1A. Each bulge structure comprised in a bulged-G structure is typically composed of 3-5 bases. Bulged-G structure is also known by many other names, and is also called, for example, sarcin/ricin loop structure, α-sarcin and ricin-sensitive loop structure, SRL, or S motif. The sequence motif of a bulged-G structure is not particularly limited, and examples include the motifs in Figure 1B.

For example, in the motif 1 in Figure 1B (alfa chain: SEQ ID NO: 1 (5'-NNNGUAN-3': wherein N is A, C, G, or U), beta chain: SEQ ID NO: 2 (5'-NGANNN-3': wherein N is A, C, G, or U)), the bases at α3-, α4-, and β3-positions form a base triple, while in the motif 2, the bases at α4-, α5-, and β4-positions form a base triple. In addition, in the motifs 1 and 2, the bases at α5- and β4- positions, and at α6- and β5-positions form Sheared type G-A base pairs, respectively. For example, in the motif 1, the regions in each nucleic acid chain inside the dashed-line box may be set as non-complementary-containing regions for the respective nucleic acid chains.

Specific examples of a sequence motif for the bulged-G structure include sequence motifs described in Correll, C. C., et al., Nucleic Acids Research, 2003, Vol. 31, No. 23, 6806-6818; and Correll, C. C., et al., Proc. Natl. Acad. Sci., 1998, Vol. 95, pp. 13436-13441. Bulged-G structures have been reported to have functions, for example, to be recognized by proteins such as α-sarcin and involved in RNA cleavage (see, for example, Gluck, A. and Wool, I. G., J. Mol. Biol., 1996, 256: 838-848).

(ii) Kink-turn Structure

As used herein, the term "Kink-turn structure" refers to a three-dimensional structure in which the double-stranded nucleic acid is bent due to a bulge structure formed in one chain, which has a bulge structure followed by a Sheared type G-A base pair as characteristics in the sequence. The appearance of an exemplary Kink-turn structure is shown in Figure 2A. The bulge structure is typically composed of 3 bases or more. Guanine (G) present on the 3' side of the bulge structure in the chain forming the bulge structure forms the Sheared type G-A base pair together with adenine (A) in the other chain. The sequence motif of the Kink-turn structure is not particularly limited, and examples include the motifs in Figures 2B and 2C.

Kink-turn structures include standard Kink-turn structures, for example, as shown in Figure 2B (alfa chain: SEQ ID NO: 3 (5'-NNNNGAN-3': wherein N is A, C, G, or U), beta chain: SEQ ID NO: 4 (5'-NGAN-3': wherein N is A, C, G, or U)), and non-standard Kink-turn structures having variation at the Sheared type G-A base pair portion, such as the structure shown in Figure 2C, both of which are included by the Kink-turn structure herein. In addition, in the standard motif 1, the bases at α4- and β1- positions, and at α5- and β2-positions form Sheared type G-A base pairs. For example, in the standard motif 1, the regions in each nucleic acid chain inside the dashed-line box may be set as non-complementary-containing regions for the respective nucleic acid chains.

Specific examples of a sequence motif for the Kink-turn structure include sequence motifs described in Huang, L. and Lilley, D. M., Journal of Molecular Biology, 2016, 428 (5 Part A): 790-801; and Huang, L. and Lilley, D. M., Quarterly Reviews of Biophysics, 2018, 51(e5). A Kink-turn structure is known to be recognized by, for example, proteins belonging to the L7Ae family.

(iii) Reverse Kink-turn Structure

As used herein, the term "Reverse Kink-turn structure" refers to a three-dimensional structure in which the double-stranded nucleic acid is bent toward the opposite direction compared to the Kink-turn structure. The appearance of an exemplary Reverse Kink-turn structure is shown in Figure 3A. The biggest difference in the sequence from the Kink-turn structure is that an A-A base pair, rather than a Sheared type G-A base pair, is adjacent to the bulge structure. The sequence motif of the Reverse Kink-turn structure is not particularly limited, and examples include the motifs in Figure 3B.

The motif in Figure 3B is different compared to the standard motif 1 of the Kink-turn structure (Figure 2B) in that the base pair between the bases at the α4- and β1-positions is an A-A base pair. For example, in the motif in Figure 3B (alfa chain: SEQ ID NO: 5 (5'-NNNNAAN-3': wherein N is A, C, G, or U), beta chain: SEQ ID NO: 6 (5'-NGAN-3':

wherein N is A, C, G, or U)), the regions in each nucleic acid chain inside the dashed-line box may be set as non-complementary-containing regions for the respective nucleic acid chains. In this specification, sequence motifs of the Reverse Kink-turn structure include a wide variety of sequence motifs similar to the Kink-turn structure, with the motif 1 (Figure 3B) as the standard sequence motif. Specific examples of a sequence motif for the Reverse Kink-turn structure include sequence motifs described in Strobel, S. A., et al., Rna, 2004, 10(12), 1852-1854.

(iv) 5S Loop E Structure

As used herein, the term "5S loop E structure" refers to a structure found in 5S ribosomal RNA of prokaryotes and having a double helix structure that is partially twisted, which has a characteristic in the sequence is that it is composed of 3 or more non-complementary base pairs including a non-complementary A-U base pair and a Sheared type G-A base pair. The appearance of an exemplary 5S loop E structure is shown in Figure 4A. Typically, the minimum unit of the 5S loop E structure is typically composed of 3 base pairs. The 5S loop E structure is also called the loop E structure. A sequence motif of the 5S loop E structure may comprise a plurality of submotifs, which are simply referred to as sequence motifs of the 5S loop E structure herein. The submotif is typically composed of 3 base pairs. The sequence motif of the 5S loop E structure is not particularly limited, and examples include the motifs in Figures 4B and 4C.

The 5S loop E structure is broadly classified into the simple type and complex type. The simple type is a structure composed of 3 base pairs, for example, as shown in the simple motifs 1-3 in Figure 4B. The complex type includes, for example, a structure comprising a plurality of simple motifs as shown in the complex motifs 1 and 2 in Figure 4C, and a structure comprising an extra base pair in the middle as shown in the complex motif 3 in Figure 4C. For example, the complex motif 1 comprises 2 submotifs composed of bases at the $\alpha$1- to $\alpha$3-positions and at the $\beta$1- to $\beta$3-positions, and of bases at the $\alpha$5- to $\alpha$7-positions and at the $\beta$5- to $\beta$7-positions. For example, in the simple motif 1 (alfa chain: SEQ ID NO: 7 (5'-NGUAN-3': wherein N is A, C, G, or U), beta chain: SEQ ID NO: 8 (5'-NGAUN-3': wherein N is A, C, G, or U)), the regions in each nucleic acid chain inside the dashed-line box may be set as non-complementary-containing regions for the respective nucleic acid chains.

Specific examples of a sequence motif for the 5S loop E structure include sequence motifs described, for example, in Correll, C. C., et al., Cell, 1997, 91(5), 705-712; and Leontis, N. B. and Westhof, E. Rna, 1998, 4(9), 1134-1153. It is known that cations such as $Mg^+$ bind to the 5S loop E structure and that double-strand breaks due to nucleases are likely to occur in the vicinity of the 5S loop E structure.

(v) C-loop Structure

As used herein, the term "C-loop structure" refers to a three-dimensional structure having bulge structures formed in both nucleic acid chains, and having a double helix structure that is partially twisted, which has characteristics in the sequence of having the bulge structures followed by U-A base pairs. The appearance of an exemplary C-loop structure is shown in Figure 5A. Typically, the bulge structures in the nucleic acid chains have different sizes, and for example, the longer bulge structure comprises C. The sequence motif of the C-loop structure is not particularly limited, and examples include the motifs in Figure 5B.

A standard C-loop structure is, for example, a structure as shown in the motif 1, having a larger bulge structure composed of 3 bases and a smaller bulge structure composed of 1 base. However, for example, the smaller bulge structure may be composed of 2 bases or more as shown in the motif 2, and the larger bulge structure may also be composed of 4 bases or more as shown in the motif 3. For example, in the motif 1 (alfa chain: SEQ ID NO: 9 (5'-NCACU-3': wherein N is A, C, G, or U), beta chain: SEQ ID NO: 10 (5'-ANN-3': wherein N is A, C, G, or U)), the regions at the $\alpha$1- to $\alpha$3-positions in the alfa chain and the region at the $\beta$1-position in the beta chain may be set as non-complementary-containing regions for the respective nucleic acid chains.

Specific examples of a sequence motif for the C-loop structure include sequence motifs described, for example, in Klein, D. J., et al., Journal of molecular biology, 2004, 340(1), 141-177; and Lescoute, A., et al., Nucleic acids research, 2005, 33(8), 2395-2409. With respect to the C-loop structure, it is known that double-stranded nucleic acids having C-loop structures are likely to form a complex with each other.

(vi) Tandem GA Structure

As used herein, the term "tandem GA structure" refers to a three-dimensional structure having bulges formed in both nucleic acid chains, which has characteristics in the sequence of comprising 2 consecutive Sheared type G-A base pairs. The appearance of an exemplary tandem GA structure is shown in Figure 6A. The tandem GA structure is also called the GA/AG loop. The sequence motif of the tandem GA structure is not particularly limited, and examples include the motif in Figure 6B. For example, in the motif 1 (alfa chain: SEQ ID NO: 11 (5'-NGAN-3': wherein N is A, C, G, or U), beta chain: SEQ ID NO: 12 (5'-NGAN-3': wherein N is A, C, G, or U)), the regions in each nucleic acid chain

inside the dashed-line box may be set as non-complementary-containing regions for the respective nucleic acid chains.

Specific examples of a sequence motif for the tandem GA structure include sequence motifs described, for example, in Jang, S. B., et al., Acta Crystallographica Section D: Biological Crystallography, 2004, 60(5), 829-835.

1-3. Configuration

An artificial nucleic acid of the present invention comprises a three-dimensional structure formation-inducing domain as an essential component and optionally, a hybridizable domain. The domains will be described in detail below.

1-3-1. Three-dimensional Structure Formation-inducing Domain

The term "three-dimensional structure formation-inducing domain" means a domain that forms a three-dimensional structure together with a target domain in a nucleic acid of interest.

As used herein, the term "nucleic acid of interest" refers to a nucleic acid to which the artificial nucleic acid can hybridize. The term "target domain" refers to a region in a nucleic acid of interest, containing the information of one sequence of a sequence motif composed of double strands forming a specific three-dimensional structure (Figure 27B). In particular, the nucleic acid of interest herein is a nucleic acid that does not form a functional three-dimensional structure. The nucleic acid of interest is not particularly limited as long as it is a nucleic acid that does not form a functional three-dimensional structure, and specific examples thereof include any RNAs, such as mRNA and microRNA (miRNA). The nucleic acid of interest may be a natural nucleic acid or an artificial nucleic acid. 2 or more types of nucleic acids of interest may be present. For example, an artificial nucleic acid may be capable of specifically hybridizing with 1 type of nucleic acid as the nucleic acid of interest, may be capable of nonspecifically hybridizing with 2 or more types of nucleic acids, or may be capable of concurrently hybridizing with 2 or more types of nucleic acids. In addition, the artificial nucleic acids of the present aspect also include, for example, artificial nucleic acids that, even if they are capable of hybridizing with 2 or more types of nucleic acids, induce three-dimensional structures only when they hybridize with some of the nucleic acids.

The conditions under which a three-dimensional structure is induced are not particularly limited as long as the conditions allow hybridization between a nucleic acid of interest and an artificial nucleic acid. For example, a three-dimensional structure may be induced *in vivo* or *ex vivo.* Three-dimensional structure(s) may be induced in one or both nucleic acid chains.

The three-dimensional structure to be induced is not particularly limited. The entirety or a part of the three-dimensional structures found in nucleic acids forming functional three-dimensional structures, such as rRNA, or three-dimensional structures that are not confirmed to exist in nature may be induced. Furthermore, the type and number of the three-dimensional structure to be induced are not particularly limited. For example, 1 artificial nucleic acid may induce a plurality of a single type of three-dimensional structures, or may induce multiple types of three-dimensional structures in combination. The single type of three-dimensional structure hereby includes 1 three-dimensional structure complex composed of a combination of a plurality of the three-dimensional structures. For example, an aptamer may be induced as 1 three-dimensional structure, or a three-dimensional structure, in which a plurality of aptamers are fused, may be induced as 1 three-dimensional structure.

As described above, a three-dimensional structure of interest induced by an artificial nucleic acid of the present invention is referred to as a specific three-dimensional structure. Specific examples of the specific three-dimensional structure include three-dimensional structures based on double strands (three-dimensional structures other than the "three-dimensional structures based on a turn of a nucleic acid" described below: e.g., Kink-turn structure, bulged-G structure, Reverse Kink-turn structure, 5S loop E structure, C-loop structure, tandem GA structure, tetraloop receptor structure, Hook-turn structure, $\Omega$-turn structure, and $\pi$-turn structure); three-dimensional structures based on a turn of a nucleic acid (three-dimensional structures composed only of the turn portion of a single-stranded nucleic acid: e.g., tetraloop structures, including GNRA tetraloop, UNCG tetraloop, and CUUG tetraloop); and combinations thereof. Preferably, the specific three-dimensional structure comprise a three-dimensional structure based on double strands. Preferably, specific examples of the specific three-dimensional structure comprise 1 or more selected from the group consisting of Kink-turn structure, bulged-G structure, Reverse Kink-turn structure, 5S loop E structure, C-loop structure, and tandem GA structure. The three-dimensional structure to be induced may be, for example, one without any known consensus sequence, or one that has not been named. Furthermore, the three-dimensional structure to be formed may be stable or unstable. For example, a three-dimensional structure that is only formed under specific conditions may be formed, or a three-dimensional structure formed may be changed according to the conditions.

The three-dimensional structures may be asymmetrically formed by a nucleic acid of interest and the artificial nucleic acid. In that case, either nucleic acid may form the larger three-dimensional structure. For example, in the case of the standard motif 1 illustrated as a sequence motif of the Kink-turn structure (Figure 2B), the alfa chain may be a nucleic acid of interest, and the beta chain may be an artificial nucleic acid, or vice versa.

Whether a three-dimensional structure of interest can be formed can be determined using methods known in the art. For example, in the case where a sequence motif or a consensus sequence known to form a specific three-dimensional structure is used, the specific three-dimensional structure can be estimated to be formed. In this case, it is not required to confirm whether the specific three-dimensional structure is actually formed. In order to confirm whether a specific three-dimensional structure is actually formed by an artificial nucleic acid, it may be confirmed, for example, by *in silico* analysis, by structural analysis, by observation of events that occur specifically for a specific three-dimensional structure. For the *in silico* analysis, for example, a program for predicting three-dimensional structures known in the art may be used, such as RNAComposer, RNAMotifScan, 3dRNA, ModeRNA, MacroMoleculeBuilder, NAST, iFoldRNA, Vfold3D, SimRNA, or a combination thereof. Alternatively, a structural analysis of a complex of a nucleic acid having the same base sequence as the entirety or a portion of a nucleic acid of interest and an artificial nucleic acid allows the observation of the three-dimensional structure. In this case, the conditions and methods used in the crystallization and structural analysis are not particularly limited. Specific examples of the methods include neutron crystallographic analysis, small-angle neutron scattering (SANS), nuclear magnetic resonance (NMR), X-ray crystallographic analysis, small-angle X-ray scattering, cryoelectron microscopy, and a combination thereof. Furthermore, the formation of the three-dimensional structure can be confirmed by the observation of events that occur specifically for a specific three-dimensional structure. For example, for the Kink-turn structure, it is known that proteins belonging to the L7Ae family or the like are bound. Therefore, the formation of a Kink-turn structure can be confirmed by detecting the binding of the proteins. Events that can occur upon formation of the three-dimensional structures are exemplified in the sections on the definition of the three-dimensional structures.

The term "three-dimensional structure formation-inducing domain" means a domain that forms a three-dimensional structure together with a target domain in a nucleic acid of interest (Figure 27A). A three-dimensional structure formation-inducing domain and a target domain compose a sequence motif for a specific three-dimensional structure (Figure 27C). In a sequence motif, the three-dimensional structure formation-inducing domain and the target domain comprise a complementary region consisting of mutually complementary base sequences (italics in Figure 27C), and the three-dimensional structure formation-inducing domain and/or the target domain further comprise a non-complementary-containing region with 1 or more bases comprising mutually non-complementary base sequences (bold letters in Figure 27C). The non-complementary-containing region hereby comprises non-complementary sequences at its both ends. In other words, the region is one between a non-complementary base at the most 5'-side and a non-complementary base at the most 3'-side of the domain to which the region belongs. In the case where a three-dimensional structure formation-inducing domain and/or a target domain comprises a non-complementary-containing region, complementary region(s) is/are adjacent to one or both side of the non-complementary-containing region in the domain comprising the non-complementary-containing region. For example, a three-dimensional structure formation-inducing domain may be located at an end of an artificial nucleic acid, and in that case, the base pair located at the end may be non-complementary.

The number of bases composing a complementary region is not particularly limited. Specific examples of the number of bases include 1 base or more, 2 bases or more, 3 bases or more, 4 bases or more, 5 bases or more, 10 bases or more, 20 bases or more, or 30 bases or more. The base sequence of a complementary region is not particularly limited.

The number of complementary regions comprised in a three-dimensional structure formation-inducing domain and a target domain is not particularly limited. The number of complementary regions comprised in a three-dimensional structure formation-inducing domain and the number of complementary regions comprised in a target domain do not need to be the same. Complementary regions are consisted of mutually complementary base sequences, and thus the total number of bases comprised in complementary regions in a three-dimensional structure formation-inducing domain and a complementary region are usually equal.

In the case where a three-dimensional structure formation-inducing domain or a target domain comprises a plurality of complementary regions, a non-complementary-containing region is usually located between the plurality of complementary regions. Preferably, a three-dimensional structure formation-inducing domain and/or a target domain comprises a plurality of complementary regions, and a non-complementary-containing region is located between the plurality of complementary regions.

The size of a non-complementary-containing region is not particularly limited as long as it is 1 base or more. The specific number of the bases may be, for example, 1 to 1,000 bases, 1 to 500 bases, 1 to 400 bases, 1 to 200 bases, 1 to 100 bases, 1 to 80 bases, 1 to 60 bases, 1 to 40 bases, 1 to 30 bases, 2 to 20 bases, 2 to 15 bases, 2 to 10 bases, 2 to 9 bases, 2 to 8 bases, or 2 to 7 bases. The base sequence of a non-complementary-containing region is not particularly limited as long as it comprises non-complementary base sequences at its both ends. Non-complementary-containing regions may comprise mutually complementary base sequences. For example, in cases where a non-complementary-containing region in one nucleic acid chain is composed of 1 base, the non-complementary base can be considered as the non-complementary bases at both ends of the non-complementary-containing region. For example, in cases where a non-complementary-containing region in one nucleic acid chain is composed of 3 bases, the 2 bases at both ends are non-complementary bases, but the 1 base located between them may be a non-complementary base

or a complementary base.

The number of non-complementary-containing regions comprised in a three-dimensional structure formation-inducing domain and a target domain is not particularly limited. At least any one of the three-dimensional structure formation-inducing domain and the target domain is required to comprise at least 1 non-complementary-containing region. For example, in a secondary structure of a double-strand of an artificial nucleic acid and a nucleic acid of interest, having the artificial nucleic acid with a bulge structure and the nucleic acid of interest without a base that matches the bulge structure portion, only the three-dimensional structure formation-inducing domain comprises 1 non-complementary-containing region. Non-complementary-containing regions are core regions of the three-dimensional structure, and thus the number of non-complementary-containing regions depends on the type and number of the three-dimensional structure to be induced.

A three-dimensional structure formation-inducing domain and a target domain compose a sequence motif forming a specific three-dimensional structure. The sequence motif does not need to comprise the entire three-dimensional structure formation-inducing domain and/or target domain. For example, the entire sequence motif may be comprised in the non-complementary-containing region, or a part of the sequence motif may be comprised in the non-complementary-containing region. The sequence motif comprised in a three-dimensional structure formation-inducing domain and a target domain is not particularly limited. As a sequence motif, for example, a consensus sequence may be used, or a portion or the entirety of the base sequence of a nucleic acid forming a functional three-dimensional structure may also be used directly or with partial alteration. Specific sequence motifs for each of the three-dimensional structures include, for example: a Kink-turn structure is composed of SEQ ID NO: 3 (5'-NNNNGAN-3': wherein N is A, C, G, or U) and SEQ ID NO: 4 (5'-NGAN-3': wherein N is A, C, G, or U) (the 1st N in SEQ ID NO: 3 and the 4th N in SEQ ID NO: 4, and the 7th N in SEQ ID NO: 3 and the 1st N in SEQ ID NO: 4 are complementary to each other); a bulged-G structure is composed of SEQ ID NO: 1 (5'-NNNGUAN-3': wherein N is A, C, G, or U) and SEQ ID NO: 2 (5'-NGANNN-3': wherein N is A, C, G, or U) (the 1st N in SEQ ID NO: 1 and the 6th N in SEQ ID NO: 2, and the 7th N in SEQ ID NO: 1 and the 1st N in SEQ ID NO: 2 are complementary to each other); a Reverse Kink-turn structure is composed of SEQ ID NO: 5 (5'-NNNNAAN-3': wherein N is A, C, G, or U) and SEQ ID NO: 6 (5'-NGAN-3': wherein N is A, C, G, or U) (the 1st N in SEQ ID NO: 5 and the 4th N in SEQ ID NO: 6, and the 7th N in SEQ ID NO: 5 and the 1st N in SEQ ID NO: 6 are complementary to each other); a 5S loop E structure composed of SEQ ID NO: 7 (5'-NGUAN-3': wherein N is A, C, G, or U) and SEQ ID NO: 8 (5'-NGAUN-3': wherein N is A, C, G, or U) (the 1st N in SEQ ID NO: 7 and the 5th N in SEQ ID NO: 8, and the 5th N in SEQ ID NO: 7 and the 1st N in SEQ ID NO: 8 are complementary to each other); a C-loop structure is composed of SEQ ID NO: 9 (5'-NCACU-3': wherein N is A, C, G, or U) and SEQ ID NO: 10 (5'-ANN-3': wherein N is A, C, G, or U) (the 1st N in SEQ ID NO: 9 and the 4th N in SEQ ID NO: 10, and the 6th N in SEQ ID NO: 9 and the 1st N in SEQ ID NO: 10 are complementary to each other); or a tandem GA structure is composed of SEQ ID NO: 11 (5'-NGAN-3': wherein N is A, C, G, or U) and SEQ ID NO: 12 (5'-NGAN-3': wherein N is A, C, G, or U) (the 1st N in SEQ ID NO: 11 and the 4th N in SEQ ID NO: 12, and the 4th N in SEQ ID NO: 11 and the 1st N in SEQ ID NO: 12 are complementary to each other).

1-3-2. Hybridizable Domain

An artificial nucleic acid of the present invention may comprise a hybridizable domain(s) adjacent to one or both side(s) of the three-dimensional structure formation-inducing domain. The hybridizable domain is a domain that can hybridize with a nucleic acid of interest. In cases where 2 or more hybridizable domains exist, the individual domains are referred to as hybridizable subdomains. For example, a hybridizable domain is composed of 2 subdomains adjacent to both sides of the three-dimensional structure formation-inducing domain. The size of the hybridizable domain may be 6 bases to 120 bases. Specifically, the size may be, for example, 6 bases or more, 7 bases or more, 8 bases or more, 9 bases or more, 10 bases or more, 11 bases or more, 12 bases or more, 13 bases or more, 14 bases or more, 15 bases or more, 16 bases or more, 17 bases or more, 18 bases or more, 19 bases or more, 20 bases or more, 21 bases or more, or 22 bases or more, and may be, for example, 120 bases or less, 110 bases or less, 100 bases or less, 90 bases or less, 80 bases or less, 70 bases or less, 60 bases or less, 50 bases or less, 40 bases or less, or 30 bases or less. The base sequence of the hybridizable domain is not particularly limited as long as hybridization with the nucleic acid of interest is possible. Preferably, the hybridization with the nucleic acid of interest may be performed under high stringent conditions.

As used herein, the term "hybridize" refers to the formation of a double strand by polynucleotides having base sequences completely or partially complementary to each other. The hybridization conditions are not particularly limited, and may be, for example, various stringent conditions such as low stringent conditions and high stringent conditions. The term "low stringent conditions" means conditions under which nucleic acids are likely to hybridize. The low stringent conditions refer to a condition with low-temperature and high-salt concentration during washing after hybridization. For example, the low stringent conditions during washing after hybridization are conditions for washing using, for example, a buffer containing 5 × SSC and 0.1% SDS at 42°C to 50°C. The term "high stringent conditions" means conditions

under which nonspecific hybrids are not formed. As used herein, the low-salt concentration specifically refers to, for example, 15-750 mM, and preferably 15 to 500 mM, 15 to 300 mM, or 15 to 200 mM. As used herein, the high temperature is specifically, for example, 50 to 68°C, or 55 to 70°C. Specific high stringent conditions may be, for example, washing with 0.1 × SSC and 0.1% SDS at 65°C. 1 × SSC hereby contains 150 mM sodium chloride and 15 mM sodium citrate.

Whether being hybridizable can be determined by using methods known in the art. For example, it may be determined based on the base identity. Usually, a second nucleic acid having a base sequence with a certain level or more of base identity with a sequence completely complementary to the base sequence of a first nucleic acid can hybridize with the first nucleic acid. Specifically, for example, hybridization can be made in cases where the base identity is 70% or more, 80% or more, 90% or more, 95% or more, 96% or more, 97% or more, 98% or more, 99% or more, or 100%. As used herein, the term "base identity" refers to the percentage (%) of identical bases in one polynucleotide relative to the total base number of the other polynucleotide when the base sequences of the two polynucleotides are aligned, and gaps are introduced into either of the base sequences as necessary to maximize the base agreement therebetween. The % identity can be easily determined by using known programs such as searching with a homology searching program BLAST (Basic local alignment search tool; Altschul, S. F. et al., J. Mol. Biol., 215, 403-410, 1990) and the like. Usually, a second nucleic acid having a base sequence completely complementary to the base sequence of a first nucleic acid, in which 1 or plural bases are substituted by other bases, can hybridize with the first nucleic acid. As used herein, plural refers to 2 or more, specifically for example, 2 to 30, 2 to 14, 2 to 12, 2 to 10, for example, 2 to 8, 2 to 6, 2 to 5, 2 to 4, or 2 to 3.

An artificial nucleic acid of the present invention may comprise any additional sequence with any length adjacent to one or both end(s) of the three-dimensional structure formation-inducing domain and/or hybridizable domain.

1-3-3. Modification

An artificial nucleic acid of the present aspect may comprise 1 or more modified nucleotides. The modification to be introduced is not particularly limited. For example, phosphodiester bonds, sugar moieties, and/or bases in a hybridizable domain can be modified.

The three-dimensional structure formation-inducing domain may comprise 1 or more modified nucleotides. In cases where the modified nucleotides in the three-dimensional structure formation-inducing domain include modification of a sugar moiety, the modification preferably has no effect on the conformation of the sugar moiety during the formation of a three-dimensional structure. For example, in cases where a modified nucleotide is introduced at the position of a nucleotide where the conformation of the sugar moiety during the formation of a three-dimensional structure is C3'-endo type, the modified nucleotide preferably can take the C3'-endo conformation. Specific examples of the modified nucleotide that can take the C3'-endo conformation include 2'-OMe RNA, 2'-MOE RNA, LNA, and DNA. For example, in cases where a modified nucleotide is introduced at the position of a nucleotide where the conformation of the sugar moiety during the formation of a three-dimensional structure is C2'-endo type, the modified nucleotide preferably can take the C2'-endo conformation. Specific examples of the modified nucleotide that can take the C2'-endo conformation include 2'-O,5'-N BNA, 2'-deoxy-trans-3',4'-BNA, and DNA. For example, modification of a phosphodiester bond or a base generally has no effect on the conformation of the sugar moiety.

In cases where a modified nucleotide is introduced at the position of a nucleotide where the hydroxy group at the 2' position of ribose is involved in hydrogen bonding during the formation of a three-dimensional structure, the modified nucleotide preferably can form such a hydrogen bond, or has a substituent that is not bulky at the 2' position of ribose. The hydroxy group involved in hydrogen bonding during the formation of a three-dimensional structure may have an electron-donating or electron-accepting aspect, or both aspects. Specific examples of the substituent that is not bulky include a hydrogen group, halogen groups (e.g., a fluoro group, a chloro group, and a bromo group), a methyl group, an amino group, and a cyano group.

The conformation of the sugar moiety and the presence of hydrogen bond formation during the formation of a three-dimensional structure can be determined by using methods known in the art. For example, they may be investigated visually or using software such as 3DNA, based on the crystal structure analysis results of a three-dimensional structure downloaded from database such as Nucleic Acid Database or Protein Data Bank. The sections of defining the three-dimensional structures exemplify the conformation of the sugar moieties and the presence of hydrogen bond formation.

The artificial nucleic acid of the present aspect can stably hybridize with a nucleic acid of interest while being in a state forming a specific three-dimensional structure. In particular, in cases where a three-dimensional structure can be formed regardless of the base sequence of a portion of the non-complementary-containing region, the base sequence of the portion may have mutations. For example, the artificial nucleic acid of the present aspect can form a double strand together with a region in a nucleic acid of interest in which individual variation due to mutation exists, with comparable stability regardless of individual differences in its base sequence. The type of the mutation is not particularly limited, and examples include single nucleotide variant, insertion-deletion mutation, structural variant, or a combination thereof. Specifically, for example, considering the alfa chain as a target domain in the standard motif 1 of the Kink-turn structure

(Figure 2B), mutations can be comprised at the α1-, α2-, or α3-position, or 1 or more thereof.

2. Gene Expression Inhibiting agent

2-1. Summary

A second aspect of the present invention is a gene expression inhibiting agent. The gene expression inhibiting agent of the present invention comprises the artificial nucleic acid described in the first aspect as an active ingredient, and has the effect of reducing gene expression in a subject. The gene expression inhibiting agent of the present invention can be used to more efficiently inhibit the expression of genes that have been difficult to stably inhibit.

2-2. Configuration

2-2-1. Components

The components of the gene expression inhibiting agent of the present invention will be described. The gene expression inhibiting agent of the present invention comprises an artificial nucleic acid as an essential component. The components will be described in detail below.

As used herein, the term "gene expression inhibiting agent" refers to an agent having a gene expression inhibiting effect.

As used herein, the term "gene expression inhibition" means inhibition of the expression of transcription products and/or the expression of a protein from a gene of interest by an artificial nucleic acid. As used herein, the term "transcription products" refers to any RNAs synthesized from a gene region in DNA by an RNA polymerase. Specific examples include mRNAs transcribed from the gene (including, for example, mature mRNAs, mRNA precursors, and mRNAs without base modification), noncoding RNAs (ncRNAs) such as miRNAs, long noncoding RNAs (lncRNAs), and natural antisense RNAs. The method of expression inhibition is not particularly limited. For example, the expression inhibition may be performed in accordance with conventional expression inhibition methods, such as methods using RNA molecules or precursors thereof having RNA interference (RNAi) effects, such as siRNAs, shRNAs, and double-stranded RNAs; and methods using nucleic acid molecules that inhibit the translation, such as miRNAs, antisense nucleic acids (e.g., antisense DNAs and antisense RNAs), and ribozymes.

The artificial nucleic acid is as described in the first aspect, and thus a detailed explanation is omitted.

The artificial nucleic acid of the present invention is improvement of methods of inhibiting the gene expression by nucleic acids, and thus the effect of inhibiting the gene expression herein includes, for example, all effects known to be obtained by conventional gene expression inhibiting methods. Specific examples include inhibition or reduction of gene expression or the amount of transcription products expressed; inhibition of translation; RNA editing or splicing function-modifying effects (including, for example, splicing switch, exon inclusion, and exon skipping); and decomposition of transcription products.

The artificial nucleic acid of the present invention can be used to comprise a mutation portion in a nucleic acid of interest in a bulge structure, enabling design of artificial nucleic acids exhibiting comparable levels of gene expression inhibiting effects regardless of mutation. The artificial nucleic acid of the present invention can also more efficiently inhibit gene expression than conventional ASOs.

The genes whose expression is inhibited by the present aspect are not particularly limited, and examples include genes that have increased expression in various diseases. The diseases to be targeted are not particularly limited, and examples include those involving, for example, overexpression of normal proteins, expression of abnormal proteins, and overexpression of RNAs that directly or indirectly control the expression of proteins. The overexpression of normal proteins refers to production in abnormal amounts of proteins expressed in normal individuals, and is observed, for example, in inflammatory diseases and autoimmune diseases. The expression of abnormal proteins refers to production of proteins that are not expressed in normal individuals at least under certain conditions, and includes the cases, for example, where proteins themselves are in abnormal forms, or where proteins themselves are normal, but the timing when or the cell where the expression occurs is different from the normal case. This abnormality is observed in, for example, neurodegenerative diseases. Overexpression of RNAs that directly or indirectly control the expression of proteins refers to the cases where RNAs (e.g., miRNA) that positively or negatively control the expression of proteins are overexpressed, and the expression of the proteins being controlled is abnormal. The abnormality is observed in, for example, cancers.

Specific examples of diseases to which the gene expression inhibiting agent of the present aspect is applied include muscular dystrophy; cancers; cardiovascular diseases; hypertension; infections; kidney diseases; neurodegenerative diseases such as Alzheimer's disease, Parkinson's disease, multiple sclerosis, amyotrophic lateral sclerosis, Huntington's disease, and Creutzfeldt-Jakob disease; autoimmune diseases such as lupus, and rheumatoid arthritis;

endocarditis; Graves' disease; ALD; respiratory diseases such as asthma or cystic fibrosis; bone diseases such as osteoporosis, and joint diseases; liver diseases; skin diseases such as psoriasis and eczema; ocular diseases; otorhinolaryngologic diseases; other neurological diseases such as Tourette's syndrome, schizophrenia, depression, autism, and stroke; and metabolic diseases such as glycogen storage diseases and diabetes mellitus.

The inhibition levels of the gene expression by the gene expression inhibiting agent of the present aspect are not particularly limited. Specifically, and for example, when determined based on the expression levels of the transcription products and/or protein from the gene as indicators, the gene expression is inhibited by 100%, 90% or more, 75% or more, 60% or more, 50% or more, 40% or more, 30% or more, 25% or more, 20% or more, or 10% or more, compared to the case where the gene expression inhibiting agent is not introduced. Alternatively, the gene expression may be significantly inhibited compared to the case where the gene expression inhibiting agent is not introduced. The inhibition level of the gene expression may be determined based on the expression levels of the transcription products or proteins from the target gene as indicators. For example, the expression levels of the transcription product may be determined, for example, by Northern hybridization or RT-PCR. The expression level of the protein may be determined, for example, by Western blotting, ELISA, protein activity assay, or fluorescence intensity of fluorescent proteins.

2-2-2. Pharmaceutical Composition

The gene expression inhibiting agent of the present aspect can be formulated as an active ingredient with a carrier and the like into a pharmaceutical composition.

Carriers used may include, for example, pharmaceutically acceptable carriers. The term "pharmaceutically acceptable carriers" refers to additives commonly used in the pharmaceutical art. Examples thereof include solvents, bases, emulsifiers, suspending agents, surfactants, pH-adjusting agents, stabilizing agents, flavoring agents, flavors, excipients, vehicles, preservatives, binders, diluents, isotonizing agents, sedatives, fillers, disintegrants, buffering agents, coating agents, lubricants, coloring agent, sweeteners, thickeners, corrigents, solubilizing agents, and other additives.

The solvents may be, for example, water or other pharmaceutically acceptable aqueous solutions, or pharmaceutically acceptable organic solvents (e.g., vegetable oils). Examples of the aqueous solutions include physiological saline, isotonic solutions containing glucose or other adjuvants, phosphate buffers, and sodium acetate buffers. Examples of the adjuvants include D-sorbitol, D-mannose, D-mannitol, and sodium chloride, as well as low-concentration nonionic surfactants, and polyoxyethylene sorbitan fatty acid esters.

The carriers are used to avoid or reduce the decomposition of the artificial nucleic acid that is an active ingredient by enzymes and the like *in vivo,* as well as to facilitate the formulation or administration method, thereby maintaining the dosage form and drug efficacy, and may be appropriately used as necessary.

The dosage form of the pharmaceutical composition is not particularly limited as long as the delivery of the gene expression inhibiting agent described in the present aspect, an active ingredient, to a target site without inactivation due to decomposition or the like, and the exhibition of the pharmacological effect (gene expression inhibition effect) of the active ingredient *in vivo* are possible.

The specific dosage form varies depending on the administration method and/or formulation conditions. The administration methods can be broadly classified into parenteral administration and oral administration, and thus a dosage form suitable for each administration method may be employed.

A preferred administration form of the pharmaceutical composition is not particularly limited, and may be oral administration or parenteral administration. Specific examples of the parenteral administration include intramuscular administration, intravenous administration, intraarterial administration, intraperitoneal administration, subcutaneous administration (including implantable continuous subcutaneous administration), intradermal administration, intratracheal/intrabronchial administration, rectal administration, administration via transfusion, intraventricular administration, intrathecal administration, nasal administration, and intramuscular administration.

In cases where the administration method is parenteral administration, preferred dosage forms include solutions that can be systemically administered to a target site directly or via the circulatory system. Examples of the solutions include injectable agents. The injectable agents may be formulated by mixing the pharmaceutical composition in a unit dosage form as required by generally accepted pharmaceutical practice in combination with, for example, the excipients, elixirs, emulsifiers, suspensions, surfactants, stabilizing agents, and pH-adjusting agents as described above as appropriate. Other dosage forms may be used, including ointments, plasters, cataplasms, transdermal drugs, lotions, inhalants, aerosols, eyedrops, and suppositories.

With respect to the specific shapes and sizes of the dosage forms described above, they may be encompassed within dosage forms known in the art, and are not particularly limited. With respect to the method for producing the pharmaceutical composition of the present invention, formulation may be according to the conventional method in the art.

The amount (content) of the gene expression inhibiting agent comprised in the pharmaceutical composition varies depending on the type of the artificial nucleic acid, the delivery site, the dosage form of the pharmaceutical composition, the dose of the pharmaceutical composition, and the type of the carrier. Therefore, the amount may be

determined as appropriate considering each condition. As usual, the amount is adjusted such that a single-dose of the pharmaceutical composition contains an effective amount of the gene expression inhibiting agent. The "effective amount" refers to an amount that is required for the gene expression inhibiting agent to exert the function as an active ingredient, and provides little or no adverse effects to the living body to which it is applied. The effective amount may vary depending on various conditions, such as the information of the subject, the route of administration, and the frequency of administration. The final decision will be made based on the judgment by a physician, a veterinarian, a pharmacist, or the like. The "information of the subject" is various individual information of the living body to which the pharmaceutical composition is applied. For example, when the subject is human, the information includes, for example, the age, body weight, sex, diet, health status, progression and severity of the disease, drug sensitivity, and the presence of drug in combination.

3. Nucleic Acid Detecting agent

3-1. Summary

A third aspect of the present invention is a nucleic acid detecting agent. The nucleic acid detecting agent of the present invention comprises the artificial nucleic acid described in the first aspect as an active ingredient, and forms a specific three-dimensional structure in the presence of a nucleic acid of interest. The nucleic acid detecting agent of the present invention can be used to detect a nucleic acid of interest from a test sample.

3-2. Configuration

3-2-1. Components

The components of the nucleic acid detecting agent of the present invention will be described. The nucleic acid detecting agent of the present invention comprises an artificial nucleic acid as an essential component, and comprises a detectable label as an optional component. The components will be described in detail.

As used herein, the term "nucleic acid detecting agent" refers to an agent for detecting a nucleic acid of interest from a test sample. The nucleic acid detecting agent of the present invention allows a mutation portion in a nucleic acid of interest to be contained in a bulge structure, enabling detection of the nucleic acid of interest at comparable levels regardless of mutation.

The "test sample" is a material to be tested for the presence or the amount of a nucleic acid of interest. The test sample is not particularly limited as long as it is a sample that can contain a nucleic acid of interest. Specific examples of the test sample include biological materials such as blood, serum, blood cells, urine, stool, sweat, saliva, oral mucosa, sputum, lymph, spinal fluid, lacrimal fluid, breast milk, amniotic fluid, semen, tissues, biopsy, and cultured cells; environmental materials collected from environments; artificially synthesized nucleic acids, and combinations thereof. The test sample may be subjected to known pretreatment such as chopping, homogenization, and extraction before application of the nucleic acid detecting agent of the present aspect.

The artificial nucleic acid is as described in the first aspect, and thus a detailed explanation is omitted.

The nucleic acid detecting agent of the present aspect optionally comprises a detectable label. The type of the detectable label is not particularly limited, and may be determined as appropriate depending on detection methods. Specific examples of the detectable label include fluorescent dyes (e.g., FITC, Texas, Cy3, Cy5, Cy7, FAM, HEX, VIC, JOE, Rox, TET, Bodipy493, NBD, and TAMRA), luminescent substances (e.g., acridinium esters), uncolored small molecules that act as substrates of enzymes or as antigens (e.g., biotin and DIG), and radioactive isotopes (e.g., $^{32}P$, $^{3}H$, and $^{14}C$).

A fluorescent base can also be used as a label. The type of the fluorescent base is not particularly limited as long as it is a nucleobase emitting fluorescence. Specific fluorescent bases are those described, for example, in the Glen research catalog (https://www.glenresearch.com/media//folio3/productattachments/product_catalog/Glen_Product_Catalog_2021.pdf). Examples of the fluorescent base include, but not limited to, 2-aminopurine, pyrrolocytosine, 9-aminoethyl-1,3-diaza-2-oxophenoxazine, 1,$N^6$-ethenoadenine, 5-(1-pyrenyl-ethinyl)uracil, 1,3-diaza-2-oxophenothiazine, and 1,3-diaza-2-oxophenoxazine. The fluorescent base may be bound to, for example, the 1'-position of the sugar.

The fluorescent base-containing nucleotide may be, for example, a nucleotide having 2-aminopurine or pyrrolocytosine.

3-2-2. Detection

The nucleic acid detecting agent of the present aspect can be used to detect a nucleic acid of interest from a test sample.

The detection method is not particularly limited as long as a nucleic acid of interest hybridizing with the nucleic

acid detecting agent can be detected. For example, detection may be performed without detectable labels or the like, with detectable labels, with detectable three-dimensional structure-recognizing molecules, or combinations thereof.

With the nucleic acid detecting agent of the present aspect, a nucleic acid of interest can be detected without detectable labels or the like. The detection method in this case is not particularly limited as long as it is a detection method for nucleic acids. Specific examples include methods using electrophoresis, methods using nucleases, and methods using melting curves for double-stranded nucleic acids. The artificial nucleic acid of the present invention and a nucleic acid of interest hybridize with each other and form a specific three-dimensional structure. Thus, the mobility of a double-stranded nucleic acid composed of the artificial nucleic acid and a nucleic acid of interest is different from that of normal complementary double-stranded nucleic acids composed of comparable number of bases. Therefore, a nucleic acid of interest may be detected, for example, by detecting a double-stranded nucleic acid with the same mobility as that expected when a specific three-dimensional structure is formed. The double-stranded nucleic acid composed of the artificial nucleic acid and a nucleic acid of interest may contain a single-stranded portion having a specific base sequence in the target domain and/or three-dimensional structure formation-inducing domain. Thus, the nucleic acid of interest may be detected, for example, by processing with nucleases that specifically cleave double-stranded nucleic acids obtained from a test sample, followed by detection of a nucleic acid having the specific base sequence. Furthermore, the melting curve of a double-stranded nucleic acid composed of the artificial nucleic acid of the present invention and a nucleic acid of interest is different from that of normal complementary double-stranded nucleic acids composed of comparable number of bases. Therefore, a nucleic acid of interest may be detected, for example, by detecting a double-stranded nucleic acid showing the same melting curve as that expected when a specific three-dimensional structure is formed.

The nucleic acid detecting agent of the present aspect may be detected using a detectable label. The detection method is not particularly limited, and is usually determined depending on the type of the label used and the nature of the test sample. In cases where a fluorescent dye or a luminescent substance is used, detection can be performed, for example, by visual inspection, by using a microscope (e.g., fluorescence microscope), by using a detector (e.g., fluorescence-activated cell sorting (FACS), luminescence spectrophotometer, absorption spectrometer), or a combination thereof. In cases where uncolored small molecules that act as substrates of enzymes or as antigens are used as labels, detection may be performed, for example, by similar methods as in cases where fluorescent dyes or luminescent substances are used after treatment such as enzyme treatment. In cases where radioactive isotopes are used as labels, detection can be performed, for example, by autoradiography, scintillation counter, positron-emission tomography (PET), or a combination thereof.

The nucleic acid detecting agent of the present aspect may be detected using a recognizing molecule for the three-dimensional structure formation. The artificial nucleic acid of the present invention hybridizes with a nucleic acid of interest and form a specific three-dimensional structure. Therefore, a nucleic acid of interest may be detected via observation of the event that occurs specifically during the formation of a specific three-dimensional structure. For example, a nucleic acid of interest may be detected by detecting the binding of a specific protein (including, for example, an antibody against the specific three-dimensional structure), the binding of a specific type of nucleic acid, the cleavage of the double-stranded nucleic acid at a specific position with respect to the three-dimensional structure, or the like. The events that can occur during the formation of the three-dimensional structures are exemplified in the sections of definition of the three-dimensional structures. The detection method to be used may be determined as appropriate depending on the event to be detected.

The nucleic acid detecting agent of the present aspect may be provided as a composition in which the nucleic acid detecting agent is mixed with other molecules or agents needed for the detection, or a kit together with other molecules or agents needed for the detection. The other molecules needed for the detection may include some or all of the molecules needed for the detection depending on the desired detection method as described above.

The nucleic acid detecting agent of the present invention may also be used, for example, in Southern blotting, Northern blotting, and in situ hybridization.

## 4. Method for Producing Artificial Nucleic Acid

### 4-1. Summary

A fourth aspect of the present invention is a method for producing an artificial nucleic acid. The method for producing an artificial nucleic acid of the present aspect comprises a target domain selecting step, a three-dimensional structure formation-inducing domain determining step, and a nucleic acid synthesizing step as essential steps, and a hybridizable domain determining step as an optional step. The present method enables synthesis of the artificial nucleic acid described in the first aspect.

4-2. Methods

The method of the present aspect comprises a target domain selecting step, a three-dimensional structure formation-inducing domain determining step, and a nucleic acid synthesizing step as essential steps, and a hybridizable domain determining step as an optional step. The steps will be described in detail below.

4-2-1. Target Domain Selecting Step

The "target domain selecting step" is a step of searching a nucleic acid of interest for the sequence information for one side of a sequence motif composed of double strands forming a specific three-dimensional structure, and selecting 1 or more of the information as a target domain.
In the target domain selecting step, the base sequence of the nucleic acid of interest is compared with the sequence information for one side of a sequence motif of a specific three-dimensional structure, and a target domain that can form the specific three-dimensional structure is selected.
The nucleic acid of interest and the specific three-dimensional structure can be selected as appropriate according to the description of the first aspect.
As described in the first aspect, in the sequence motif, the target domain and the three-dimensional structure formation-inducing domain comprise complementary regions consisted of mutually complementary sequences; the target domain and/or the three-dimensional structure formation-inducing domain further comprises a non-complementary-containing region with 1 or more bases comprising a mutually non-complementary sequence; and the non-complementary-containing region comprises non-complementary sequences at its both ends. In cases where a non-complementary-containing region exists, a complementary region(s) can be adjacent to one or both sides of the non-complementary-containing region. The selection criteria for whether a complementary region(s) is/are adjacent to one or both sides are not particularly limited. For example, it may be determined randomly or based on previous reports.
In the present step, the nucleic acid of interest is searched for a region having a base sequence identical to a portion or the entirety of the sequence information for one side of a sequence motif of the specific three-dimensional structure. The phrase "sequence information for one side of a sequence motif' hereby means separating the sequence information of a sequence motif composed of double strands into single strands to use for the search. For example, the sequence information of only 1 chain of a sequence motif may be used for the search, or each of the sequence information of both chains, which is being separated into single strands, may be used for the search. The base sequence searching method used in this case is not particularly limited. For example, an identity searching program that is commonly used, such as an identity searching program BLAST (Basic local alignment search tool; Altschul, S. F. et al., J. Mol. Biol., 215, 403-410, 1990) may be used for the search. In this case, the specific three-dimensional structures to be searched for may be 1 or more types.
The sequence motif to be used in this case is not particularly limited. For example, a consensus sequence may be used as the sequence motif, or a sequence in which the entire base sequence has been determined may be used as a sequence motif. In particular, in cases where a consensus sequence is used, a region with identical alignment to conserved bases therein is searched for in a nucleic acid of interest. In cases where a sequence in which the entire base sequence has been determined, not only a region completely identical to the sequence, but also a region with a sequence having a certain level or more of identity to the sequence may be selected as a candidate of the target domain. For example, a region with a sequence in which 1 base, 2 bases, 3 bases, 4 bases, 5 bases, or 6 bases are substituted may be selected as a candidate for the target domain. Alternatively, for example, the region may be selected as a candidate for the target domain (hereinafter, referred to as "target domain candidate"), in cases where the identity is 80% or more, 90% or more, 95% or more, 96% or more, 97% or more, 98% or more, 99% or more, 99.5% or more, 99.9% or more, or 100%. The number of the sequence motifs used for the search is not particularly limited. For example, 2 or more types of sequence motifs may be searched for at once.
In cases where a plurality of target domain candidates are obtained, any one or more of them may be selected for a target domain(s). The selection criteria are not particularly limited. For example, the selection may be made at random, based on the regions on the genes in which the target domain candidates are comprised, based on the three-dimensional structures that can be induced for the target domain candidates, based on the sequence identity with the sequence motifs, based on the information on the base sequences and the ease of forming the three-dimensional structures, or by a combination thereof. For example, the sequence of a target domain candidate selected may be a sequence that has been demonstrated to form the three-dimensional structure. Preferably, but without limitation, a target domain candidate is comprised in a region to which an artificial nucleic acid can be hybridized to exhibit a desired effect. In cases where the nucleic acid of interest comprises a mutation described in the first aspect, a target domain candidate in which the position corresponding to the mutation is the position of a not uniquely determined base in a sequence motif, may be preferentially selected. In cases where, for example, a plurality of target domain candidates exist contiguously with some overlap, the plurality of contiguous smaller target domain candidates may be grouped together as one

larger target domain.

The order of performing the sequence motif search and target domain selection is not particularly limited, and preferably the target domain selection is performed concurrently with or after the sequence motif search.

4-2-2. Three-dimensional Structure Formation-inducing Domain Determining Step

The "three-dimensional structure formation-inducing domain determining step" is a step of determining the sequence of a three-dimensional structure formation-inducing domain such that the three-dimensional structure formation-inducing domain forms a sequence motif together with the target domain. The present step may be performed concurrently with or after the target domain selecting step described above.

Based on the sequence information of the strand corresponding to the artificial nucleic acid in a sequence motif, which contains the sequence information of the target domain, the sequence of the three-dimensional structure formation-inducing domain is determined.

In cases where the base sequence of a sequence motif is not uniquely determined, the bases may be arbitrarily determined. In cases where, for example, the sequence is uniquely defined based on the base at the corresponding position in the other strand for a not uniquely determined sequence, the determination may be made accordingly. The selection criteria for bases are not particularly limited. For example, in cases where many RNAs that form the specific three-dimensional structure have a specific base at a specific position, said base may be selected.

In the present step, some or all of the nucleotides in the three-dimensional structure formation-inducing domain may be determined to be modified nucleotides. The introduction of modified nucleotides in the three-dimensional structure formation-inducing domain is in accordance with the content described in the first aspect.

In cases where the present step is performed concurrently with the target domain selecting step, for example, the sequences of the three-dimensional structure formation-inducing domains are determined for all target domain candidates, and a target domain may be selected from the target domain candidates thereafter.

The order of performing the determination of the sequence and the determination of the modified nucleotide introduction is not particularly limited, and the determination of the modified nucleotide introduction is preferably performed concurrently with or after the determination of the sequence.

4-2-3. Hybridizable Domain Determining Step

The "hybridizable domain determining step" is a step of determining the base sequence of a hybridizable domain composed of 6 bases to 120 bases, adjacent to one or both side(s) of the three-dimensional structure formation-inducing domain. The present step is an optional step, and may be performed concurrently with or after the three-dimensional structure formation-inducing domain determining step described above.

In the present step, the base sequence of a hybridizable domain in the artificial nucleic acid is determined. The hybridizable domain may be adjacent to one or both side(s) of the three-dimensional structure formation-inducing domain, and any of which may be selected. For example, in cases where hybridizable domains are adjacent to both sides of 1 three-dimensional structure formation-inducing domain, the hybridizable domains comprise 2 subdomains. The base sequence of the hybridizable domain may be determined based on the base sequence of the region other than the target domain in the nucleic acid of interest. The method of determining the base sequence is not particularly limited. The level of the base identity between the base sequence is also not particularly limited as long as they are hybridizable.

The method of the present aspect may further comprise a step of determining modification or an optional additional sequence contained in the artificial nucleic acid. The timing when these steps are performed is not particularly limited. The details are as described in the first aspect, and the determination methods are not particularly limited.

4-2-4. Nucleic Acid Synthesizing Step

The "nucleic acid synthesizing step" is a step of synthesizing the artificial nucleic acid based on the sequence information determined in the three-dimensional structure formation-inducing domain determining step. The present step may be performed concurrently with or after the three-dimensional structure formation-inducing domain determining step described above. In cases where a hybridizable domain determining step is performed, the nucleic acid synthesizing step may be performed concurrently with or after the step.

The artificial nucleic acid of the present invention can be produced by a person skilled in the art by selecting a known method as appropriate. An artificial nucleic acid may be synthesized based on the information on the base sequence of the artificial nucleic acid determined in the three-dimensional structure formation-inducing domain determining step, using a commercially available automatic nucleic acid synthesizer, such as from GE Healthcare, Thermo Fisher Scientific, or Beckman Coulter. In cases where a hybridizable domain determining step is performed, an artificial nucleic acid can be synthesized based on the information on the base sequence of the artificial nucleic acid determined

in the three-dimensional structure formation-inducing domain determining step and the three-dimensional structure formation-inducing domain determining step. After the synthesis, post-treatment such as purification of the obtained artificial nucleic acid using a reversed-phase column or the like may be performed.

## Examples

<Example 1: Evaluation of Stability of RNA Molecule and Nucleic Acid Molecule That Hybridizes Thereto to Induce Three-dimensional Structure>

(Purpose)

The feasibility of actively forming a three-dimensional structure between 2 nucleic acid chains is assessed, and the stability of a double-stranded nucleic acid in which a three-dimensional structure is induced is investigated.

(Method)

1. Design and Synthesis of Nucleic Acid Molecule

An RNA molecule having the base sequence shown in SEQ ID NO: 13 was designed as a nucleic acid of interest (ROI: the uppers in Figures 7A to 7G). As artificial nucleic acids, based on the consensus sequence of the three-dimensional structure, an RNA molecule that induces a bulged-G structure (RNA-BG; SEQ ID NO: 14: the lower in Figure 7B); an RNA molecule that induces a Kink-turn structure (RNA-KT; SEQ ID NO: 15: the lower in Figure 7C); an RNA molecule that induces a Reverse Kink-turn structure (RNA-RKT; SEQ ID NO: 16: the lower in Figure 7D); an RNA molecule that induces a 5S loop E structure (RNA-5S; SEQ ID NO: 17: the lower in Figure 7E); an RNA molecule that induces a C-loop structure (RNA-CL; SEQ ID NO: 18: the lower in Figure 7F); and an RNA molecule that induces a tandem GA structure (RNA-GA; SEQ ID NO: 19: the lower in Figure 7G) were designed. In addition, an RNA molecule having the complementary sequence to the nucleic acid of interest (RNA-ASO; SEQ ID NO: 20: the lower in Figure 7A) was designed as a control.

The RNA molecule synthesis was performed using an automatic nucleic acid synthesizer NTS-M2-MX (Nihon Techno Service Co, Ltd). The synthesized samples were purified by gel filtration using NAP-10 columns (Cytiva). After purification, electrophoresis on a 20% denaturing polyacrylamide gel containing 7 M urea was performed to confirm that the samples were purified properly at high purities.

2. Measurement of Thermostability of Double-stranded RNA

Temperature control was performed using an absorption spectrometer V-630 (Jasco) connected to a temperature controller PAC-743R (Jasco). The thermal melting of the double-stranded nucleic acids was determined by measuring the absorbance of ultraviolet light at a wavelength of 260.0 nm in the temperature range of 20 to 100°C (measurement interval of 1°C at a rate of temperature rise of 1 °C/min). The ROI and the artificial nucleic acids or the RNA-ASO were added to a solution containing 10 mM sodium cacodylate (pH 7.0) and 100 mM sodium chloride to the final concentrations of 1 μM, to prepare test solutions as samples.

In order to prepare a thermal melting curve, the relative absorbance (A) was calculated from the measured absorbance using the following equation.

$$A = (A_t - A_{min})/(A_{max} - A_{min})$$

($A_t$: absorbance at t°C, $A_{min}$: minimum absorbance, $A_{max}$: maximum absorbance)

The inflection point of the thermal melting curve prepared as a sigmoid curve was approximately determined as an intersecting point of a straight line approximated in the straight-line region of the temperature change data by the least-squares method and the straight-line region, and the temperature was defined as the double strand melting temperature ($T_m$).

(Results)

The results are shown in Figure 8 and Table 1.

[Table 1]

Table 1. Melting temperatures of double-stranded nucleic acids formed by unmodified artificial nucleic acids and nucleic acids of interest

| Artificial nucleic acid | $T_m$ (°C) | $\Delta T_m$ (°C) |
|---|---|---|
| RNA-ASO | 80.5 | - |
| RNA-BG | 66.2 | -14.3 |
| RNA-KT | 69.1 | -11.4 |
| RNA-RKT | 69.7 | -10.8 |
| RNA-5S | 70.1 | -10.4 |
| RNA-CL | 67.7 | -12.8 |
| RNA-GA | 72.3 | -8.2 |

* $\Delta T_m$ indicates a value obtained by subtracting the $T_m$ value for the double-stranded nucleic acid formed by the nucleic acid of interest and the artificial nucleic acid from the $T_m$ value for the nucleic acid of interest and RNA-ASO.

The melting curves were sigmoidal for any of the nucleic acids used, demonstrating that stable double strands can be formed by using any of the nucleic acids together with the nucleic acid of interest (Figure 8). Compared to the thermal melting curve for the double-stranded nucleic acid formed between the nucleic acid of interest and the control (Figure 8A), the thermal melting curves for the double-stranded nucleic acids formed with the nucleic acid of interest when using 6 types of artificial nucleic acids (Figures 8B to 8G) were shifted to lower temperature, and the $T_m$ values were also lower (Table 1). However, it was demonstrated that the double strands were sufficiently stable at temperatures near the body temperature (40°C or lower). Among the 6 types of artificial nucleic acids, RNA-GA was the most stable (Figure 8G), while RNA-BG was the most unstable (Figure 8B).

<Example 2: Evaluation of Effect of Modification on Stability of Double-stranded Nucleic Acid>

(Purpose)

The stability of double-stranded nucleic acids when using modified nucleotides was investigated.

(Method)

The experiment was performed in accordance with the method in Example 1. The nucleic acids used were as follows.

As in Example 1, an RNA molecule having the base sequence shown in SEQ ID NO: 13 was used as a nucleic acid of interest (ROI). 3 types of nucleic acid molecules which had the same base sequence as Example 1, but in which all nucleotides thereof had 2'-O-methyl (2'-OMe) modification were used as artificial nucleic acids. A nucleic acid molecule that induces a bulged-G structure (OMe-BG; SEQ ID NO: 21), a nucleic acid molecule that induces a 5S loop E structure (OMe-5S; SEQ ID NO: 22), and a nucleic acid molecule that induces a tandem GA structure (OMe-GA; SEQ ID NO: 23) were used. A nucleic acid molecule that had the same base sequence as Example 1, but in which all nucleotides thereof had 2'-OMe modification (OMe-ASO; SEQ ID NO: 24) was used as a control.

(Results)

The results are shown in Figure 9 and Table 2.

[Table 2]

Table 2. Melting temperatures of double-stranded nucleic acids formed by 2'-OMe modified artificial nucleic acids and nucleic acids of interest

| Artificial nucleic acid | $T_m$ (°C) | $\Delta T_m$ (°C) | $\Delta T_{m\ (modi)}$ (°C) |
|---|---|---|---|
| OMe-ASO | 77.4 | - | -3.1 |
| OMe-BG | 67.5 | -9.9 | 1.3 |
| OMe-5S | 72.2 | -5.2 | 2.1 |

(continued)

| Table 2. Melting temperatures of double-stranded nucleic acids formed by 2'-OMe modified artificial nucleic acids and nucleic acids of interest | | | |
|---|---|---|---|
| Artificial nucleic acid | $T_m$ (°C) | $\Delta T_m$ (°C) | $\Delta T_{m\ (modi)}$ (°C) |
| OMe-GA | 78.0 | 0.6 | 5.7 |

* $\Delta T_m$ indicates a value obtained by subtracting the $T_m$ value for the double-stranded nucleic acid formed by the nucleic acid of interest and the artificial nucleic acid from the $T_m$ value for the nucleic acid of interest and RNA-ASO.
* $\Delta T_{m\ (modi)}$ is a value obtained by subtracting the $T_m$ value for the case using an unmodified artificial nucleic acid from the $T_m$ value for the case using a modified artificial nucleic acid.

Unlike the case using the control (Figure 9A), the melting curves for all cases using the artificial nucleic acids were shifted to higher temperature compared to Example 1 using unmodified RNAs (Figures 9B-9D), and the $T_m$ values were also higher ($\Delta T_{m\ (modi)}$ column in Table 2). In particular, the $T_m$ value for OMe-GA was higher than that for the control, demonstrating that the case where a three-dimensional structure was introduced can be more stable compared to the cases with complete complementarity to the nucleic acid of interest, in some cases. The improvement in the stability of artificial nucleic acids due to modification was remarkable particularly in relatively lower temperature region, and all the artificial nucleic acids tested resulted in more stable than ROI/OMe-ASO in the temperature range of about 50°C or less (Figure 9). Among the 3 types of artificial nucleic acids, even after modification, RNA-GA was the most stable (Figure 9D), while RNA-BG was the most unstable (Figure 9B). This demonstrated that the same modification resulted in no significant change in the stability ranking of artificial nucleic acids before and after the modification.

These results confirm that artificial nucleic acids can form stable double strand together with a nucleic acid of interest, even though the artificial nucleic acids have a plurality of bases that are non-complementary to the nucleic acid of interest. Furthermore, it is demonstrated that nucleotide modification such as 2'-OMe is an effective means to improve the stability of double strands.

<Example 3: Evaluation of Effect of Nucleotide Modification on Three-dimensional Structure (Bulged-G Structure)>

(Purpose)

The effect of nucleotide modification on the bulged-G structure is investigated.

(Method)

1. Design and Synthesis of Nucleic Acid Molecule

An RNA molecule that forms the bulged-G structure (unmodified BG; Figure 10A; SEQ ID NO: 25: PDB-ID=1Q9A), and an RNA molecule with the following modification on nucleotides contained in 1 nucleic acid chain in the double-stranded region of the bulged-G structure (modified BG; Figure 10D; SEQ ID NO: 26) were used.

- 2'-OMe modification was introduced into all nucleotides having a sugar moiety in the C3'-endo conformation, and having a hydroxy group at the 2' position that was not involved in hydrogen bonding (the lowercase letters in Figure 10D).
- All nucleotides having a sugar moiety in the C2'-endo conformation were replaced by DNA (the italicized lowercase letters in Figure 10D).

The nucleic acid molecules were chemically synthesized using an automatic nucleic acid synthesizer. Thereafter, purification was performed by electrophoresis on a 20% denaturing polyacrylamide gel (30 cm × 40 cm) containing 7 M urea, followed by gel filtration using NAP-10 columns for desalting.

2. X-ray Crystallography

Crystallization was performed by the hanging drop vapor diffusion crystallization method. 0.2 μL of 1 mM or 2 mM oligonucleotide for crystallization and 0.2 μL of a buffer for crystallization were mixed to prepare a drop, which was then equilibrated with a reservoir solution. For the nucleic acids, crystallization was performed under 2 types of crystallization conditions. The details of the crystallization conditions are shown in Table 3.

[Table 3]

| Table 3. Crystallization conditions | | | | |
|---|---|---|---|---|
| | Unmodified BG (crystal I) | Unmodified BG (crystal II) | Modified BG (crystal I) | Modified BG (crystal II) |
| Sample solution: 0.2 μL | | | | |
| Oligonucleotide | 1 mM | 2 mM | 1 mM | 1 mM |
| Crystallization solution: 0.2 μL | | | | |
| Sodium cacodylate (pH 7.0) | 50 mM | - | 50 mM | 50 mM |
| MOPS (pH 7.0) | - | 50 mM | - | - |
| Hexaamminecobalt chloride | 10 mM | - | 10 mM | |
| Spermine tetrahydrochloride | - | - | - | 1 mM |
| Lithium chloride | - | - | 10 mM | - |
| Potassium chloride | 10 mM | - | - | - |
| Magnesium chloride | | 10 mM | | |
| Calcium chloride | - | - | - | 100 mM |
| Ammonium sulfate | | 3.5 M | - | - |
| 2-methyl-2,4-pentanediol | 10% | - | 10% | 1% |
| Reservoir solution: 250 μL | | | | |
| 2-methyl-2,4-pentanediol | 40% | - | 40% | 40% |
| Others | - | Crystallization solution | - | - |

Single crystals were scooped up with CryoLoop (Hampton Research), and immersed in 40% 2-methyl-2,4-pentanediol (MPD) solution for several seconds. Thereafter, the resulting products were quick-frozen in liquid nitrogen and used for the X-ray diffraction experiment.

The X-ray diffraction experiment was performed using the Structural Biology Beamlines BL-17A and ARNE-3A of the Photon Factory. The diffraction data was processed using a program XDS. The initial phase was determined by the molecular replacement method using a program Phaser (Phenix). The atomic parameter values were refined using a program phenix.refine (Phenix).

(Results)

The results are shown in Tables 4 and 5, and Figures 10 to 12.

[Table 4]

| Table 4. Crystallographic data, as well as data measurement and three-dimensional structure refinement statistics | | |
|---|---|---|
| Crystal code | Unmodified BG (crystal I) | Unmodified BG (crystal II) |
| **Crystal data** | | |
| Space group | $C222_2$ | $P4_3$ |
| Unit cell (Å) | a = 25.1, b = 53.3, c = 105.6 | a = b = 29.7, c = 76.8 |
| **Data collection** | | |
| Beam line | Photon Factory BL-17A | Photon Factory BL-17A |
| Wavelength (Å) | 1.10567 | 1.10567 |
| Resolution (Å) | 26.7-2.6 | 27.7-2.2 |

(continued)

| Data collection | | |
|---|---|---|
| Outer-shell resolution (Å) | 2.7-2.6 | 2.3-2.2 |
| Unique reflections | 4236 | 6691 |
| Completeness (%) | 99.1 | 99.8 |
| Outer-shell completeness (%) | 96.5 | 97.7 |
| $R_{anom}$ (%) | 8.9 | 11.8 |
| Outer-shell $R_{anom}$ (%) | 37.4 | 33.5 |
| Multiplicity | 3.4 | 6.8 |
| Outer-shell multiplicity | 3.2 | 6.0 |
| I/σ (I) | 10.9 | 13.6 |
| Outer-shell I/σ (I) | 4.0 | 7.2 |
| **Structure refinement** | | |
| Resolution (Å) | 26.7-2.6 | 27.7-2.2 |
| Reflection used | 4236 | 6691 |
| R factor (%) | 18.0 | 17.7 |
| $R_{free}$ (%) | 24.4 | 21.4 |
| R.m.s.d. bond length (Å) | 0.005 | 0.005 |
| R.m.s.d. bond angle (°) | 0.9 | 1.0 |

[Table 5]

Table 5. Crystallographic data, as well as data measurement and three-dimensional structure refinement statistics

| Crystal code | Modified BG (crystal I) | Modified BG (crystal II) |
|---|---|---|
| **Crystal data** | | |
| Space group | $P2_1$ | $P4_32_12$ |
| Unit cell (Å, °) | a = 24.8, b = 116.4, c = 27.9 β = 107.2 | a = b = 68.9, c = 116.0 |
| **Data collection** | | |
| Beam line | Photon Factory BL-17A | Photon Factory BL-17A |
| Wavelength (Å) | 1.60496 | 0.98 |
| Resolution (Å) | 29.1-2.7 | 30.3-1.7 |
| Outer-shell resolution (Å) | 2.8-2.7 | 1.74-1.7 |
| Unique reflections | 7414 | 30956 |
| Completeness (%) | 90.3 | 98.2 |
| Outer-shell completeness (%) | 85.3 | 98.3 |
| $R_{merge}$ (%) | - | 8.6 |
| Outer-shell $R_{merge}$ (%) | - | 36.5 |
| $R_{anom}$ (%) | 5.4 | - |
| Outer-shell $R_{anom}$ (%) | 26.7 | - |
| Multiplicity | 1.6 | 6.5 |
| Outer-shell multiplicity | 1.6 | 6.8 |
| I/σ (I) | 8.3 | 12.0 |
| Outer-shell I/σ (I) | 2.4 | 4.7 |
| **Structure refinement** | | |
| Resolution (Å) | 29.1-2.7 | 30.3-1.7 |
| Reflection used | 7414 | 30941 |
| R factor (%) | 20.7 | 18.9 |

(continued)

| Structure refinement | | |
|---|---|---|
| $R_{free}$ (%) | 26.5 | 23.7 |
| R.m.s.d. bond length (Å) | 0.006 | 0.006 |
| R.m.s.d. bond angle (°) | 1.5 | 1.5 |

For unmodified BG and modified BG, crystals were obtained under 2 types of crystallization conditions (Table 3: referred to as crystal I and crystal II, respectively), both of which successfully underwent structural analysis (Tables 4 and 5).

The 2 types of crystals gave 2 three-dimensional structures. It was demonstrated that the crystals I and II did not differ in the three-dimensional structure, and formed a certain three-dimensional structure regardless of the crystallization conditions (Figure 10). In particular, the three-dimensional structure for unmodified BG was completely consistent with that demonstrated by previous research (Correll, C. C., et al., Nucleic Acids Research, 2003, 31(23), 6806-6818) (Figures 10B and 10C). Similarly for modified BG, it was demonstrated that bulged-G structures were formed regardless of the crystallization conditions (Figures 10E and 10F). Observation of the interaction between nucleotides in every base pair demonstrated that the internucleotide interactions in the unmodified BG and modified BG were completely the same (Figures 11 and 12).

This demonstrates that the modification mode performed in this Example does not affect the three-dimensional structure. In other words, it is demonstrated that the cases where the conformation of the sugar moiety does not change before and after modification basically result in no effect on the three-dimensional structure, and in addition, modification of the hydroxy group at the 2' position of C3'-endo ribose when the group is not involved in hydrogen bonding is not problematic.

<Example 4: Evaluation of Effect of Nucleotide Modification on Three-dimensional Structure (Kink-turn structure)>

(Purpose)

The effect of nucleotide modification on the Kink-turn structure is investigated.

(Method)

1. Design and Synthesis of Nucleic Acid Molecule

An RNA molecule that forms 2 Kink-turn structures via hybridization between 2 molecules (unmodified KT; Figure 13A; SEQ ID NO: 27: PDB-ID=4CD1), and an RNA molecule with the following modification on nucleotides contained in one nucleic acid chain in the double-stranded region forming the Kink-turn structures (modified KT; Figure 13C; SEQ ID NO: 28) were used.

- 2'-OMe modification was introduced into all nucleotides having a sugar moiety in the C3'-endo conformation and having a hydroxy group at the 2' position that was not involved in hydrogen bonding (the lowercase letters in Figure 13C).
- A bulky OMe modification was also introduced into 1 nucleotide at the 2' position, which has a sugar moiety in the C3'-endo conformation and has a hydroxy group at the 2' position that was involved in hydrogen bonding (the lowercase letters in Figure 13C).
- All nucleotides having a sugar moiety in the C2'-endo conformation were replaced by DNA (the italicized lowercase letters in Figure 13C).

The nucleic acid molecules were synthesized and purified according to Example 3.

2. X-ray Crystallography

The crystallization and structural analysis for the nucleic acids were performed under 1 type of crystallization conditions in accordance with Example 3. The details of the crystallization conditions are shown in Table 6.

[Table 6]

| Table 6. Crystallization conditions | | |
|---|---|---|
| | Unmodified KT | Modified KT |
| Sample solution: 0.2 μL | | |
| Oligonucleotide | 2 mM | 2 mM |
| Crystallization solution: 0.2 μL | | |
| Sodium cacodylate (pH 7.0) | 50 mM | 50 mM |
| Spermine tetrahydrochloride | 10 mM | 10 mM |
| Sodium chloride | 500 mM | - |
| Potassium chloride | - | 400 mM |
| 2-methyl-2,4-pentanediol | 10% | 10% |
| Reservoir solution: 250 μL | | |
| 2-methyl-2,4-pentanediol | 40% | 40% |

(Results)

The results are shown in Table 7 and Figures 13 to 16.

[Table 7]

| Table 7. Crystallographic data, as well as data measurement and three-dimensional structure refinement statistics | | |
|---|---|---|
| Crystal code | Unmodified KT | Modified KT |
| **Crystal data** | | |
| Space group | $P6_322$ | $P6_322$ |
| Unit cell (Å) | a = b = 70.2, c = 47.7 | a = b = 70.6, c = 48.2 |
| **Data collection** | | |
| Beam line | Photon Factory BL-17A | Photon Factory BL-17A |
| Wavelength (Å) | 0.98 | 0.98 |
| Resolution (Å) | 37.5-2.6 | 37.8-2.9 |
| Outer-shell resolution (Å) | 2.7-2.6 | 3.0-2.9 |
| Unique reflections | 2386 | 1761 |
| Completeness (%) | 99.9 | 98.5 |
| Outer-shell completeness (%) | 99.4 | 99.3 |
| $R_{merge}$ (%) | 5.6 | 5.7 |
| Outer-shell $R_{merge}$ (%) | 29.9 | 36.6 |
| Multiplicity | 33.5 | 4.5 |
| Outer-shell multiplicity | 33.5 | 4.5 |
| I/σ (I) | 55.9 | 16.9 |
| Outer-shell I/σ (I) | 16.0 | 4.0 |
| **Structure refinement** | | |
| Resolution (Å) | 37.5-2.6 | 37.8-2.9 |
| Reflection used | 2382 | 1753 |
| R factor (%) | 18.5 | 19.2 |
| $R_{free}$ (%) | 24.0 | 23.4 |
| R.m.s.d. bond length (Å) | 0.006 | 0.010 |
| R.m.s.d. bond angle (°) | 1.2 | 1.5 |

For unmodified KT and modified KT, crystals were obtained, both of which successfully underwent structural analysis (Table 7).

The three-dimensional structure for unmodified KT was completely consistent with that demonstrated by previous research (McPhee, S. A., et al., Nature communications, 2014, 5(1), 1-6), forming 2 Kink-turn structures (Figures 13A and 13B). The crystallization conditions used in this Example were different from the previous research. Therefore, similarly to the bulged-G structure, the Kink-turn structure is also a stable three-dimensional structure regardless of the crystallization conditions. In addition, no significant difference in the three-dimensional structures was found for modified KT and unmodified KT (Figures 13 and 14). Furthermore, one of the 2 formed Kink-turn structures was observed for the interaction between nucleotides in every base pair (Figures 15 and 16). The results showed that the hydrogen bonds between bases were generally maintained (Figure 15). It was found that some hydrogen bonds were destructed (cleaved) by replacement by DNA nucleotides or 2'-OMe modification in cases where the hydroxy group at the 2' position of ribose was involved in the hydrogen bond (the circles in Figures 15C and 15D, and the circles in Figures 16C and 16D). In particular, it was found that bulky 2'-OMe modification may cause destruction of some hydrogen bonds between bases (Figures 16C and 16D).

This demonstrates that the modification mode performed in this Example does not remarkably affect the three-dimensional structure, but may affect the stability. Thus, considering from the viewpoint of stability, it is demonstrated that the 2' position of ribose having the C3'-endo conformation, when the hydroxy group at the position is involved in hydrogen bonding, preferably has no modification, or has modification with an unbulky substituent that does not destruct the hydrogen bond between bases.

<Example 5: Evaluation of Effect of Nucleotide Modification on Three-dimensional Structure (Tetraloop Receptor Structure)>

(Purpose)

The effect of nucleotide modification on the tetraloop receptor structure is investigated.

(Method)

1. Design and Synthesis of Nucleic Acid Molecule

An RNA molecule (modified TLR; Figure 17C; SEQ ID NO: 30) in which nucleotides contained in one nucleic acid chain in the double-stranded region of an RNA molecule that forms the tetraloop receptor structure (unmodified TLR; Figure 17A; SEQ ID NO: 29: PDB-ID=4FNJ) were replaced by DNA (the italicized lowercase letters in Figure 17D) was used.

The nucleic acid molecules were synthesized and purified in accordance with Example 3.

2. X-ray Crystallography

The crystallization and structural analysis were performed in accordance with Example 3. Crystallization was performed under 2 types of crystallization conditions. The details of the crystallization conditions are shown in Table 8.

[Table 8]

Table 8. Crystallization conditions

| | Modified TLR (crystal I) | Modified TLR (crystal II) |
|---|---|---|
| Sample solution: 0.2 μL | | |
| Oligonucleotide | 2 mM | 2 mM |
| Crystallization solution: 0.2 μL | | |
| Sodium cacodylate (pH 7.0) | 50 mM | - |
| Sodium malonate (pH 7.0) | - | 1.1 M |
| HEPES (pH 7.0) | - | 100 mM |
| Hexaamminecobalt chloride | 10 mM | - |
| Strontium chloride | 500 mM | - |
| Jeffamine ED-2001 (pH 7.0) | - | 0.5% |
| 2-methyl-2,4-pentanediol | 10% | 10% |

(continued)

| Reservoir solution: 250 μL | | |
|---|---|---|
| 2-methyl-2,4-pentanediol | 40% | - |
| Others | - | Crystallization solution |

(Results)

The results are shown in Table 9 and Figures 17 to 20.

[Table 9]

Table 9. Crystallographic data, as well as data measurement and three-dimensional structure refinement statistics

| Crystal code | Modified TLR (crystal I) | Modified TLR (crystal II) |
|---|---|---|
| **Crystal data** | | |
| Space group | R3 | R3 |
| Unit cell (Å) | a = b = 83.2, c = 67.2 | a = b = 82.6, c = 70.2 |
| **Data collection** | | |
| Beam line | Photon Factory-Advanced Ring NE-3A | Photon Factory BL-17A |
| Wavelength (Å) | 1.0 | 1.10611 |
| Resolution (Å) | 30.4-2.9 | 31.9-2.9 |
| Outer-shell resolution (Å) | 3.0-2.9 | 3.0-2.9 |
| Unique reflections | 7623 | 7926 |
| Completeness (%) | 98.8 | 97.8 |
| Outer-shell completeness (%) | 95.0 | 99.5 |
| $R_{merge}$ (%) | 6.3 | - |
| Outer-shell $R_{merge}$ (%) | 28.6 | - |
| $R_{anom}$ (%) | - | 6.8 |
| Outer-shell $R_{anom}$ (%) | - | 27.3 |
| Multiplicity | 2.7 | 2.6 |
| Outer-shell multiplicity | 2.4 | 2.6 |
| $I/\sigma$ (I) | 13.8 | 11.1 |
| Outer-shell $I/\sigma$ (I) | 3.3 | 4.1 |
| **Structure refinement** | | |
| Resolution (Å) | 30.4-2.9 | 31.9-2.9 |
| Reflection used | 7619 | 7871 |
| R factor (%) | 17.2 | 16.5 |
| $R_{free}$ (%) | 19.9 | 20.1 |
| R.m.s.d. bond length (Å) | 0.007 | 0.007 |
| R.m.s.d. bond angle (°) | 0.9 | 1.0 |

For the modified TLR, crystals were obtained under 2 types of crystallization conditions (referred to as crystal I and crystal II, respectively), both of which successfully underwent structural analysis (Tables 9, and the three-dimensional structure of the crystal I is shown in Figure 17D).

The secondary structure and three-dimensional structure of the unmodified TLR are shown in Figures 17A and 17B, respectively (Coonrod, L. A., et al., Biochemistry, 2012, 51(42), 8330-8337). Compared thereto, no remarkable difference was found in the three-dimensional structure of the modified TLR (Figure 17D). The crystals I and II did not differ in the three-dimensional structure, demonstrating that similarly to the bulged-G structure and Kink-turn structure, the tetraloop receptor structure was also a stable three-dimensional structure regardless of the crystallization conditions. Furthermore, the interaction between 2 molecules between the tetraloop structure (the inside of the dashed-line box in Figure 18) and the tetraloop receptor structure (the space-filling model in Figure 18) was observed to find that the modified

TLR and the unmodified TLR did not significantly differ in the interaction (Figure 18).

Observation of the conformation by every nucleotide showed that the conformation of the sugar moiety of the nucleotides was maintained between the modified TLR and unmodified TLR. This is thought to be because DNA and RNA can take both C3'-endo and C2'-endo conformation. However, replacement of RNA, which relatively tends to take the C3'-endo conformation, by DNA, which relatively tends to take the C2'-endo conformation, still resulted in conformation constraint, indicating the importance of maintaining the conformation of the sugar moiety before and after modification.

In addition, the interaction between nucleotides found in the interaction between the tetraloop (TL) structure and the tetraloop receptor (TLR) structure was observed (Figures 19 and 20). It was found that some hydrogen bonds were destructed by replacement of the hydroxy group at the 2' position of ribose, involved in hydrogen bonding, by hydrogen (the circles in Figures 19E and 19F, and the circles in Figure 20). However, hydrogen bonding between bases was not affected, and the interaction between the tetraloop structure and the tetraloop receptor structure was maintained, and this indicates that even when the hydroxy group at the 2' position of ribose is involved in hydrogen bonding, unbulky substituent such as hydrogen would not significantly affect the structure and function.

<Example 6: Evaluation of Stability of RNA Molecule with mutation and Nucleic Acid Molecule That Hybridizes Thereto to Induce Three-dimensional Structure>

(Purpose)

The stability of double-stranded nucleic acids in which a three-dimensional structure is induced by hybridization when RNA molecules have mutation is investigated.

(Method)

1. Design and Synthesis of Nucleic Acid Molecule

The experiment was performed in accordance with the method in Example 1. The nucleic acids used were as follows.

As nucleic acids of interest with mutation, an RNA molecule which has the base sequence UUU near the center (ROI-U; the uppers in Figures 21A and 21C; SEQ ID NO: 31) and an RNA molecule which has a mutation in the base sequence near the center into AAA (ROI-A; the uppers in Figures 21B and 21D; SEQ ID NO: 32) were designed. As an artificial nucleic acid, an RNA molecule that induces a Kink-turn structure such that the mutated portion described above will be comprised in a bulge structure (KT-SKIP; the lowers in Figures 21C and 21D; SEQ ID NO: 34) was designed based on the consensus sequence of the three-dimensional structure. In addition, an RNA molecule having the complementary sequence to the ROI-U (ASO-A; the lowers in Figures 21A and 21B; SEQ ID NO: 33) was designed as a control.

(Results)

The results are shown in Figure 22.

The melting curves were sigmoidal for any of the nucleic acids used, demonstrating that double strands can be formed by using any of the nucleic acids together with the nucleic acid of interest (Figure 22). In the case where the control ASO-A was used, the thermal melting curve of the double-stranded nucleic acid formed with the ROI-A was remarkably shifted to lower temperature compared to the double-stranded nucleic acid formed with the ROI-U (Figure 22A). The $T_m$ value for the double-stranded nucleic acid formed with the ROI-U was 86°C, while the value for the double-stranded nucleic acid formed with the ROI-A was 78°C, demonstrating that the mutation significantly affected the stability of the double-stranded nucleic acid. In cases using the KT-SKIP in which a three-dimensional structure was induced such that the mutated portion was comprised in a bulge structure, the thermal melting curve for the double-stranded nucleic acid formed with the ROI-U and the thermal melting curve for the double-stranded nucleic acid formed with the ROI-A were not remarkably different (Figure 22B), and both $T_m$ values were 75°C.

This demonstrates that, by inducing a three-dimensional structure such that the mutated portion is comprised in a bulge structure, an artificial nucleic acid that hybridizes with a nucleic acid of interest at comparable levels regardless of the presence of mutation can be designed.

<Example 7: Inhibition of Protein Expression Using Nucleic Acid Molecules that Hybridize to Induce Three-dimensional Structure>

(Purpose)

The possibility of inhibition of the protein expression using nucleic acid molecules that hybridize to induce three-dimensional structures is determined, and compared to the inhibition effects when using antisense nucleic acids.

(Method)

As nucleic acids of interest, mRNAs that were transcription products from the pUC-frGFP DNA (SEQ ID NO: 35) included in a protein synthesis kit "Musaibo Kun N Mini" (TAIYO NIPPON SANSO Corporation) were used. As target regions for hybridization, specific regions (the 277- to 301-positions in SEQ ID NO: 35; the uppers in Figures 23A to 23C and the uppers in Figures 28A to 28C; SEQ ID NO: 36) were used. As artificial nucleic acids, RNA molecules that hybridize with a target region to induce a tandem GA structure (GA-GFP; the lower in Figure 23B and the lower in Figure 28B; SEQ ID NO: 38) or induce a Kink-turn structure (KT-GFP; the lower in Figure 23C and the lower in Figure 28C; SEQ ID NO: 39) were designed based on the consensus sequence of the three-dimensional structure. As control RNA molecules, an RNA molecule having the same sequence as the target region (SO-GFP; the uppers in Figures 23A to 23C and the uppers in Figures 28A to 28C; SEQ ID NO: 36) and an RNA molecule having the complementary sequence to the target region (ASO-GFP; the lower in Figure 23A and the lower in Figure 28A; SEQ ID NO: 37) were designed.

The nucleic acids were synthesized in accordance with Example 1.

Protein synthesis was performed with or without addition of the designed nucleic acid molecules. Protein synthesis was performed using a protein synthesis kit "Musaibo Kun N Mini" (TAIYO NIPPON SANSO Corporation) according to the protocol recommended by the manufacturer. Briefly, the protocol is as follows.

The premix reaction solution and the pUC-frGFP DNA (final concentration of 0.5 nM) from "Musaibo Kun N Mini", and designed RNA molecules (SO-GFP, ASO-GFP, GA-GFP, KT-GFP: each final concentration of 20 μM) were mixed to prepare 50 μl of reaction solutions. As a negative control, a reaction solution without addition of RNA was also prepared. These reaction solutions were incubated at 30°C for 90 minutes in PCR Thermal Cycler Dice Touch (Takara) to allow the protein synthesis reaction, and then incubated at 4°C for 5 minutes to terminate the reaction. The reaction solutions were used for the following experiments.

The fluorescence spectrum was measured to investigate the protein synthesis inhibition effect.

For the measurement of the fluorescence spectrum, the fluorescence spectrum within the range of 500-600 nm was measured with FP-8300 (Jasco) using an excitation wavelength of 480 nm.

The experiments were performed at room temperature.

(Results)

The results are shown in Figure 24.

For the reaction solution without addition of any designed RNA molecules, a clear peak derived from frGFP was observed at 510 nm (Figure 24A). In the case with addition of SO-GFP, a comparable peak was observed at the same wavelength (Figure 24B). This demonstrates that addition of RNA molecules *per se* does not affect the protein synthesis or fluorescence detection. In the case with addition of ASO-GFP, the height of the peak was reduced, indicating that the protein translation was inhibited (Figure 24C). Both the cases with addition of GA-GFP and KT-GFP resulted in little or no peak observed at 510 nm (Figures 24D and 24E).

This demonstrates that nucleic acids that induce three-dimensional structures can be used to inhibit protein synthesis with higher efficiency than the case using conventional ASOs.

<Example 8: Detection of Target RNA with Fluorescence-labeled RNA Probe Containing 2-aminopurine (Fluorescence-labeled Nucleic Acid Probe Containing RNA as Main Constituent Nucleotides)>

Using a target RNA and non-target RNAs of SEQ ID NOs: 40-43 (Table10), detection of the target RNA with 2-aminopurine-containing fluorescence-labeled RNA probe was investigated.

[Table 10]

Table 10. Base sequence of target and non-target RNAs

| | Base sequence |
|---|---|
| Target RNA | 5'-ACU CUC CUC GAC UCU UCA UCA UCA UGU-3' (SEQ ID NO: 40) |
| Non-target RNA 1 | 5'-CGA GCG CCC CAC CAC CC-3' (SEQ ID NO: 41) |
| Non-target RNA 2 | 5'-GGG CCG GUC CCC CGG G-3' (SEQ ID NO: 42) |
| Non-target RNA 3 | 5'-CCC GGU UCU UGG CCG GCC C-3'(SEQ ID NO: 43) |

As shown in Figure 25, the base sequence of the fluorescence-labeled RNA probe was designed (Figure 25) to hybridize with the target RNA (SEQ ID NO: 40) to form a Kink-turn structure, and to contain a 2'-deoxyribonucleotide having a fluorescent base, 2-aminopurine (2-aminopurine, 2AP), at the L3-position in the Kink-turn motif (X in the sequence in Figure 25). All nucleotides in the fluorescence-labeled RNA probe were RNA other than the 2'-deoxyribonucleotide having a fluorescent base described above (herein, thus-designed 2AP-containing fluorescence-labeled RNA probe is referred to as RNA-2AP). The base sequence of the designed RNA-2AP is shown below.

5'-ACA UGA UGA UGA AGA GGA XGA GAG GAG AGU-3' (SEQ ID NO: 44) (wherein, X = 2AP)

The above-described fluorescence-labeled RNA probe (RNA-2AP), target RNA, and 3 types of non-target RNAs (non-target RNAs 1-3) were chemically synthesized by a phosphoramidite method using an automatic nucleic acid synthesizer NTS-M2-MX (Nihon Techno Service Co., Ltd). As phosphoramidite for 2-aminopurine-containing nucleotide, 2-aminopurine-CE phosphoramidite (Glen Research) was used. The samples obtained by the chemical synthesis described above were purified by gel filtration using NAP-10 columns (Cytiva), and then subjected to electrophoresis using a 20% denaturing polyacrylamide gel containing 7 M urea to confirm that the desired RNAs were properly synthesized with high purity.

Whether the synthesized fluorescence-labeled RNA probe (RNA-2AP) can specifically detect the target RNA in a base sequence-specific manner was investigated by the fluorescence spectrum measurement as described below.

RNA-2AP and 0.01 mM target RNA or non-target RNAs were added to a solution containing 10 mM sodium cacodylate (pH7) and 100 mM sodium chloride to the final concentrations of 0.01 mM, to prepare solutions (test solutions). As a control, only 0.01 mM RNA-2AP was added to a solution containing 10 mM sodium cacodylate (pH7) and 100 mM sodium chloride, to prepare a solution (control solution).

The 3D fluorescence spectrum for the control solution was measured using FP-8300 (Jasco) to determine the maximum excitation wavelength and the maximum fluorescence wavelength. Based on the results, the measurement conditions used in the following fluorescence spectrum measurement (the excitation wavelength and the range of the fluorescence wavelength to be detected) were determined.

Using the measurement conditions determined as described above, the fluorescence spectra for the test solutions were measured with FP-8300 (Jasco). Specifically, the fluorescence spectra in the range of 330 to 450 nm were measured using an excitation wavelength of 305 nm.

Furthermore, the rate of change in the fluorescence intensity ΔF when the target RNA or non-target RNAs were added to RNA-2AP was calculated by the following equation, to evaluate the ability of RNA-2AP to detect the target RNA.

$$\Delta F = (F - F_0) / F$$

(F: the fluorescence intensities at a specific wavelength for test solutions, $F_0$: the fluorescence intensity at the same wavelength for the control solution)

The experiments were performed at room temperature.

The results are shown in Figure 26.

RNA-2AP showed little or no change in the fluorescence intensity in all the cases where 3 types of the non-target RNAs were added, but showed significantly increased fluorescence intensity in the case where the target RNA was added (Figure 26 A). The rate of change in the fluorescence intensity at the wavelength of 370 nm in cases where the target RNA or non-target RNAs were added to RNA-2AP was calculated. The rate of change in the fluorescence intensity when the target RNA was added was ΔF = 0.59, which was remarkably higher than the rate of change in the fluorescence intensity when the non-target RNAs were added (ΔF = -0.05 to 0.07) (Figure 26B). These results show that RNA-2AP can detect the target RNA in a base sequence-specific manner.

All publications, patents, and patent applications cited herein are incorporated herein by reference in their entirety.

## Claims

1. An artificial nucleic acid for inducing a specific functional three-dimensional structure by hybridizing with a nucleic acid of interest that does not form a functional three-dimensional structure, wherein

   said artificial nucleic acid comprises a three-dimensional structure formation-inducing domain that forms a three-dimensional structure together with a target domain in said nucleic acid of interest,
   said target domain and said three-dimensional structure formation-inducing domain form a sequence motif composed of double strands forming the specific three-dimensional structure,
   in said sequence motif, said three-dimensional structure formation-inducing domain and said target domain comprises complementary regions consisting of mutually complementary sequences,
   in said sequence motif, said three-dimensional structure formation-inducing domain and/or said target domain further comprises a non-complementary-containing region with 1 or more bases comprising a mutually non-complementary sequence, and
   said non-complementary-containing region comprises non-complementary sequences at its both ends.

2. The artificial nucleic acid of claim 1, further comprising a hybridizable domain(s) composed of 6 to 120 bases, adjacent to one or both side(s) of said three-dimensional structure formation-inducing domain.

3. The artificial nucleic acid of claim 1 or 2, wherein said three-dimensional structure formation-inducing domain and/or said target domain comprises a plurality of said complementary regions, and wherein said non-complementary-containing region is located between said plurality of the complementary region.

4. The artificial nucleic acid of any one of claims 1 to 3, wherein said non-complementary-containing region is composed of 2 to 7 bases.

5. The artificial nucleic acid of any one of claims 1 to 4, wherein said specific three-dimensional structure comprises 1 or more selected from the group consisting of Kink-turn structure, bulged-G structure, Reverse Kink-turn structure, 5S loop E structure, C-loop structure, and tandem GA structure.

6. The artificial nucleic acid of claim 5, wherein

   the Kink-turn structure is composed of 5'-NNNNGAN-3' and 5'-NGAN-3',
   the bulged-G structure is composed of 5'-NNNGUAN-3' and 5'-NGANNN-3',
   the Reverse Kink-turn structure is composed of 5'-NNNNAAN-3' and 5'-NGAN-3',
   the 5S loop E structure is composed of 5'-NGUAN-3' and 5'-NGAUN-3',
   the C-loop structure is composed of 5'-NCACU-3' and 5'-ANN-3', or
   the tandem GA structure is composed of 5'-NGAN-3' and 5'-NGAN-3', and
   wherein N is A, C, G, or U in the base sequences.

7. The artificial nucleic acid of any one of claims 1 to 6, wherein the nucleic acid of interest is mRNA or miRNA.

8. The artificial nucleic acid of any one of claims 1 to 7, wherein said hybridization is performed under high stringent conditions.

9. The artificial nucleic acid of any one of claims 1 to 8, wherein said three-dimensional structure formation-inducing domain comprises 1 or more modified nucleotides.

10. The artificial nucleic acid of claim 9, wherein said modified nucleotide is selected from the group consisting of 2'-OMe RNA, 2'-MOE RNA, LNA, 2'-O, 5'-N BNA, 2'-deoxy-trans-3',4'-BNA, and DNA.

11. The artificial nucleic acid of claim 9 or 10, wherein said modified nucleotide comprises fluoro group-modification at the 2' position of the ribose.

12. The artificial nucleic acid of any one of claims 1 to 11, wherein said target domain comprises said non-complementary-containing region containing a mutation.

13. The artificial nucleic acid of claim 12, wherein said mutation is a single nucleotide variant, insertion-deletion mutation,

structural variant, or a combination thereof.

**14.** A gene expression inhibiting agent, comprising the artificial nucleic acid of any one of claims 1 to 13 as an active ingredient.

**15.** A nucleic acid detecting agent, comprising the artificial nucleic acid of any one of claims 1 to 13 as an active ingredient.

**16.** A method for producing an artificial nucleic acid for inducing a specific three-dimensional structure by hybridizing with a nucleic acid of interest that does not form a functional three-dimensional structure, comprising:

a target domain selecting step of searching said nucleic acid of interest for the sequence information for one side of a sequence motif composed of double strands forming said specific three-dimensional structure, and selecting 1 or more of said sequence information as a target domain;
a three-dimensional structure formation-inducing domain determining step of determining the sequence of said three-dimensional structure formation-inducing domain such that said three-dimensional structure formation-inducing domain forms a sequence motif together with said target domain; and
a nucleic acid synthesizing step of synthesizing said artificial nucleic acid based on the sequence information determined in said three-dimensional structure formation-inducing domain determining step, wherein
in said sequence motif, said target domain and said three-dimensional structure formation-inducing domain comprise complementary regions consisting of mutually complementary sequences,
in said sequence motif, said target domain and/or said three-dimensional structure formation-inducing domain further comprises a non-complementary-containing region with 1 or more bases comprising a mutually non-complementary sequence, and
said non-complementary-containing region comprises non-complementary sequences at its both ends.

Fig. 1

A

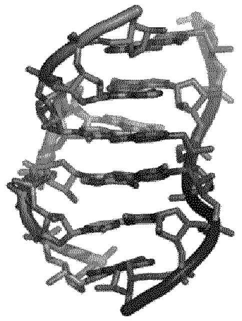

B

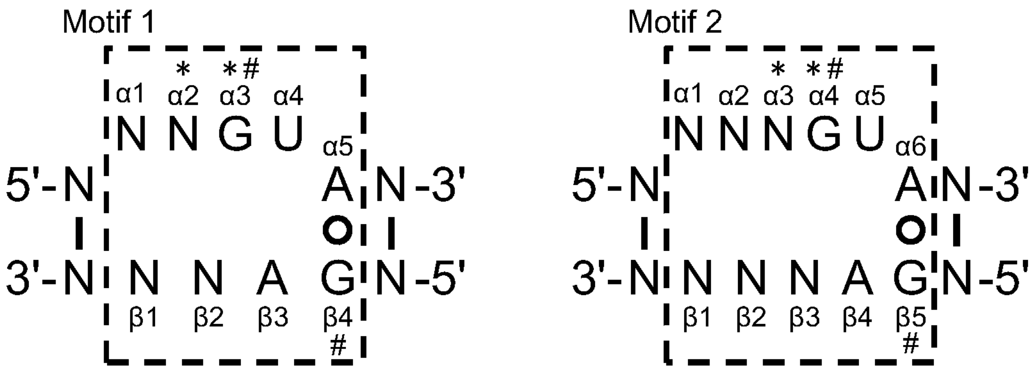
Motif 1
α1 α2 α3 α4 α5
5'-N N N G U A N-3'
3'-N N N A G N-5'
β1 β2 β3 β4
Motif 2
α1 α2 α3 α4 α5 α6
5'-N N N N G U A N-3'
3'-N N N N A G N-5'
β1 β2 β3 β4 β5

Fig. 2

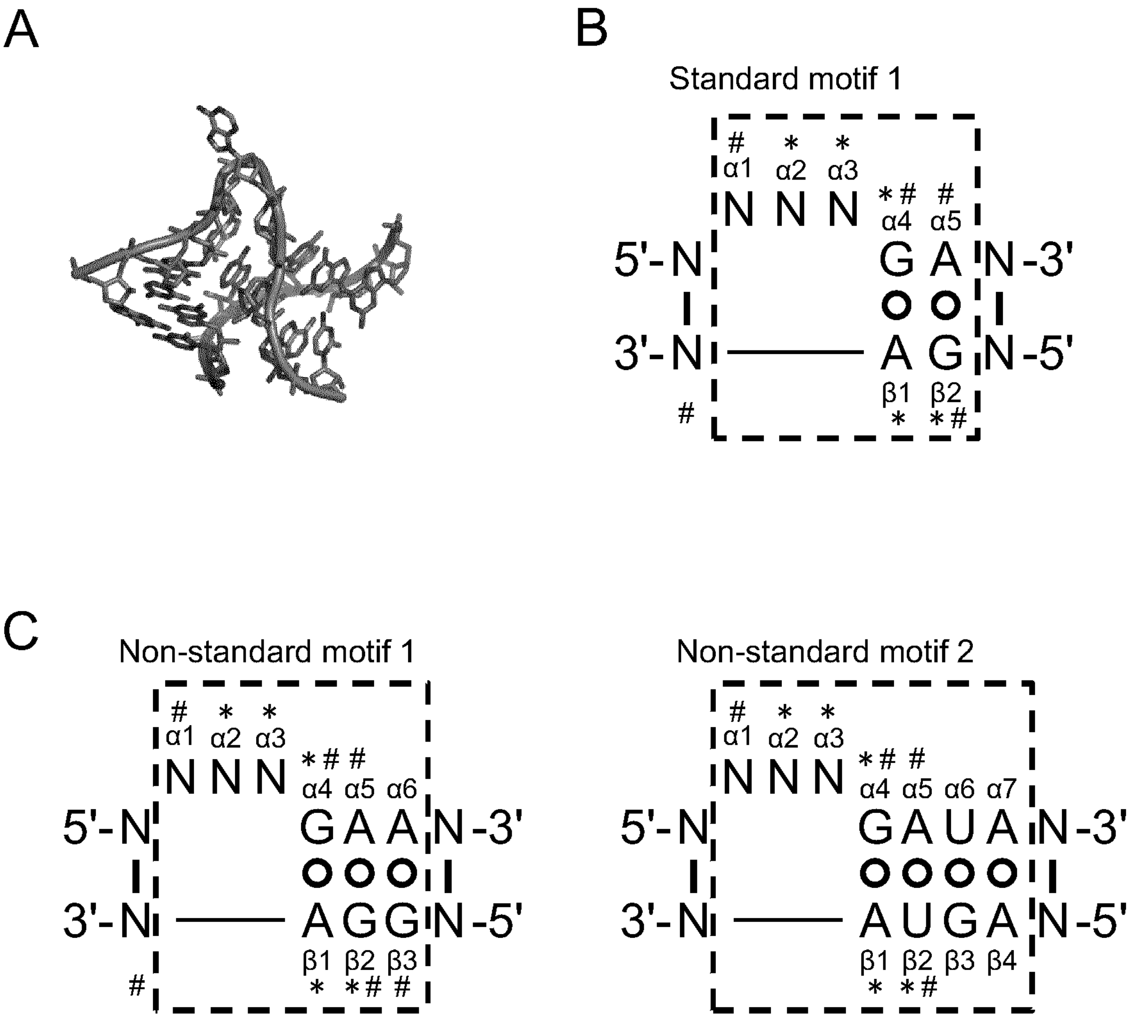
A
B
Standard motif 1
# * *
α1 α2 α3
N N N
*# #
α4 α5
5'-N G A N-3'
3'-N A G N-5'
β1 β2
#
* *#
C
Non-standard motif 1
# * *
α1 α2 α3
N N N
*# #
α4 α5 α6
5'-N G A A N-3'
3'-N A G G N-5'
β1 β2 β3
#
* *# #
Non-standard motif 2
# * *
α1 α2 α3
N N N
*# #
α4 α5 α6 α7
5'-N G A U A N-3'
3'-N A U G A N-5'
β1 β2 β3 β4
* *#

Fig. 3

A

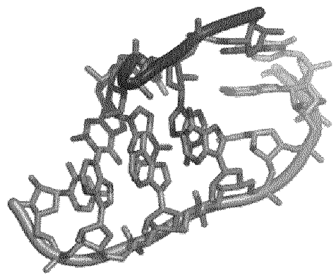

B

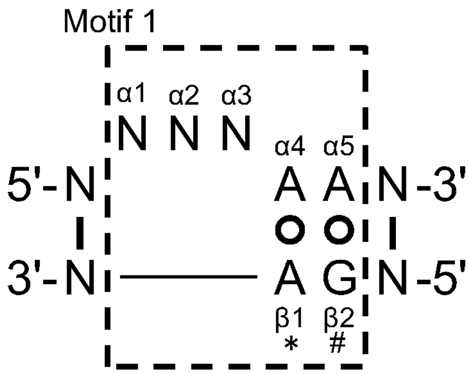

Fig. 4

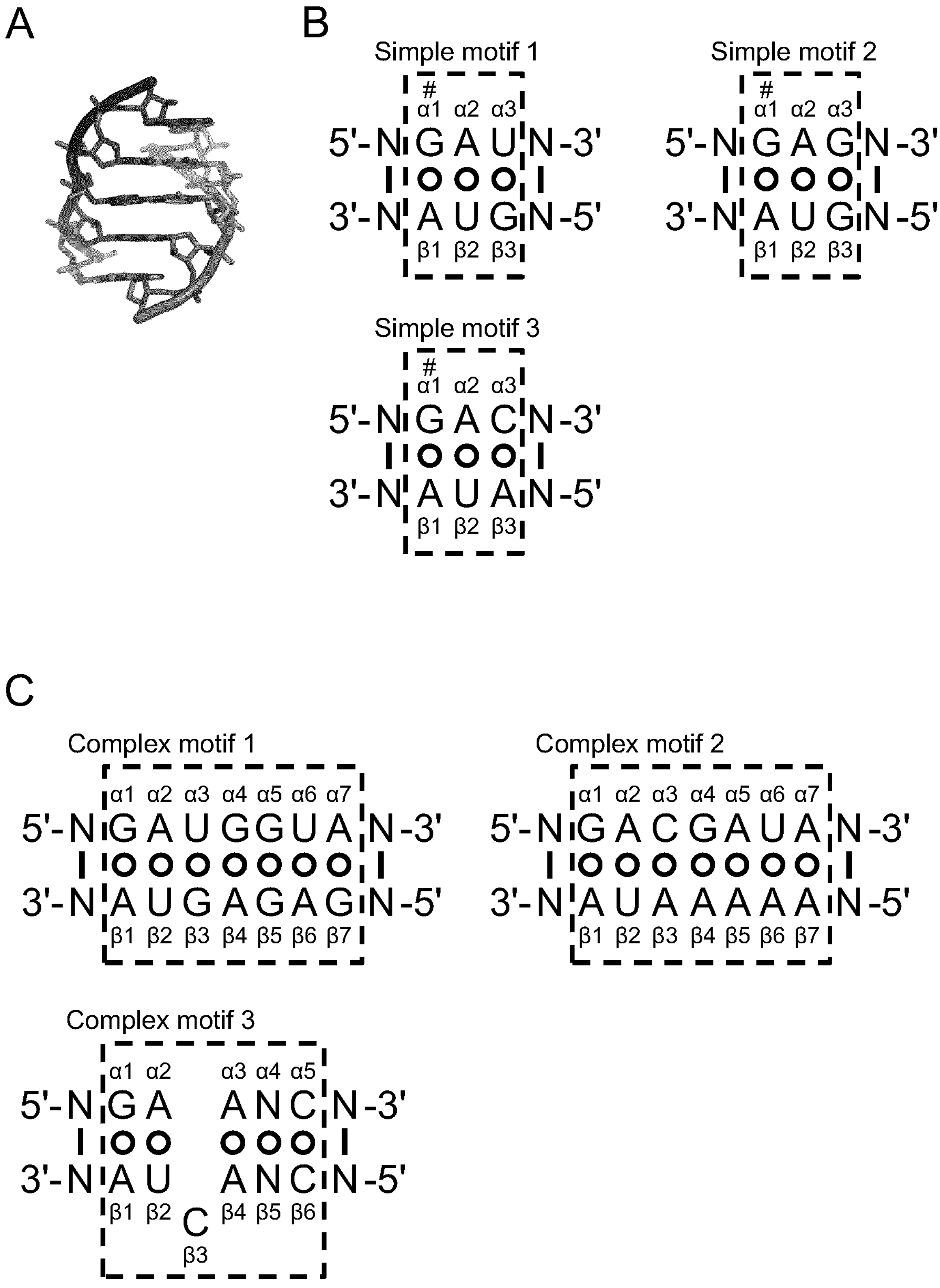
A
B
Simple motif 1
#
α1 α2 α3
5'-N G A U N-3'
3'-N A U G N-5'
β1 β2 β3
Simple motif 2
#
α1 α2 α3
5'-N G A G N-3'
3'-N A U G N-5'
β1 β2 β3
Simple motif 3
#
α1 α2 α3
5'-N G A C N-3'
3'-N A U A N-5'
β1 β2 β3
C
Complex motif 1
α1 α2 α3 α4 α5 α6 α7
5'-N G A U G G U A N-3'
3'-N A U G A G A G N-5'
β1 β2 β3 β4 β5 β6 β7
Complex motif 2
α1 α2 α3 α4 α5 α6 α7
5'-N G A C G A U A N-3'
3'-N A U A A A A A N-5'
β1 β2 β3 β4 β5 β6 β7
Complex motif 3
α1 α2 α3 α4 α5
5'-N G A A N C N-3'
3'-N A U A N C N-5'
β1 β2 C β4 β5 β6
β3

Fig. 5

A

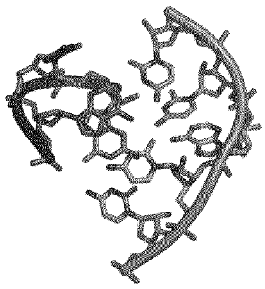

B

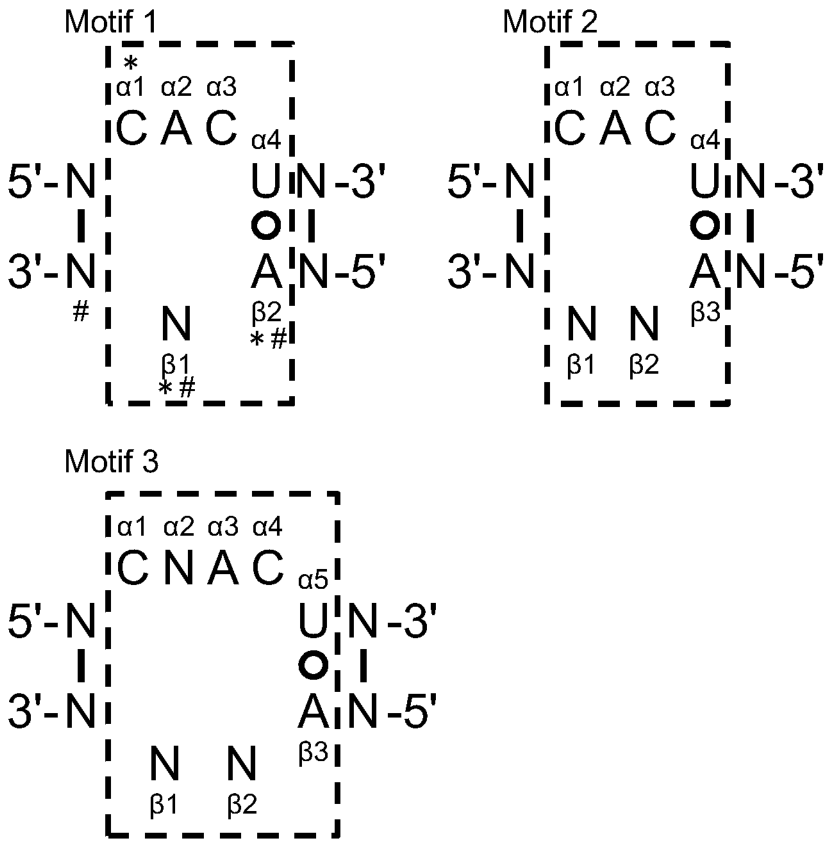
Motif 1
Motif 2
Motif 3
α1 α2 α3
C A C
α4
5'-N
U N-3'
3'-N
A N-5'
N
β1
β2
β3
N N
β1 β2
α1 α2 α3 α4
C N A C
α5

Fig. 6

A

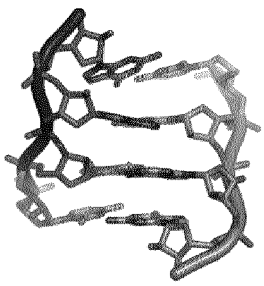

B

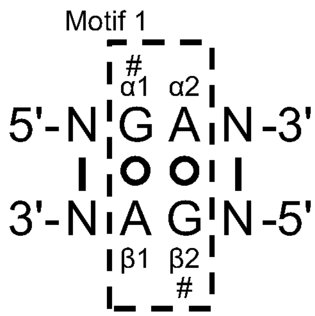

# Fig. 7

A

```
     ROI: 5'-CAU GGC UCU UGC AGA UGC GAU GUU GC -3'  (SEQ ID NO: 13)
             ||| ||| ||| ||| ||| ||| ||| ||| ||
 RNA-ASO: 3'-GUA CCG AGA ACG UCU ACG CUA CAA CG -5'  (SEQ ID NO: 20)
```

B

```
     ROI: 5'-CAU GGC UCU UGC AGA UGC GAU GUU GC -3'  (SEQ ID NO: 13)
             ||| ||| ||| ||| |o      | ||| ||| ||
  RNA-BG: 3'-GUA CCG AGA ACG UA      G CUA CAA CG -5'  (SEQ ID NO: 14)
                              UGAU
```

C

```
     ROI: 5'-CAU GGC UCU UGC AGA UGC GAU GUU GC -3'  (SEQ ID NO: 13)
             ||| ||| ||| ||| |oo ||| ||| ||| ||
  RNA-KT: 3'-GUA CCG AGA ACG UAG ACG CUA CAA CG -5'  (SEQ ID NO: 15)
                              AAG
```

D

```
     ROI: 5'-CAU GGC UCU UGC AGA UGC GAU GUU GC -3'  (SEQ ID NO: 13)
             ||| ||| ||| ||| |oo ||| ||| ||| ||
 RNA-RKT: 3'-GUA CCG AGA ACG UAA ACG CUA CAA CG -5'  (SEQ ID NO: 16)
                              ACA
```

E

```
     ROI: 5'-CAU GGC UCU UGC AGA UGC GAU GUU GC -3'  (SEQ ID NO: 13)
             ||| ||| ||| ||| |oo o|| ||| ||| ||
  RNA-5S: 3'-GUA CCG AGA ACG UAU GCG CUA CAA CG -5'  (SEQ ID NO: 17)
```

F

```
     ROI: 5'-CAU GGC UCU UGC AGA UGC GAU GUU GC -3'  (SEQ ID NO: 13)
             ||| ||| ||| ||| |oo  || ||| ||| ||
  RNA-CL: 3'-GUA CCG AGA ACG UAU  CG CUA CAA CG -5'  (SEQ ID NO: 18)
                               CAC
```

G

```
     ROI: 5'-CAU GGC UCU UGC AGA UGC GAU GUU GC -3'  (SEQ ID NO: 13)
             ||| ||| ||| ||| |oo ||| ||| ||| ||
  RNA-GA: 3'-GUA CCG AGA ACG UCG ACG CUA CAA CG -5'  (SEQ ID NO: 19)
```

Fig. 8

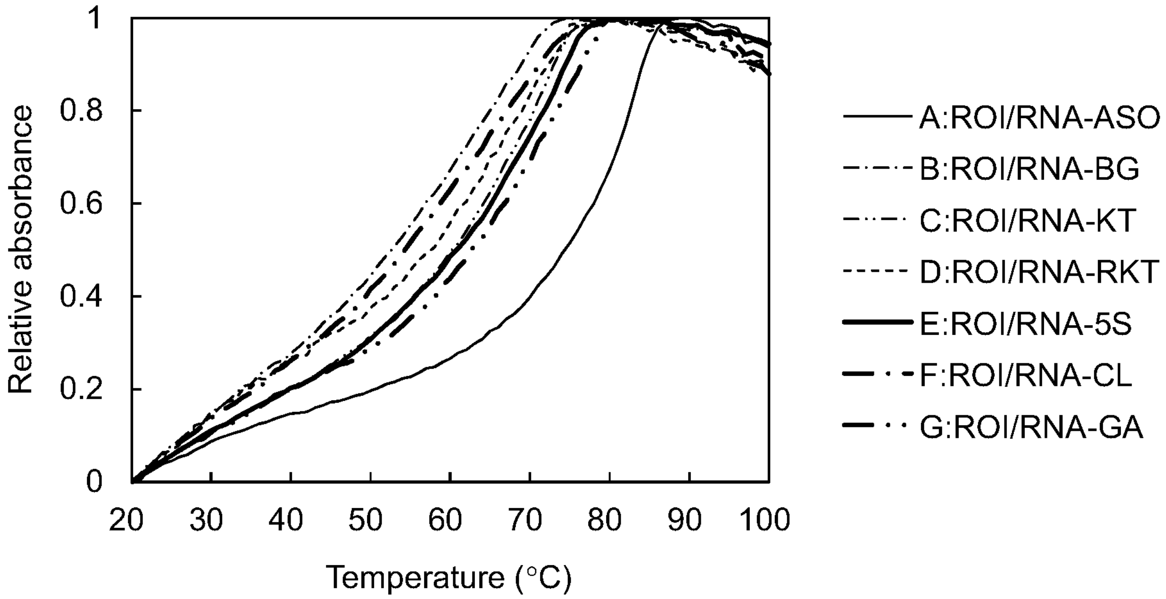
1
0.8
0.6
0.4
0.2
0
Relative absorbance
20
30
40
50
60
70
80
90
100
Temperature (°C)
A:ROI/RNA-ASO
B:ROI/RNA-BG
C:ROI/RNA-KT
D:ROI/RNA-RKT
E:ROI/RNA-5S
F:ROI/RNA-CL
G:ROI/RNA-GA

Fig. 9

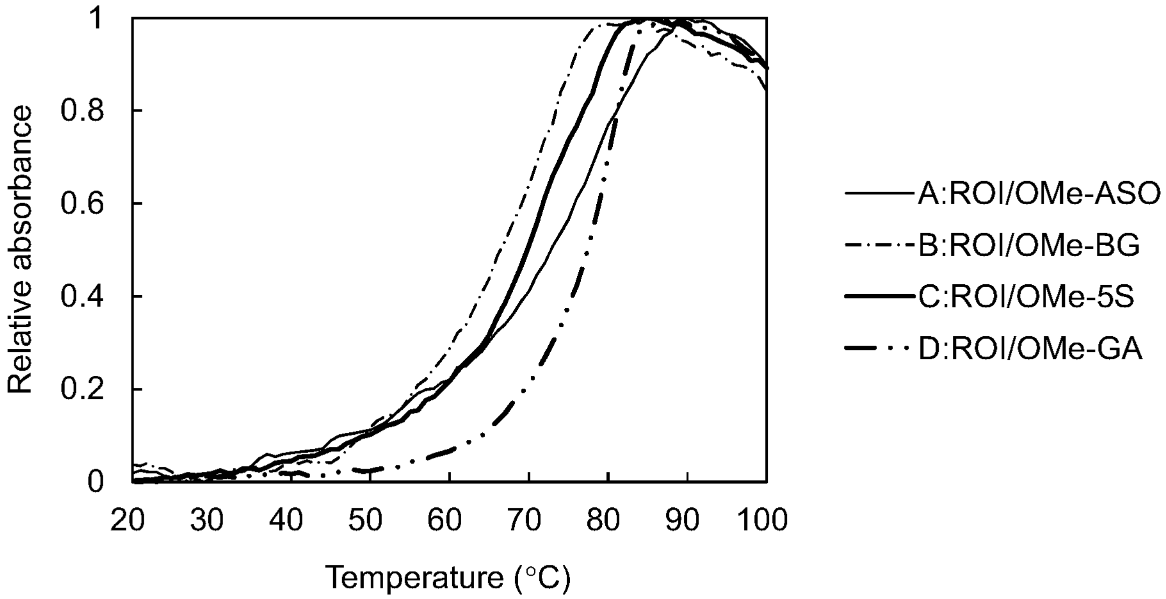
1
0.8
0.6
0.4
0.2
0
20
30
40
50
60
70
80
90
100
Relative absorbance
Temperature (°C)
A:ROI/OMe-ASO
B:ROI/OMe-BG
C:ROI/OMe-5S
D:ROI/OMe-GA

Fig. 10

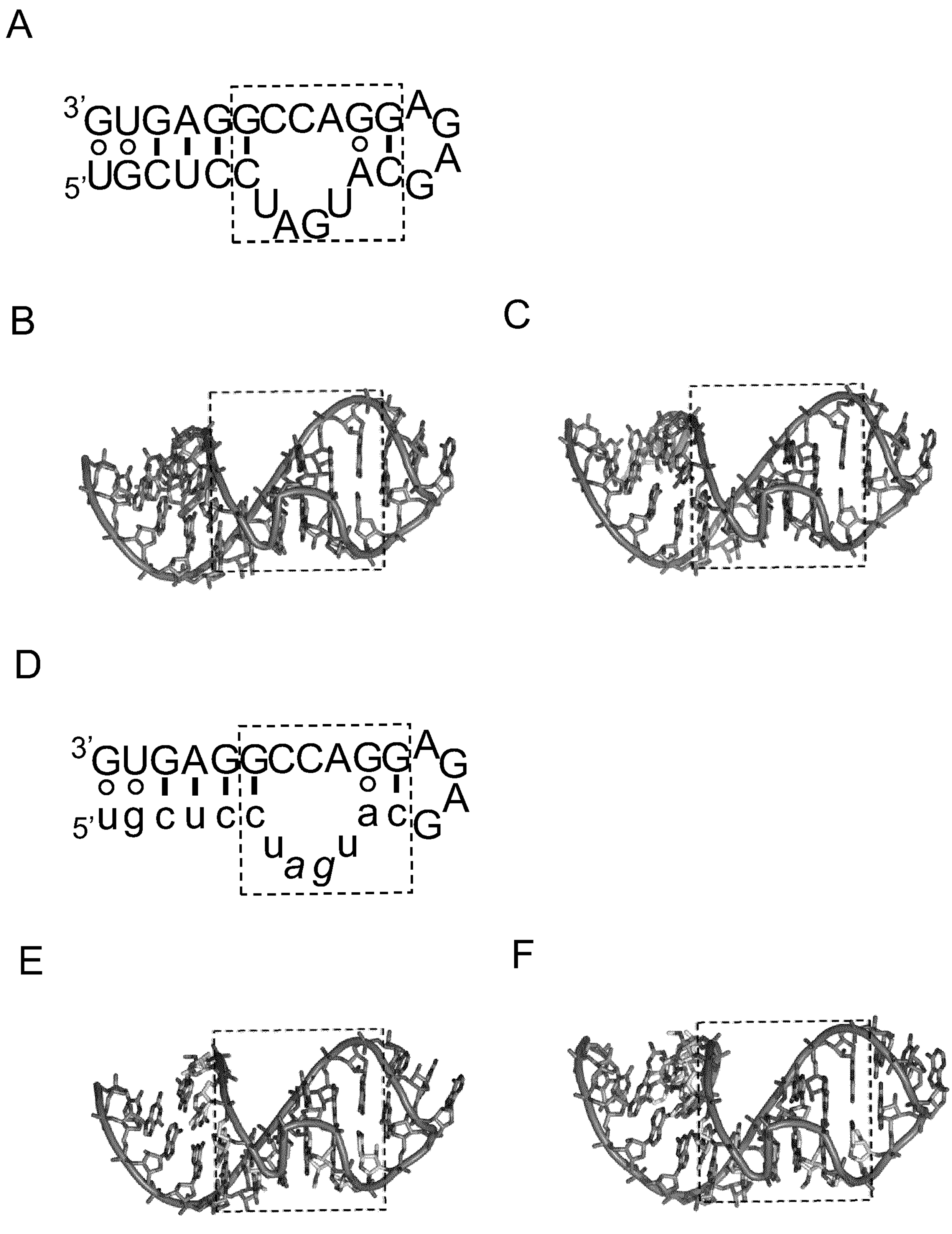
A
3'GUGAGGCCAGGAG
5'UGCUCCUAGUACGA
B
C
D
3'GUGAGGCCAGGAG
5'ugcuccuaguacGA
E
F

Fig. 11

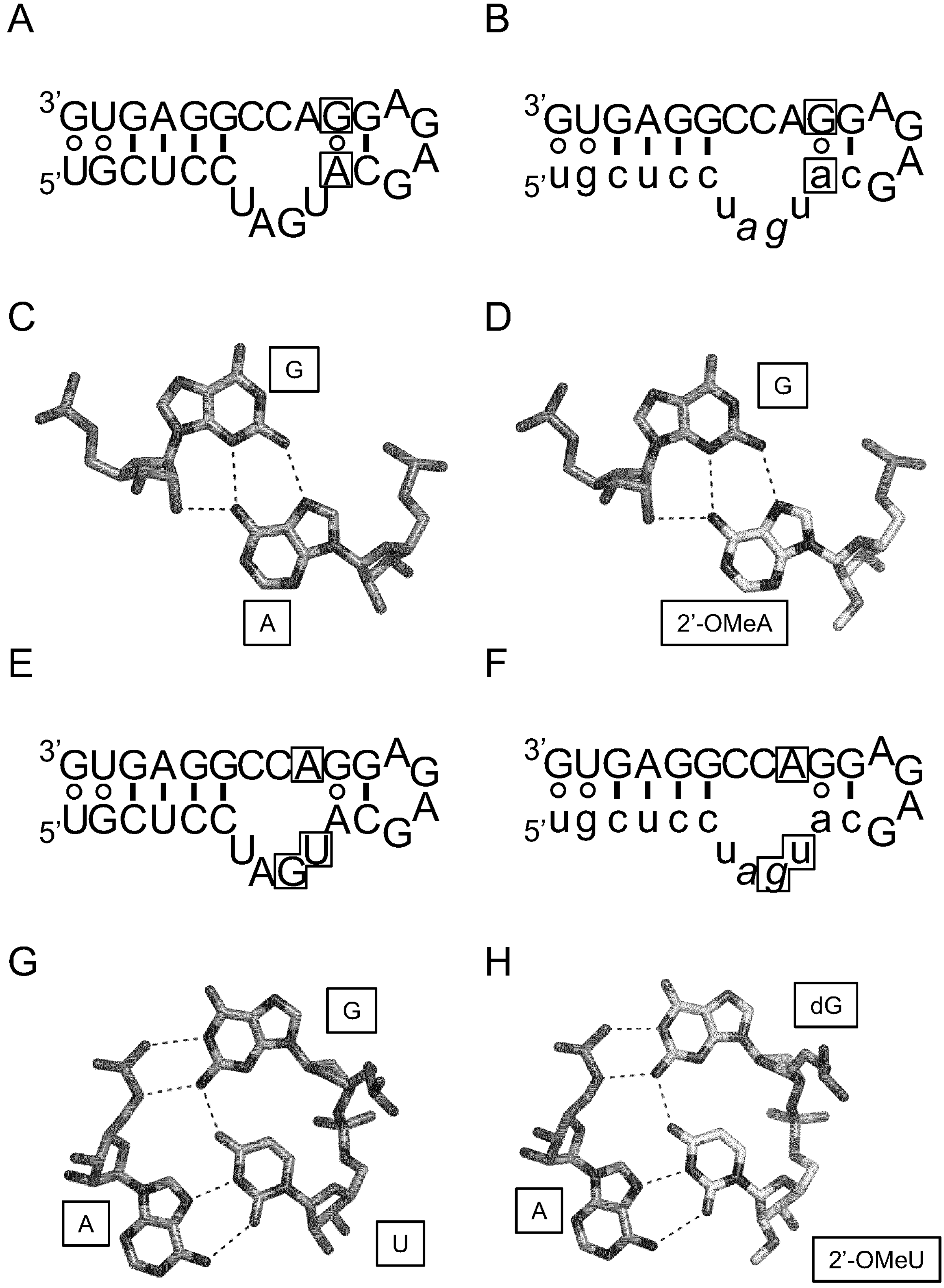

# Fig. 12

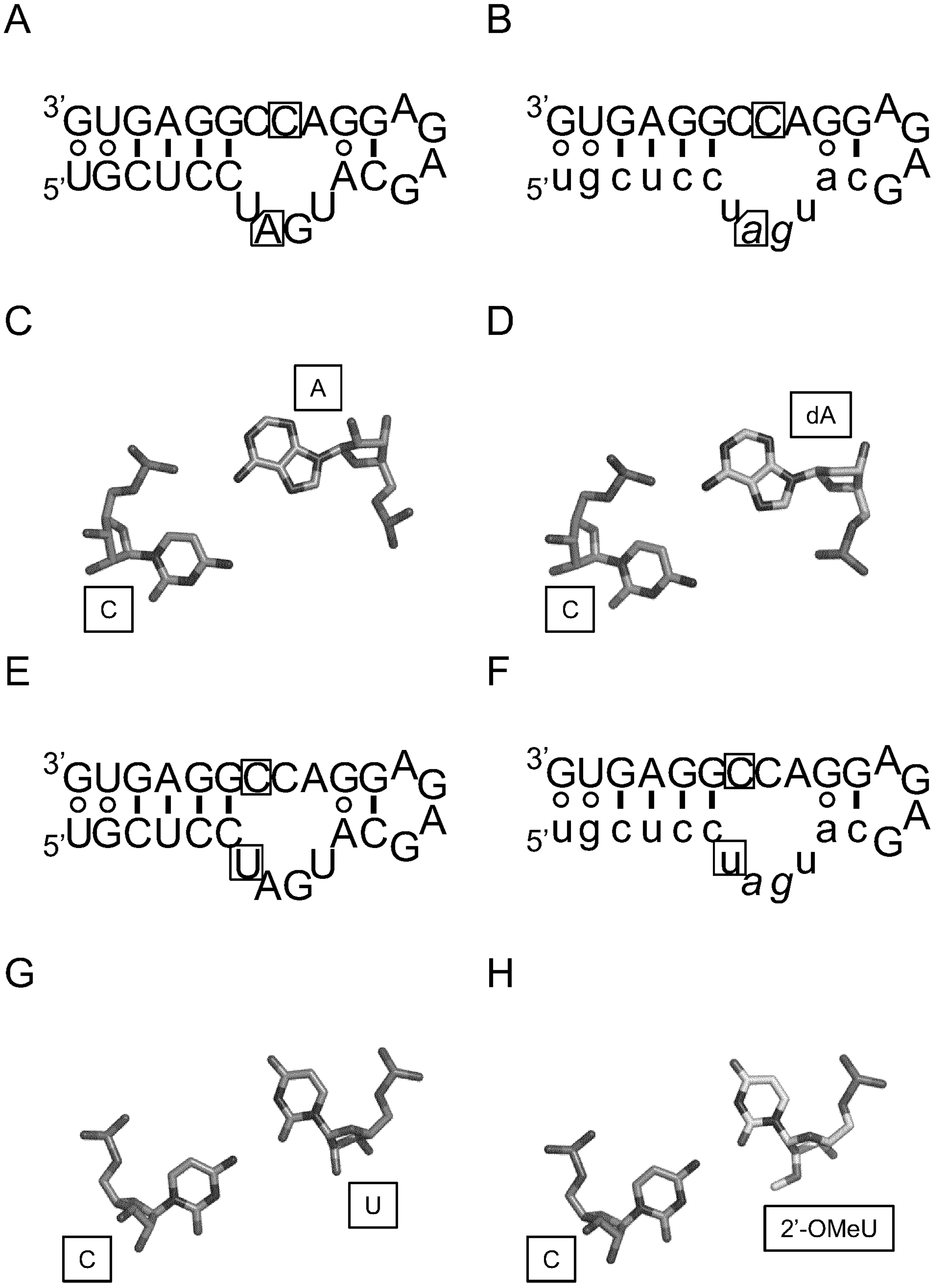
A
B
3'GUGAGGCCAGGAG
5'UGCUCCUAGUACGA
3'GUGAGGCCAGGAG
5'ugcuccuagu acGA
C
D
A
C
dA
C
E
F
3'GUGAGGCCAGGAG
5'UGCUCCUAGUACGA
3'GUGAGGCCAGGAG
5'ugcuccuagu acGA
G
H
U
C
C
2'-OMeU

Fig. 13

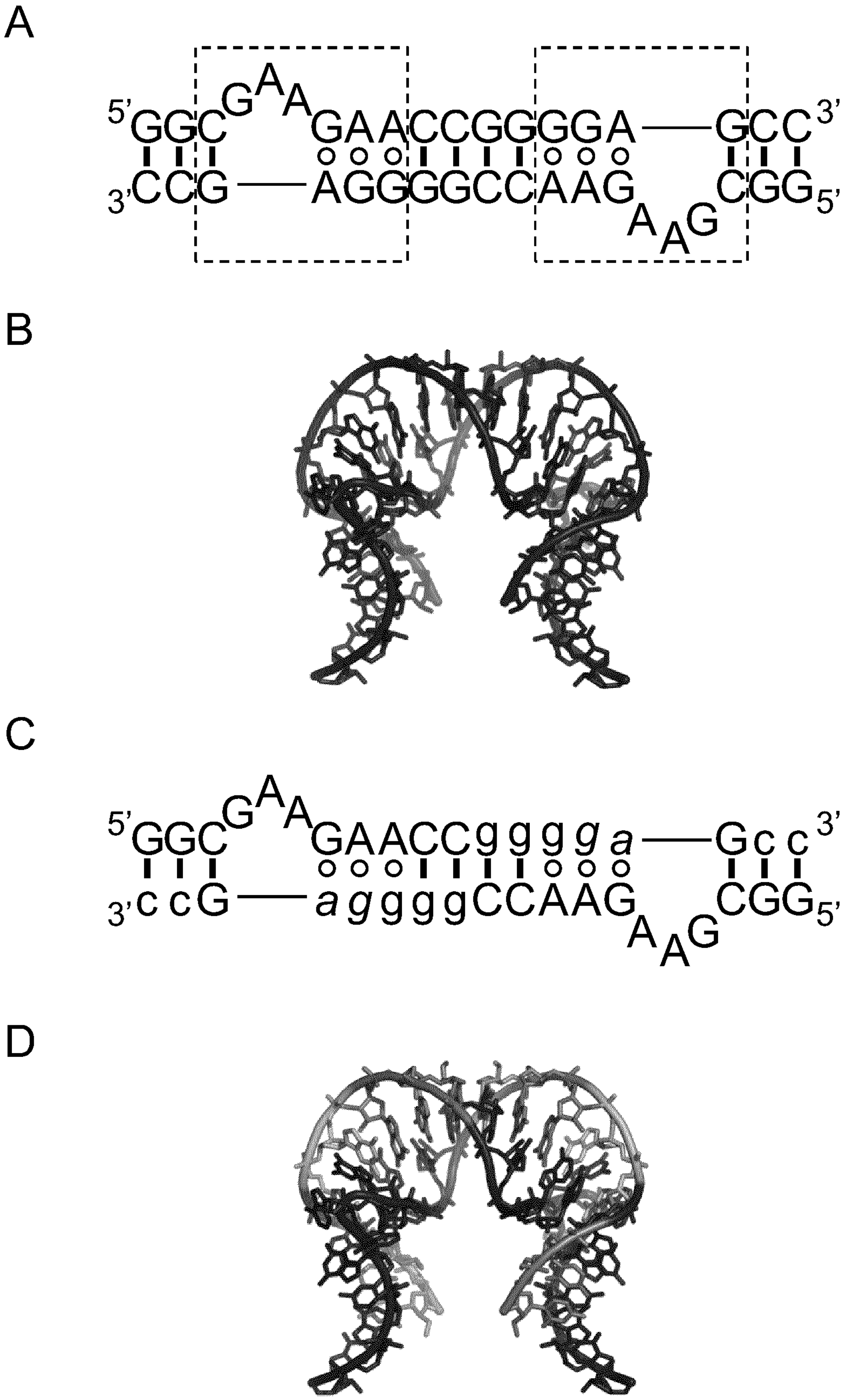
A
5' GGC GAA GAACCGGGGA — GCC 3'
3' CCG — AGGGGCCAAG AAG CGG 5'
B
C
5' GGC GAA GAACCgggga — Gcc 3'
3' ccG — aggggCCAAG AAG CGG 5'
D

Fig. 14

A

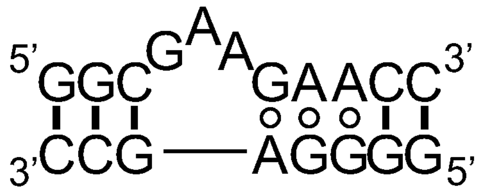


B

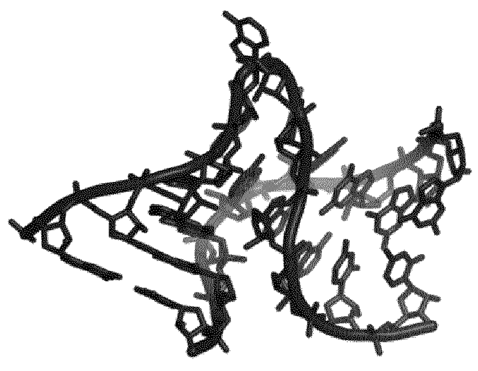

C

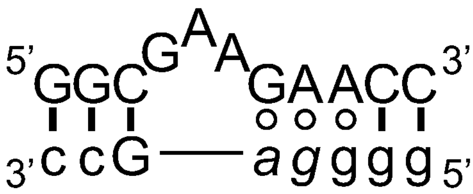


D

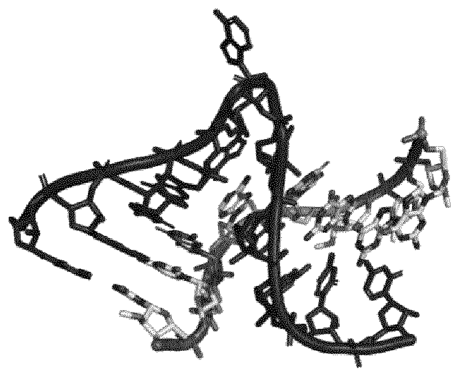

Fig. 15

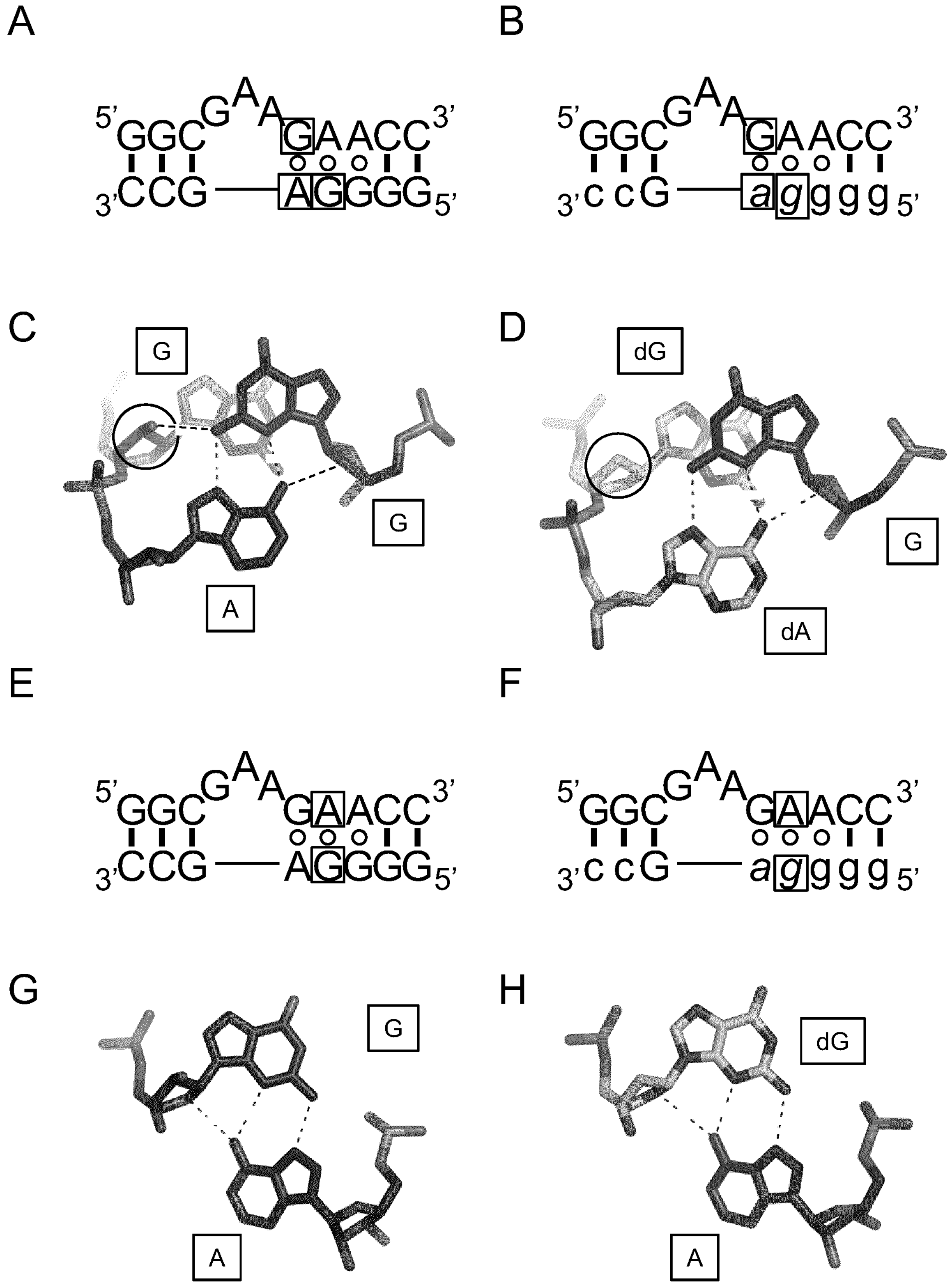

Fig. 16

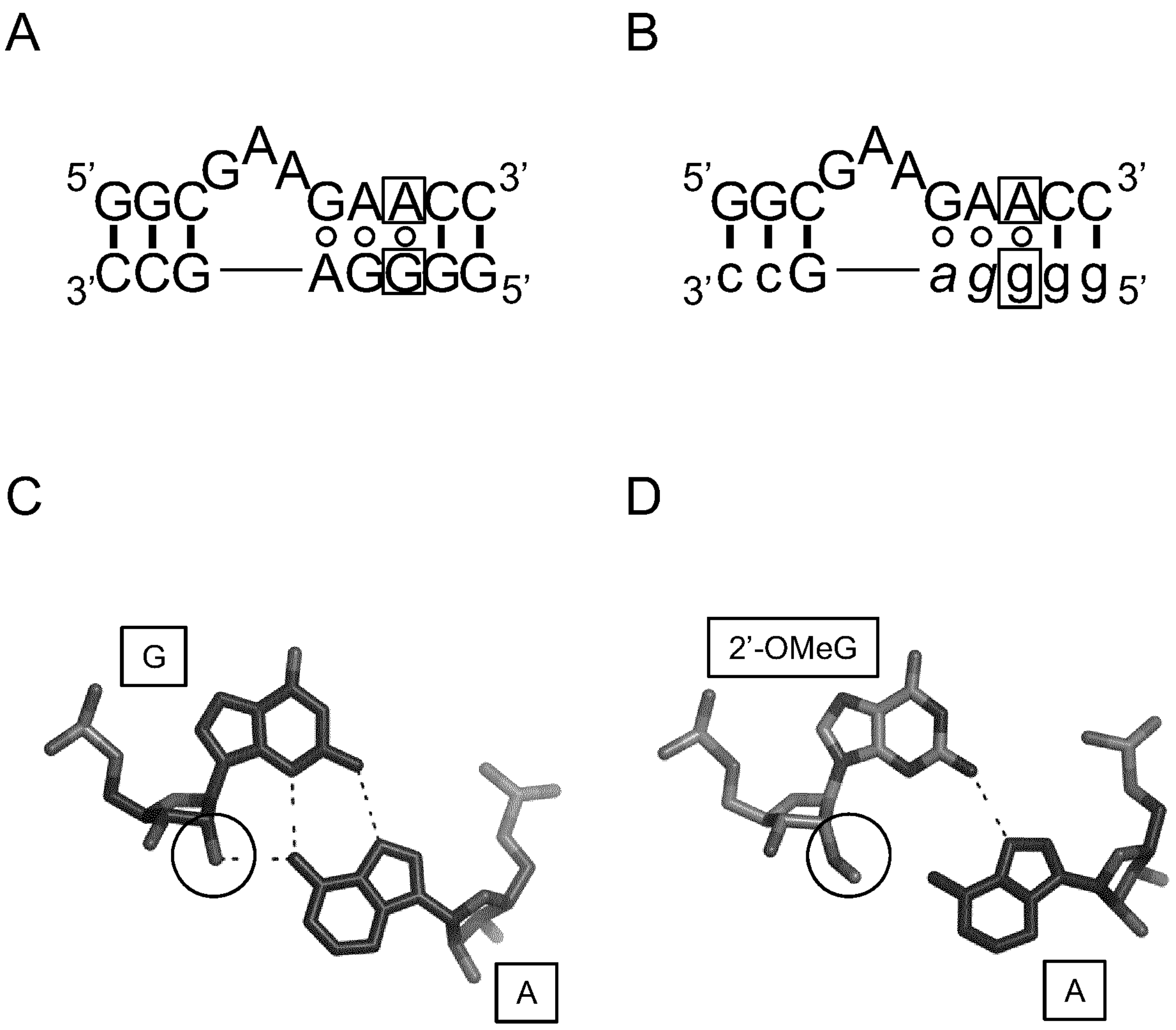
A
5' GGC GAA GAACC 3'
3' CCG AGGGG 5'
B
5' GGC GAA GAACC 3'
3' ccG agggg 5'
C
D

Fig. 17

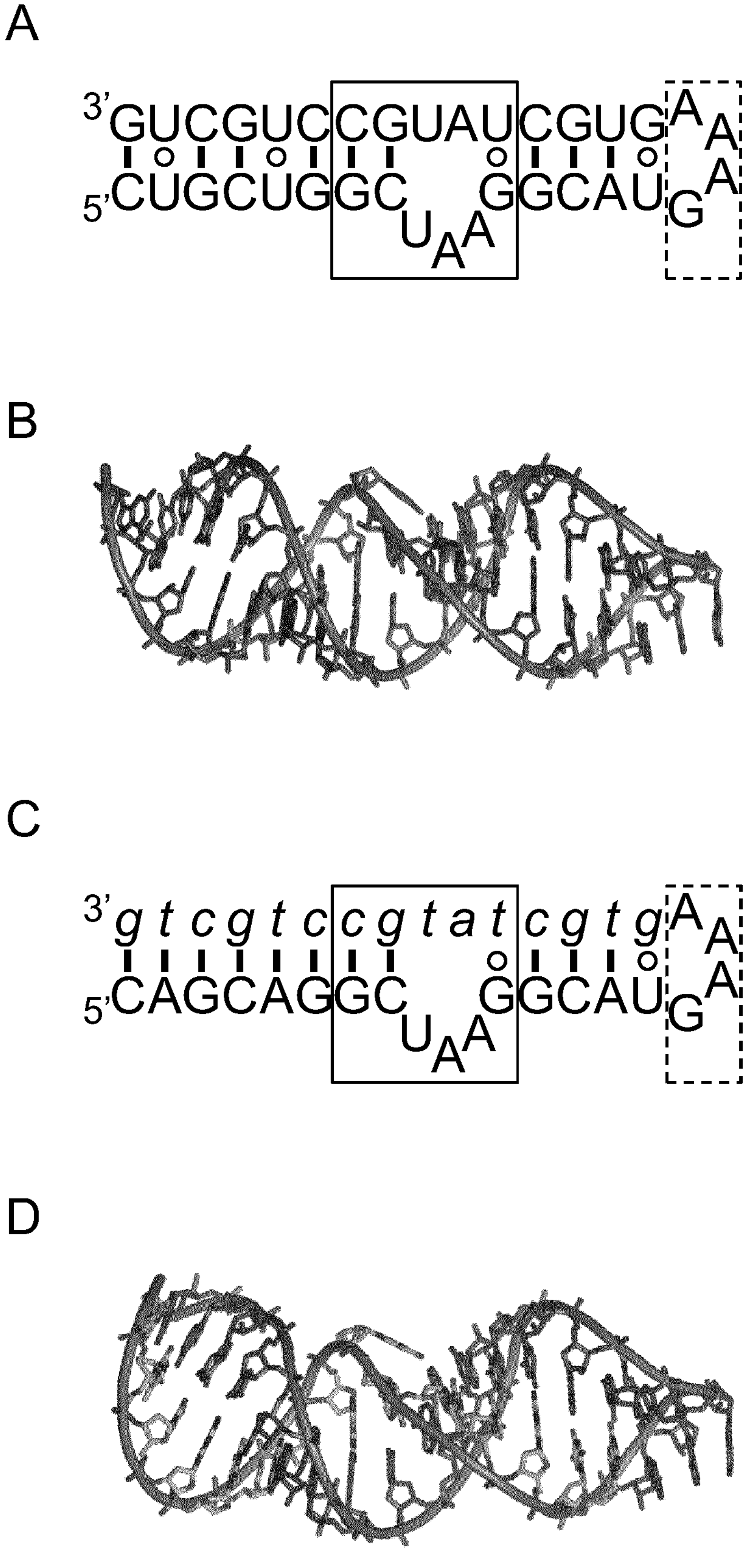
A
3'GUCGUCCGUAUCGUGAA
5'CUGCUGGCUAAGGCAUGA
B
C
3'gtcgtccgtatcgtgAA
5'CAGCAGGCUAAGGCAUGA
D

Fig. 18

A

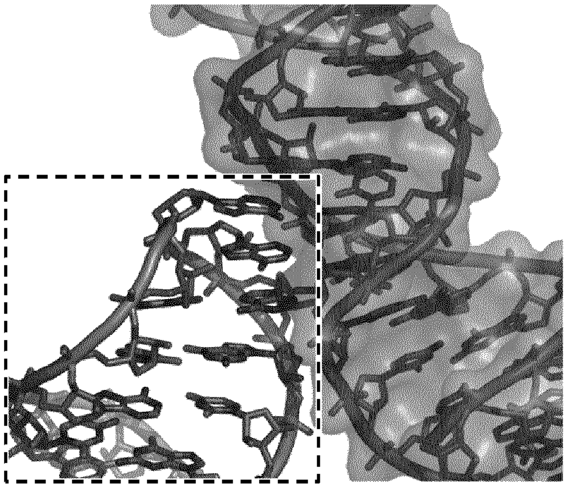

B

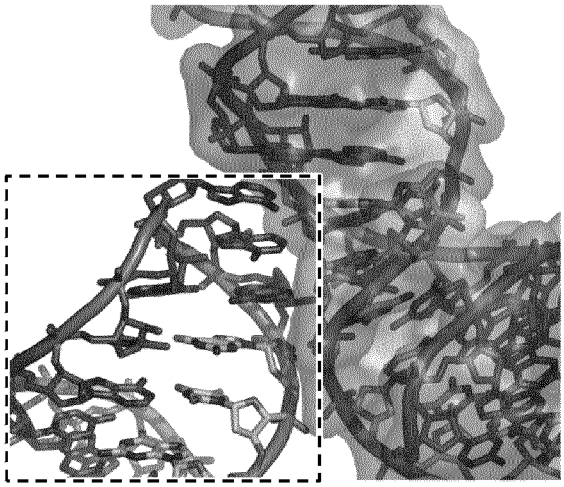

Fig. 19

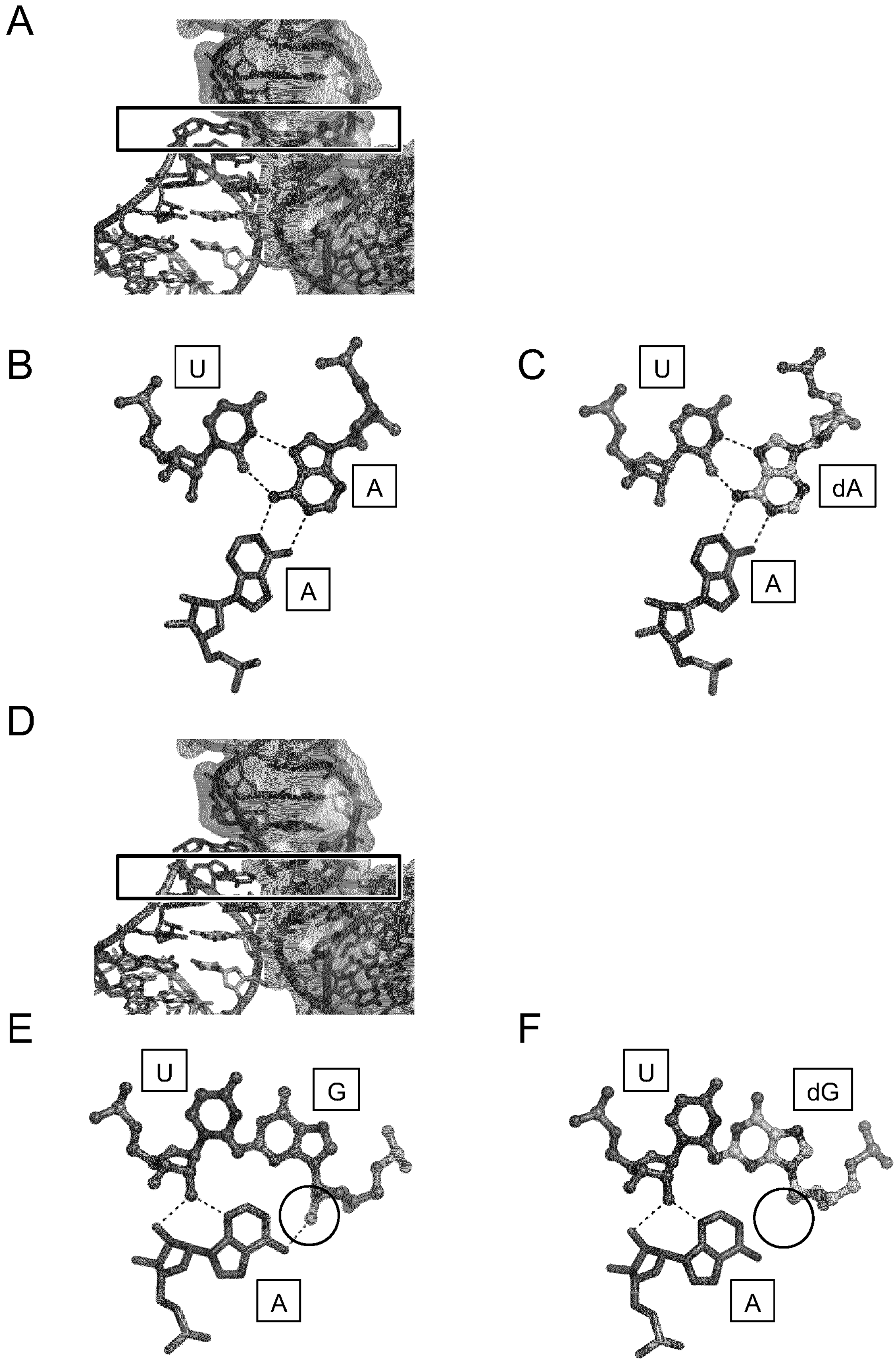

Fig. 20

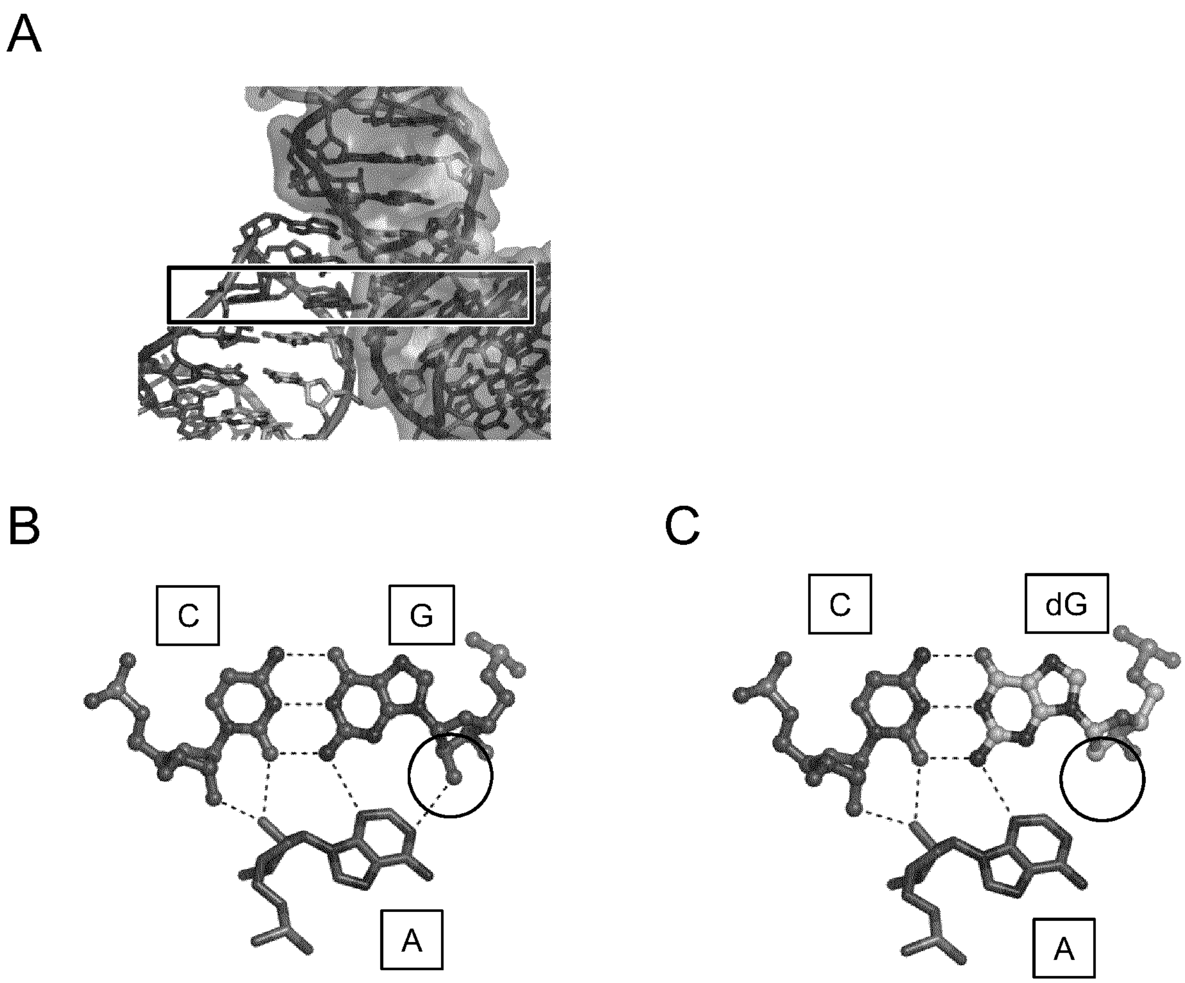

Fig. 21

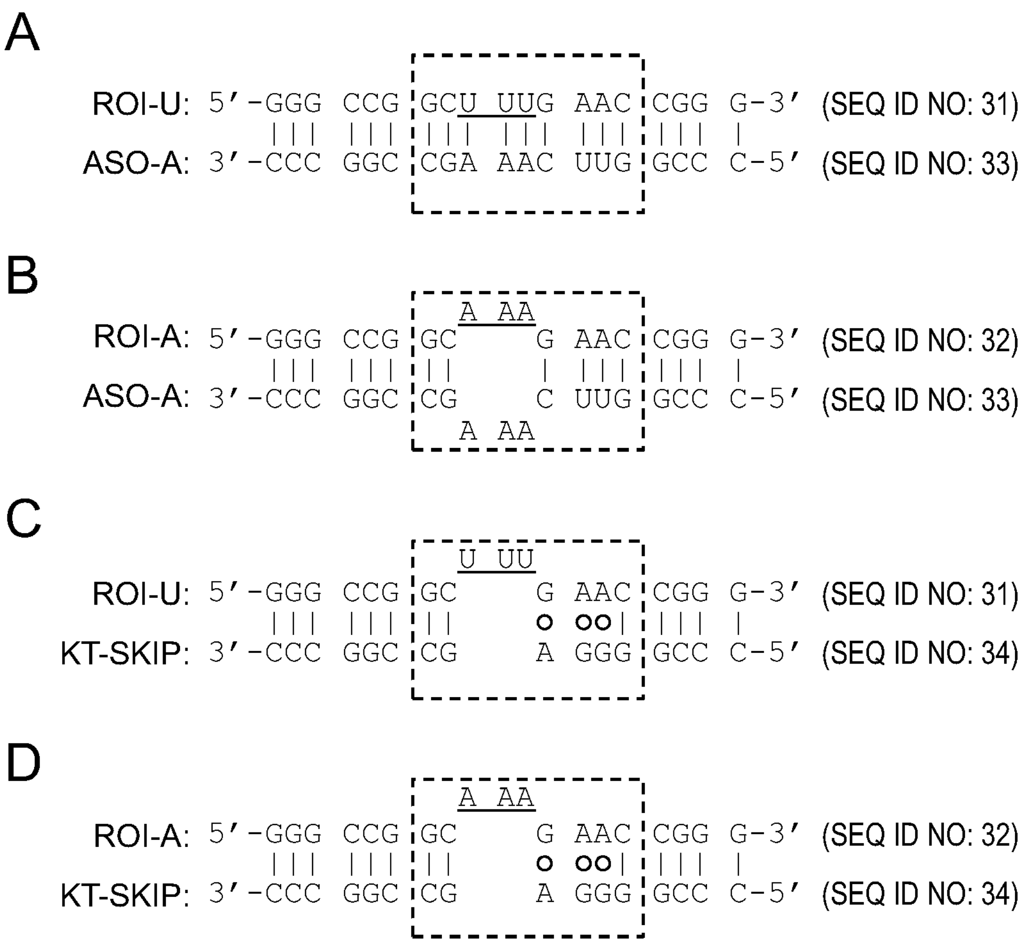
A
ROI-U: 5'-GGG CCG GCU UUG AAC CGG G-3' (SEQ ID NO: 31)
ASO-A: 3'-CCC GGC CGA AAC UUG GCC C-5' (SEQ ID NO: 33)
B
A AA
ROI-A: 5'-GGG CCG GC G AAC CGG G-3' (SEQ ID NO: 32)
ASO-A: 3'-CCC GGC CG C UUG GCC C-5' (SEQ ID NO: 33)
A AA
C
U UU
ROI-U: 5'-GGG CCG GC G AAC CGG G-3' (SEQ ID NO: 31)
KT-SKIP: 3'-CCC GGC CG A GGG GCC C-5' (SEQ ID NO: 34)
D
A AA
ROI-A: 5'-GGG CCG GC G AAC CGG G-3' (SEQ ID NO: 32)
KT-SKIP: 3'-CCC GGC CG A GGG GCC C-5' (SEQ ID NO: 34)

Fig. 22

A

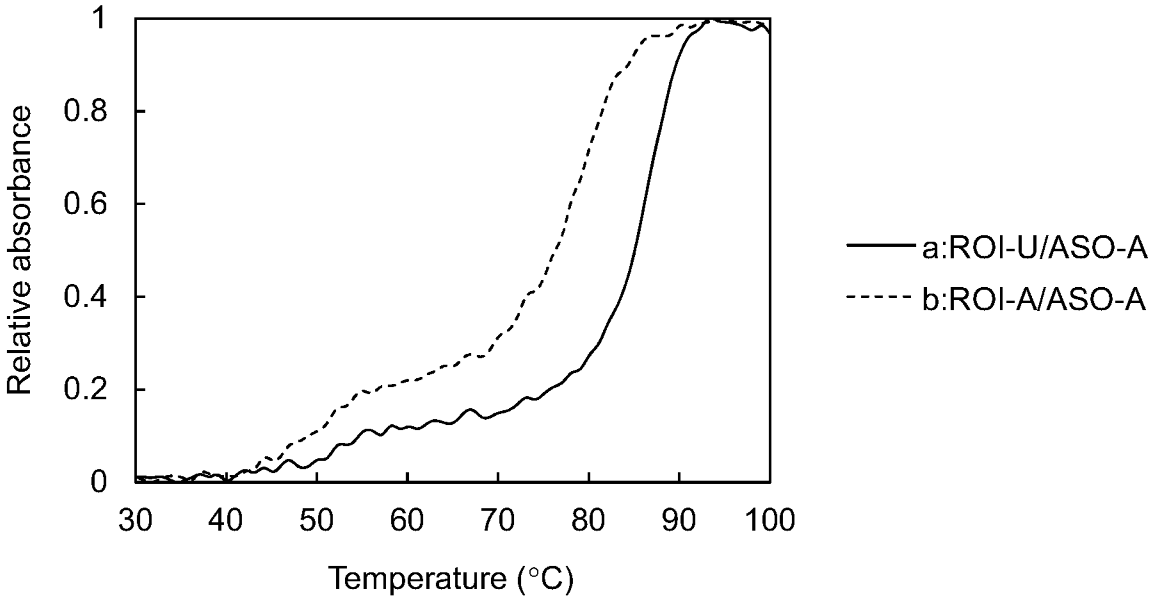

Relative absorbance
1
0.8
0.6
0.4
0.2
0
30
40
50
60
70
80
90
100
Temperature (°C)
a:ROI-U/ASO-A
b:ROI-A/ASO-A


B

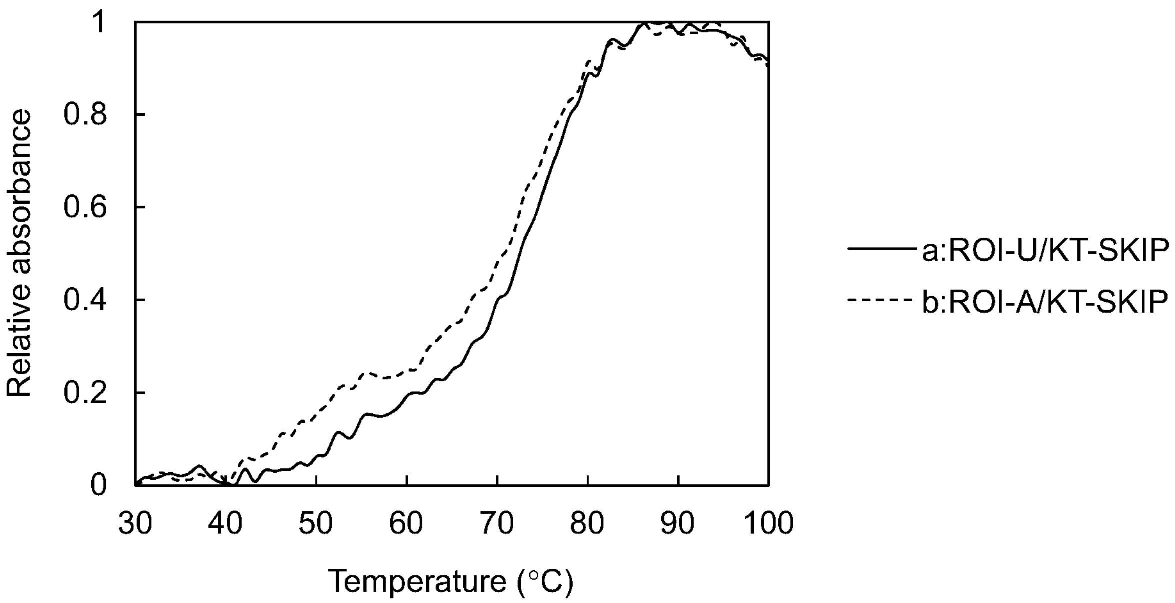

Relative absorbance
1
0.8
0.6
0.4
0.2
0
30
40
50
60
70
80
90
100
Temperature (°C)
a:ROI-U/KT-SKIP
b:ROI-A/KT-SKIP

Fig. 23

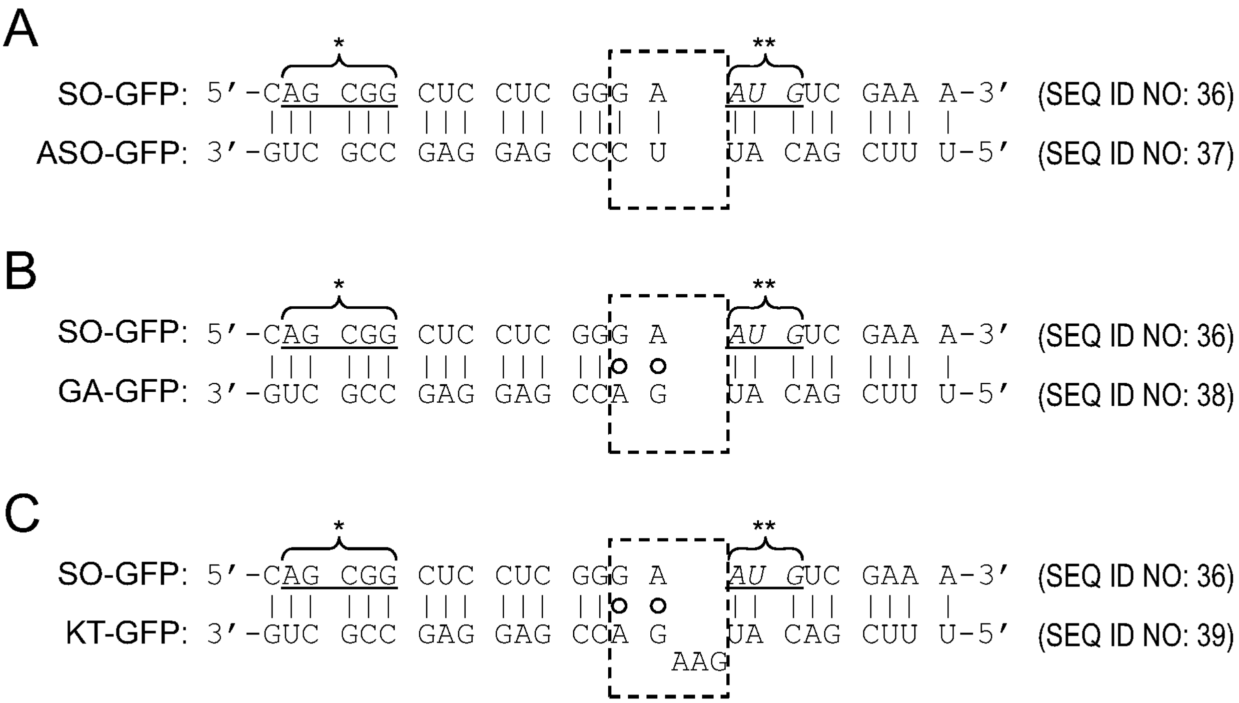
A
*
**
SO-GFP: 5′-CAG CGG CUC CUC GGG A AU GUC GAA A-3′ (SEQ ID NO: 36)
ASO-GFP: 3′-GUC GCC GAG GAG CCC U UA CAG CUU U-5′ (SEQ ID NO: 37)
B
*
**
SO-GFP: 5′-CAG CGG CUC CUC GGG A AU GUC GAA A-3′ (SEQ ID NO: 36)
GA-GFP: 3′-GUC GCC GAG GAG CCA G UA CAG CUU U-5′ (SEQ ID NO: 38)
C
*
**
SO-GFP: 5′-CAG CGG CUC CUC GGG A AU GUC GAA A-3′ (SEQ ID NO: 36)
KT-GFP: 3′-GUC GCC GAG GAG CCA G UA CAG CUU U-5′ (SEQ ID NO: 39)
AAG

Fig. 24

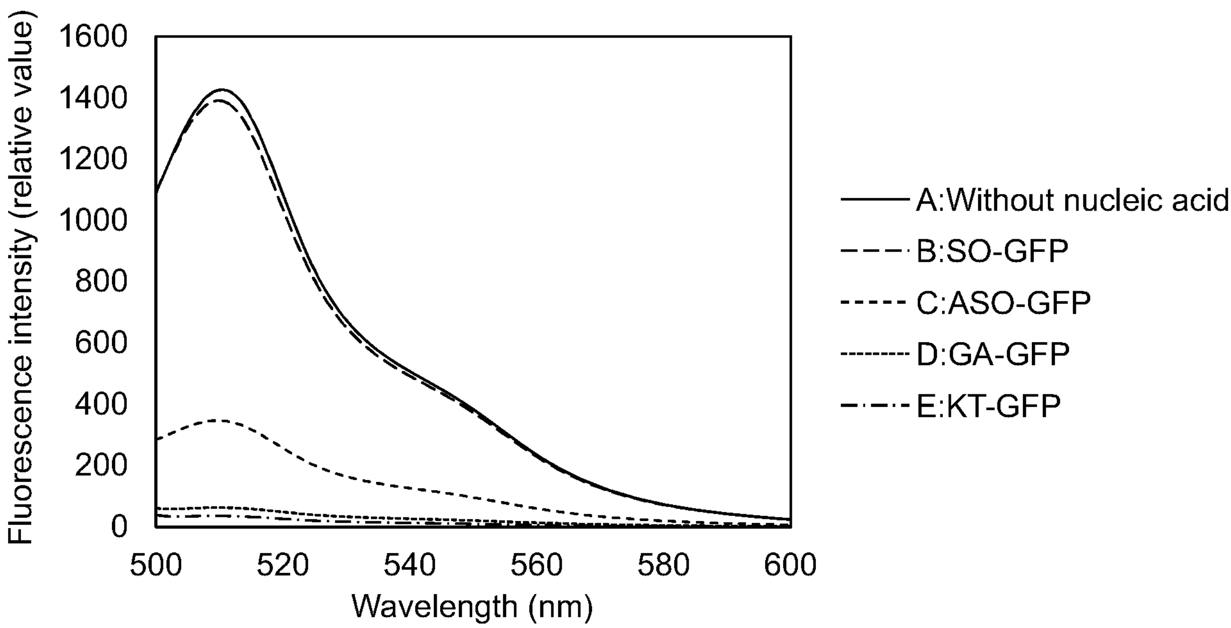
Fluorescence intensity (relative value)
1600
1400
1200
1000
800
600
400
200
0
500
520
540
560
580
600
Wavelength (nm)
A:Without nucleic acid
B:SO-GFP
C:ASO-GFP
D:GA-GFP
E:KT-GFP

Fig. 25

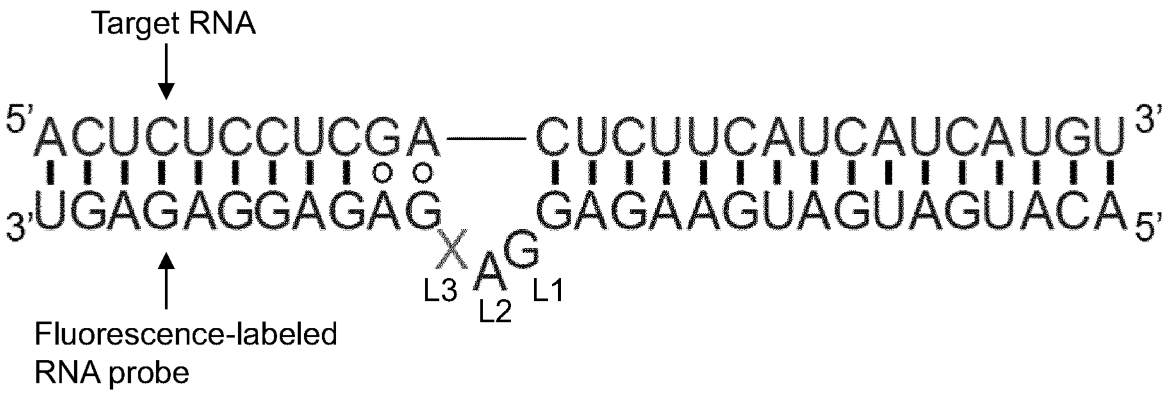
Target RNA
5' ACUCUCCUCGA CUCUUCAUCAUCAUGU 3'
3' UGAGAGGAGAG GAGAAGUAGUAGUACA 5'
X A G
L3 L2 L1
Fluorescence-labeled RNA probe

Fig. 26

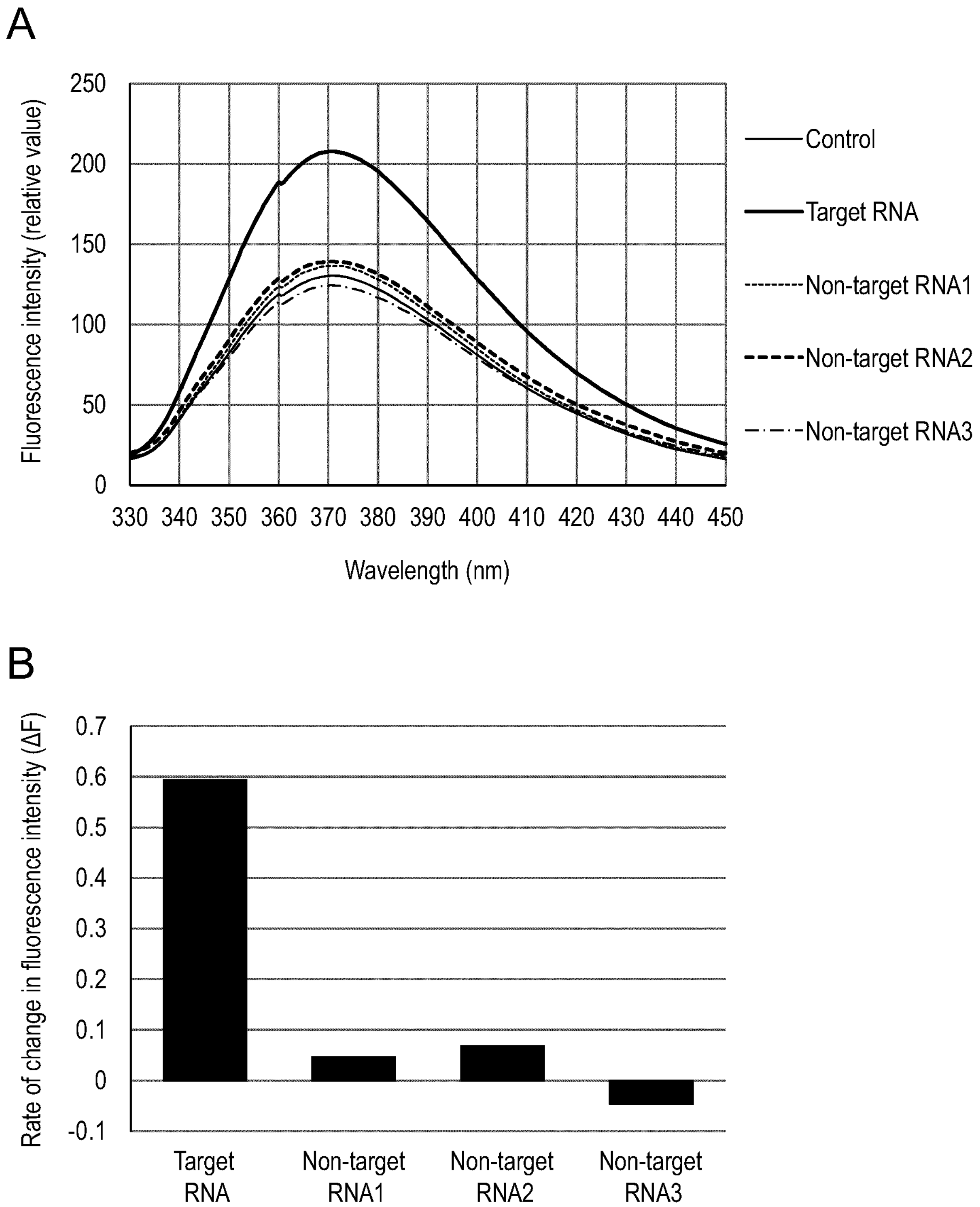
A
250
200
150
100
50
0
Fluorescence intensity (relative value)
330 340 350 360 370 380 390 400 410 420 430 440 450
Wavelength (nm)
Control
Target RNA
Non-target RNA1
Non-target RNA2
Non-target RNA3
B
0.7
0.6
0.5
0.4
0.3
0.2
0.1
0
-0.1
Rate of change in fluorescence intensity (ΔF)
Target RNA
Non-target RNA1
Non-target RNA2
Non-target RNA3

Fig. 27

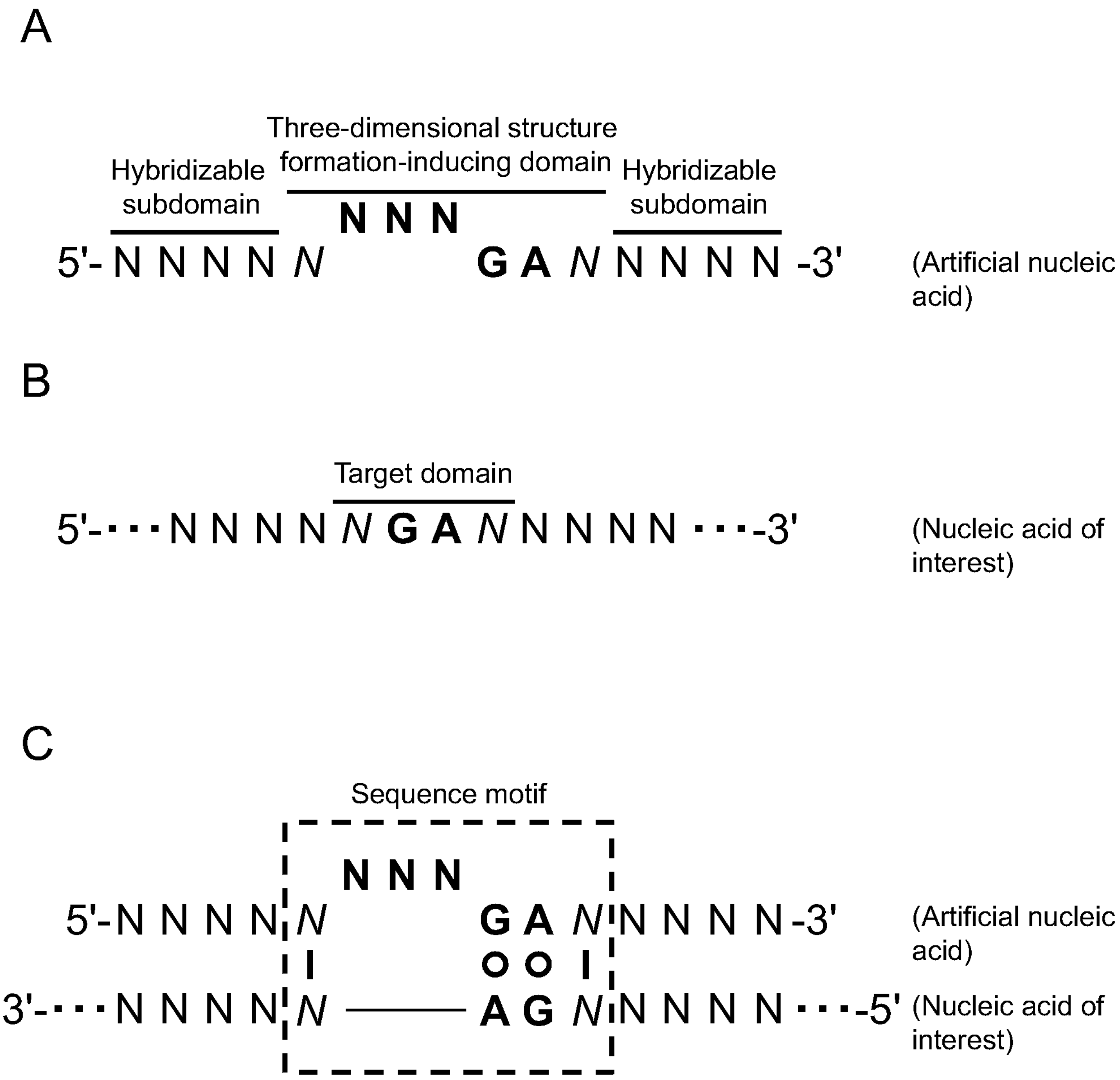
A
Three-dimensional structure formation-inducing domain
Hybridizable subdomain
Hybridizable subdomain
N N N
5'- N N N N N G A N N N N N -3'
(Artificial nucleic acid)
B
Target domain
5'-・・・N N N N N G A N N N N N・・・-3'
(Nucleic acid of interest)
C
Sequence motif
N N N
5'-N N N N N G A N N N N N -3'
(Artificial nucleic acid)
3'-・・・N N N N N A G N N N N N・・・-5'
(Nucleic acid of interest)

Fig. 28

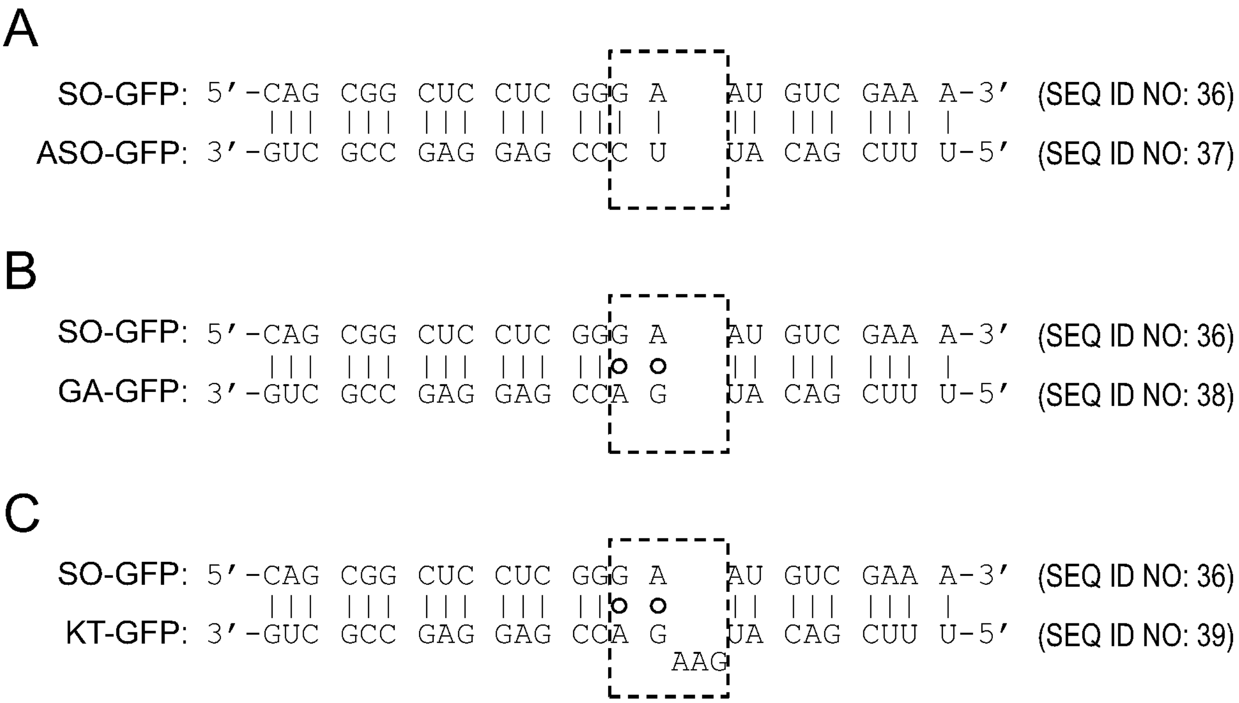
A
SO-GFP: 5′-CAG CGG CUC CUC GGG A AU GUC GAA A-3′ (SEQ ID NO: 36)
ASO-GFP: 3′-GUC GCC GAG GAG CCC U UA CAG CUU U-5′ (SEQ ID NO: 37)
B
SO-GFP: 5′-CAG CGG CUC CUC GGG A AU GUC GAA A-3′ (SEQ ID NO: 36)
GA-GFP: 3′-GUC GCC GAG GAG CCA G UA CAG CUU U-5′ (SEQ ID NO: 38)
C
SO-GFP: 5′-CAG CGG CUC CUC GGG A AU GUC GAA A-3′ (SEQ ID NO: 36)
KT-GFP: 3′-GUC GCC GAG GAG CCA G UA CAG CUU U-5′ (SEQ ID NO: 39)
AAG

**INTERNATIONAL SEARCH REPORT**

International application No.
**PCT/JP2022/029566**

**A. CLASSIFICATION OF SUBJECT MATTER**

***C12N 15/113***(2010.01)i; ***A61K 31/7088***(2006.01)i; ***A61K 31/712***(2006.01)i; ***A61K 31/713***(2006.01)i; ***A61K 48/00***(2006.01)i; ***A61P 43/00***(2006.01)i; ***C12N 15/10***(2006.01)i; ***C12Q 1/6813***(2018.01)i
FI: C12N15/113 Z ZNA; C12Q1/6813 Z; C12N15/10 Z; A61K48/00; A61K31/7088; A61K31/713; A61K31/712; A61P43/00 105

According to International Patent Classification (IPC) or to both national classification and IPC

**B. FIELDS SEARCHED**

Minimum documentation searched (classification system followed by classification symbols)

C12N15/113; A61K31/7088; A61K31/712; A61K31/713; A61K48/00; A61P43/00; C12N15/10; C12Q1/6813

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | ALEMAN, Lourdes M. et al. Comparison of siRNA-induced off-target RNA and protein effects. RNA. 2007, vol. 13, pp. 385-395<br>table 1 | 1-4, 7-16 |
| A | table 1 | 5, 6 |
| X | CHIU, Yalin et al. RNAi in Human Cells: Basic Structural and Functional Features of Small Interfering RNA. Mol. Cell. 2002, vol. 10, pp. 549-561<br>fig. 2 | 1-4, 7-16 |
| A | fig. 2 | 5, 6 |
| A | BOONE, Edna. SCHUSTER, Gary B. Long-range oxidative damage in duplex DNA: the effect of bulged G in a G-C tract and tandem G/A mispairs. Nucleic Acids Res. 2002, vol. 30, no. 3, pp. 830-837<br>fig. 2 | 1-16 |

☑ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 October 2022** | **18 October 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/029566**

**C. DOCUMENTS CONSIDERED TO BE RELEVANT**

| Category* | Citation of document, with indication, where appropriate, of the relevant passages | Relevant to claim No. |
|---|---|---|
| A | SCHROEDER, Kersten T. et al. Structure and folding of a rare, natural kink turn in RNA with an A•A pair at the 2b•2n position. RNA. 2012, vol. 18, pp. 1257-1266<br>fig. 1 | 1-16 |
| A | WIMBERLY, Brian et al. The Conformation of Loop E of Eukaryotic 5S Ribosomal RNA. Biochemistry. 1993, vol. 32, pp. 1078-1087<br>fig. 1 | 1-16 |
| A | YANG, Mei (Monica) and THOMPSON, Richard. Comparative Stability Determination of Oligonucleotide Duplexes in Gas and Solution Phase. J. Am. Soc. Mass Spectrom. 2004, vol. 15, pp. 1354-1359<br>table 1 | 1-16 |

Form PCT/ISA/210 (second sheet) (January 2015)

**INTERNATIONAL SEARCH REPORT**

International application No.
**PCT/JP2022/029566**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

The “form of an Annex C/ST.25 text file” is replaced with the “form of ST.26.”

Form PCT/ISA/210 (continuation of first sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021126578 A


### Non-patent literature cited in the description


- **LEONTIS, N.B. et al.** *Nucleic Acids Res.,* 15 August 2002, vol. 30 (16), 3497-3531
- **CORRELL, C. C. et al.** *Nucleic Acids Research,* 2003, vol. 31 (23), 6806-6818
- **CORRELL, C. C. et al.** *Proc. Natl. Acad. Sci.,* 1998, vol. 95, 13436-13441
- **GLUCK, A. ; WOOL, I. G.** *J. Mol. Biol.,* 1996, vol. 256, 838-848
- **HUANG, L. ; LILLEY, D. M.** *Journal of Molecular Biology,* 2016, vol. 428 (5), 790-801
- **HUANG, L. ; LILLEY, D. M.** *Quarterly Reviews of Biophysics,* 2018, vol. 51 (e5
- **STROBEL, S. A. et al.** *Rna,* 2004, vol. 10 (12), 1852-1854
- **CORRELL, C. C. et al.** *Cell,* 1997, vol. 91 (5), 705-712
- **LEONTIS, N. B. ; WESTHOF, E.** *Rna,* 1998, vol. 4 (9), 1134-1153
- **KLEIN, D. J. et al.** *Journal of molecular biology,* 2004, vol. 340 (1), 141-177
- **LESCOUTE, A. et al.** *Nucleic acids research,* 2005, vol. 33 (8), 2395-2409
- **JANG, S. B. et al.** *Acta Crystallographica Section D: Biological Crystallography,* 2004, vol. 60 (5), 829-835
- **ALTSCHUL, S. F. et al.** *J. Mol. Biol.,* 1990, vol. 215, 403-410
- **MCPHEE, S. A. et al.** *Nature communications,* 2014, vol. 5 (1), 1-6
- **COONROD, L. A. et al.** *Biochemistry,* 2012, vol. 51 (42), 8330-8337